# EUROPEAN PATENT APPLICATION

(11) **EP 4 501 111 A1**
(43) Date of publication of application: **05.02.2025**
(21) Application number: 23774986.6
(22) Date of filing: 23.03.2023
(51) Int. Cl.: A01N 31/08, A01P 3/00, A01N 43/12, A01N 25/00

(54) **METHOD FOR CONTROLLING PLANT DISEASES**

(30) Priority: 24.03.2022 JP 2022048030
(71) Applicant: Sumitomo Chemical Company, Limited, Tokyo 103-6020 (JP)
(72) Inventor: ARAI, Keisuke, Takarazuka-shi, Hyogo 665-8555 (JP); NOKURA, Yoshihiko, Takarazuka-shi, Hyogo 665-8555 (JP); MAEHATA, Nao, Takarazuka-shi, Hyogo 665-8555 (JP)
(74) Representative: Vossius & Partner Patentanwälte Rechtsanwälte mbB
(86) International application number: PCT/JP2023/011358
(87) International publication number: WO 2023/182391

(57) **Abstract**

The present invention provides a method for controlling a plant disease which comprises applying a compound represented by formula (I) [wherein R¹ represents a C2-C6 chain hydrocarbon group which may be optionally substituted with one or more substituents selected from Group A or the like, R² represents a C1-C6 chain hydrocarbon group which may be optionally substituted with one or more halogen atoms or the like, R³ represents a C1-C6 chain hydrocarbon group which may be optionally substituted with one or more substituents selected from Group A and the like, R⁴ and R⁵ are identical to or different from each other, and each represents a C1-C6 chain hydrocarbon group which may be optionally substituted with one or more halogen atoms or the like, n is 0 or the like, p is 0 or the like, and p is 1 or the like] to a plant or soil for cultivating the plant, which has excellent control efficacies against plant diseases.

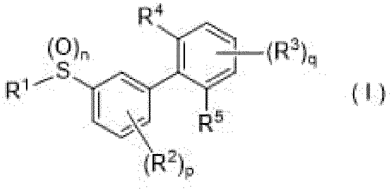

## Description

### TECHNICAL FIELD

This application claims priorities to and the benefits of Japanese Patent Application No. 2022-048030 filed on March 24, 2022, the entire contents of which are incorporated herein by reference.

The present invention relates to a method for controlling plant diseases.

### BACKGROUND ART

Patent documents 1 describes biphenyl compounds as an agent for controlling plant diseases.

### CITATION LIST

### PATENT DOCUMENT

Patent Document 1: WO 2008/053044

### SUMMARY OF THE INVENTION

### PROBLEMS TO BE SOLVED BY INVENTION

An object of the present invention is to provide a method having an excellent efficacy for controlling plant diseases

### MEANS TO SOLVE PROBLEMS

The present inventors have studied so as to find a method having an excellent efficacy for controlling plant diseases, and have found out that the compound represented by the following formula (I) has an excellent efficacy on controlling plant diseases.

That is, the present invention is as follows.
[1] A method for controlling a plant disease which comprises applying a compound represented by formula (I): 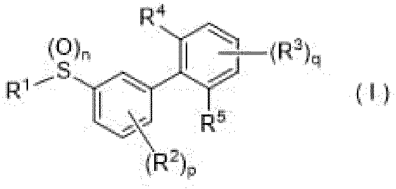 [wherein
   R¹ represents a C2-C6 chain hydrocarbon group which may be optionally substituted with one or more substituents selected from Group A, a C3-C8 alicyclic hydrocarbon group which may be optionally substituted with one or more substituents selected from Group B, a three (3)- to seven (7)- membered non-aromatic heterocyclic group which may be optionally substituted with one or more substituents selected from Group B, a methyl group which is substituted with one or more substituents selected from Group C, a C6-C10 aryl group, a five (5)- to ten (10)- membered aromatic heterocyclic group {each of the C6-C10 aryl group, and the five (5)- to ten (10)- membered aromatic heterocyclic group may optionally have one or more substituents selected from Group E}, or NR²⁹R³⁰,
   n is 0, 1, or 2,
   R² represents a C1-C6 chain hydrocarbon group which may be optionally substituted with one or more halogen atoms, a C3-C8 alicyclic hydrocarbon group which may be optionally substituted with one or more substituents selected from Group B, OR⁶, SR⁷, S(O)R⁸, S(O)₂R⁹, NR¹⁰R¹¹, C(O)R¹², a nitro group, a cyano group, or a halogen atom,
   p is 0, 1, or 2,
   when p is 2, two of the R² may be identical to or different from each other,
   R³ represents a C1-C6 chain hydrocarbon group which may be optionally substituted with one or more substituents selected from Group A, a C3-C8 alicyclic hydrocarbon group, a three (3) - to eight (8) - membered non-aromatic heterocyclic group {each of the C3-C8 alicyclic hydrocarbon group, and the three (3)- to eight (8)- membered non-aromatic heterocyclic group may be optionally substituted with one or more substituents selected from Group B}, a phenyl group, a five (5)- to six (6)- membered aromatic heterocyclic group {each of the phenyl group, and the five (5)- to six (6)-membered aromatic heterocyclic group may optionally have one or more substituents selected from Group D}, OR¹³, SR¹⁴, S(O)R¹⁵, S(O)₂R¹⁶, NR¹⁷R¹⁸, C(O)R¹⁹, C(R²⁰)=NOR²¹, a cyano group, a nitro group, an amino group, a hydroxy group, or a halogen atom,
   q is 1, 2, or 3,
   when q is 2 or 3, two or three of the R³ may be identical to or different from each other,
   R⁴ and R⁵ are identical to or different from each other, and each represents a C1-C6 chain hydrocarbon group which may be optionally substituted with one or more halogen atoms, a C3-C8 alicyclic hydrocarbon group which may be optionally substituted with one or more substituents selected from Group B, OR²², SR²³, S(O)R²⁴, S (O)₂R²⁵, NR²⁶R²⁷, C(O)R²⁸, a nitro group, a cyano group, a halogen atom, or a hydrogen atom,
   R⁶, R⁷, R¹⁰, R²², R²³, R²⁶, and R²⁹ are identical to or different from each other, and each represents a C1-C6 chain hydrocarbon group which may be optionally substituted with one or more halogen atoms, or a hydrogen atom,
   R⁸, R⁹, R²⁴, R²⁵, and R³⁰ are identical to or different from each other, and each represents a C1-C6 chain hydrocarbon group which may be optionally substituted with one or more halogen atoms,
   R¹¹, and R²⁷ are identical to or different from each other, and each represents a C1-C6 chain hydrocarbon group which may be optionally substituted with one or more halogen atoms, a (C1-C6 alkyl)carbonyl group, a (C1-C6 alkoxy)carbonyl group {each of the (C1-C6 alkyl)carbonyl group, and the (C1-C6 alkoxy) carbonyl group may be optionally substituted with one or more halogen atoms}, or a hydrogen atom,
   R¹², R¹⁹, and R²⁸ are identical to or different from each other, and each represents a C1-C6 chain hydrocarbon group, a C1-C6 alkoxy group, a C1-C6 alkylamino group, or a C2-C8 dialkylamino group {each of the C1-C6 chain hydrocarbon group, the C1-C6 alkoxy group, the C1-C6 alkylamino group, and the C2-C8 dialkylamino group may be optionally substituted with one or more halogen atoms},
   R¹³, R¹⁴, R¹⁵, R¹⁶, and R²¹ are identical to or different from each other, and each represents a C1-C6 chain hydrocarbon group which may be optionally substituted with one or more substituents selected from Group A, a C3-C8 alicyclic hydrocarbon group, a three (3)- to eight (8)-membered non-aromatic heterocyclic group {each of the C3-C8 alicyclic hydrocarbon group, and the three (3) - to eight (8)- membered non-aromatic heterocyclic group may optionally have one or more substituents selected from Group B}, a phenyl group, or a five (5)- to six (6)- membered aromatic heterocyclic group {each of the phenyl group, and the five (5)- to six (6)- membered aromatic heterocyclic group may optionally have one or more substituents selected from Group D},
   R¹⁷ represents a C1-C6 chain hydrocarbon group which may optionally have one or more substituents selected from Group A, a C3-C8 alicyclic hydrocarbon group, a three (3)- to eight (8)- membered non-aromatic heterocyclic group {each of the C3-C8 alicyclic hydrocarbon group, and the three (3)- to eight (8)- membered non-aromatic heterocyclic group may optionally have one or more substituents selected from Group B}, a phenyl group, a five (5)- to six (6)- membered aromatic heterocyclic group {each of the phenyl group, and the five (5)- to six (6)- membered aromatic heterocyclic group may optionally have one or more substituents selected from Group D}, a (C1-C6 alkyl) carbonyl group, or a (C1-C6 alkoxy)carbonyl group {each of the (C1-C6 alkyl)carbonyl group, and the (C1-C6 alkoxy) carbonyl group may be optionally substituted with one or more halogen atoms},
   R¹⁸ and R²⁰ are identical to or different from each other, and each represents a C1-C6 chain hydrocarbon group which may be optionally substituted with one or more substituents selected from Group A, or a hydrogen atom,
   the neighboring R³ and R⁴ may combine together with a carbon atom to which they are attached to form a C5-C6 alicyclic hydrocarbon, a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {each of the benzene ring, and the five (5)- to six (6)-membered aromatic heterocyclic ring may optionally have one or more substituents selected from Group E},
   two of the neighboring R³ and R³ may combine together with a carbon atom to which they are attached to form a C5-C6 alicyclic hydrocarbon, a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {each of the benzene ring, and the five (5)- to six (6)-membered aromatic heterocyclic ring may optionally have one or more substituents selected from Group E},
   Group A is a group consisting of a C1-C6 alkoxy group, a C1-C6 alkylsulfanyl group, a C1-C6 alkylsulfinyl group, a C1-C6 alkylsulfonyl group, {each of the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)-membered non-aromatic heterocyclic group {each of the C3-C8 alicyclic hydrocarbon group, and the three (3)- to eight (8)- membered non-aromatic heterocyclic group may be optionally substituted with one or more substituents selected from Group B}, a phenyl group, a five (5)- to six (6) - membered aromatic heterocyclic group {each of the phenyl group and the five (5)- to six (6)- membered aromatic heterocyclic group may be optionally substituted with one or more substituents selected from Group D}, a halogen atom, a cyano group, a nitro group, and a hydroxy group.
   Group B is a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {each of the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a halogen atom, and a cyano group.
   Group C is a group consisting of a C1-C6 alkoxy group, a C1-C6 alkylsulfanyl group, a C1-C6 alkylsulfinyl group, a C1-C6 alkylsulfonyl group {each of the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)-membered non-aromatic heterocyclic group {each of the C3-C8 alicyclic hydrocarbon group, and the three (3)- to eight (8)- membered non-aromatic heterocyclic group may be optionally substituted with one or more substituents selected from Group B}, a phenyl group, and the five (5)- to six (6)- membered aromatic heterocyclic group {each of the phenyl group and the five (5)- to six (6)- membered aromatic heterocyclic group may be optionally substituted with one or more substituents selected from Group D}.
   Group D is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonyl amino group, a (C1-C6 alkoxy)carbonyl amino group {each of the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino))carbonyl group, the (C2-C8 dialkylamino) carbonyl group, the (C1-C6 alkyl)carbonyl amino group, and the (C1-C6 alkoxy)carbonyl amino group may be optionally substituted with one or more halogen atoms}, a C3-C8 alicyclic hydrocarbon group which may optionally have one or more substituents selected from Group B, an amino group, a nitro group, a cyano group, a hydroxy group, and a halogen atom.
   Group E is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group {each of the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a halogen atom, and a cyano group] (hereinafter, referred to as "Present compound X") or an N-oxide thereof, or a salt thereof (hereinafter, the compound represented by formula (I), the N-oxide thereof, and the salt thereof are referred to as "Present compound Y") to a plant or soil for cultivating the plant.
[2] The method according to [1] wherein the compound represented by formula (I), the N-oxide thereof, or the salt thereof represents the compound represented by formula (I) wherein R¹ represents a C2-C6 chain hydrocarbon group which may be optionally substituted with one or more substituents selected from Group A, a C3-C8 alicyclic hydrocarbon group which may be optionally substituted with one or more substituents selected from Group B, or a methyl group which is substituted with one or more substituents selected from Group C (hereinafter, referred to as "Present compound N") or an N-oxide thereof, or a salt thereof (hereinafter, referred to as "Present compound").
[3] The method according to [1] or [2] wherein the compound represented by formula (I), the N-oxide thereof, or the salt thereof represents the compound represented by formula (I) wherein R¹ represents a C2-C6 chain hydrocarbon group, R² represents a C1-C6 chain hydrocarbon group which may be optionally substituted with one or more halogen atoms, or a halogen atom, R³ represents a C1-C6 chain hydrocarbon group which may be optionally substituted with one or more substituents selected from Group A, OR¹³, SR¹⁴, S(O)R¹⁵, S(O)₂R¹⁶, NR¹⁷R¹⁸, C(O)R¹⁹, C(R²⁰)=NOR²¹, a cyano group, a nitro group, an amino group, a hydroxy group, or a halogen atom, two of the neighboring R³ and R³ may combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, and R⁴ and R⁵ are identical to or different from each other, and each represents a halogen atom, or a hydrogen atom or an N-oxide thereof, or a salt thereof.
[4] Use of the compound according to any one of [1] to [3], the N-oxide thereof, or the salt thereof for controlling a plant disease.
[5] A method for controlling a soybean rust fungus which comprises applying the compound according to any one of [1] to [3], the N-oxide thereof, or the salt thereof to a soybean or soil for cultivating the soybean.
[6] A compound represented by formula (II): 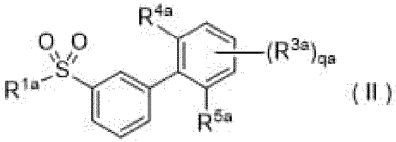 [wherein
   R^{1a} represents a C2-C3 chain hydrocarbon group which may be optionally substituted with one or more halogen atoms, a cyclopropyl group which may be optionally substituted with one or more halogen atoms, a phenyl group, or a five (5)- to six (6)- membered aromatic heterocyclic group {each of the phenyl group and the five (5)- to six (6)- membered aromatic heterocyclic group may be optionally substituted with one or more halogen atoms},
   R^{3a} represents a C1-C3 alkyl group, a C1-C3 alkoxy group, a cyclopropyl group {the cyclopropyl group may be optionally substituted with one or more halogen atoms}, or a halogen atom,
   qa is 1, 2, or 3,
   when qa is 2 or 3, two or three of the R^{3a} may be identical to or different from each other, and
   R^{4a} and R^{5a} represent a hydrogen atom] (hereinafter, referred to as "Compound D of the present invention") or an N-oxide thereof, or a salt thereof (hereinafter, the compound D of the present invention, the N-oxide thereof, and the salt thereof are referred to as "Compound of the present invention").
[7] An agrochemical composition comprising the compound according to [6], the N-oxide thereof, or the salt thereof, and an inert carrier.
[8] A composition comprising one or more ingredients selected from the group consisting of the following Group (a), Group (b), Group (c), and Group (d), as well as the compound according to [6] or an N-oxide thereof, or a salt thereof:
   Group (a): a group consisting of insecticidal active ingredients, miticidal active ingredients and nematicidal active ingredients;
   Group (b): fungicidal active ingredients;
   Group (c): plant growth regulatory ingredients;
   Group (d): repellent ingredients;
[9] A method for controlling a plant disease which comprises applying an effective amount of the compound according to [6] or the N-oxide thereof, or the salt thereof, or an effective amount of the composition according to [8] to a plant or soil for cultivating the plant.
[10] An agrochemical composition comprising the compound according to [6] or the N-oxide thereof, or the salt thereof, and an inert carrier.
[11] A seed or vegetative reproductive organ carrying an effective amount of the compound according to [6] or an N-oxide, or a salt thereof, or an effective amount of the composition according to [8].

The present invention can control plant diseases.

### MODE FOR CARRYING OUT THE INVENTION

The substituents as used herein are explained as follows.

The term "halogen atom" represents a fluorine atom, a chlorine atom, a bromine atom, or an iodine atom.

When a substituent has two or more halogen atoms or groups, the halogen atoms or groups may be identical to or different from each other.

The expression of "may be substituted by one or more substituents selected from Group X" (wherein X represents any one of A, B, C, D, E, A¹, B¹, C¹, E¹, B², C², and E²) as used herein represents that these substituents may be identical to or different from each other, when two or more substituents selected from Group X are present.

The expression "CX-CY" as used herein represents that the number of carbon atoms is from X to Y. For example, the expression "C1-C6" represents that the number of carbon atoms is from 1 to 6.

The term "chain hydrocarbon group" represents an alkyl group, an alkenyl group, or an alkynyl group.

Examples of the alkyl group include methyl group, ethyl group, propyl group, isopropyl group, 1,1-dimethylpropyl group, 1,2-dimethylpropyl group, butyl group, sec-butyl group, tert-butyl group, pentyl group, and hexyl group.

Examples of the alkenyl group include vinyl group, 1-propenyl group, 2-propenyl group, 1-methyl-1-propenyl group, 1-methyl-2-propenyl group, 1,2-dimethyl-1-propenyl group, 3-butenyl group, 4-pentenyl group, and 5-hexenyl group.

Examples of the alkynyl group include ethynyl group, 1-propynyl group, 2-propynyl group, 1-methyl-2-propynyl group, 1,1-dimethyl-2-propynyl group, 2-butynyl group, 4-pentynyl group, and 5-hexynyl group.

Examples of the alkoxy group include methoxy group, ethoxy group, propoxy group, isopropoxy group, butoxy group, tert-butoxy group, pentyloxy group, and hexyloxy group.

Examples of the "alkylsulfanyl group" include methylsulfanyl group, ethylsulfanyl group, isopropylsulfanyl group, and hexylsulfanyl group.

Examples of the "alkylsulfinyl group" include methylsulfinyl group, ethylsulfinyl group, isopropylsulfinyl group, and hexylsulfinyl group.

Examples of the "alkylsulfonyl group" include methane sulfonyl group, ethane sulfonyl group, isopropane sulfonyl group, and hexane sulfonyl group.

Examples of the "alkylcarbonyl group" include acetyl group, and propionyl group.

Examples of the "alkoxycarbonyl group" include methoxycarbonyl group, isopropoxycarbonyl group, and hexyloxycarbonyl group.

Examples of the "alicyclic hydrocarbon group" include cycloalkyl group and cycloalkenyl group.

Examples of the "cycloalkyl group" include cyclopropyl group, cyclobutyl group, cyclopentyl group, and cyclohexyl group.

Examples of the "cycloalkenyl group" include cyclopentenyl group and cyclohexenyl group.

Examples of the "aryl group" include phenyl group and naphthyl group.

Examples of the "aromatic heterocyclic group" include a five-membered aromatic heterocyclic group (such as pyrrolyl group, furanyl group, thienyl group, pyrazolyl group, imidazolyl group, triazolyl group, tetrazolyl group, oxazolyl group, isoxazolyl group, thiazolyl group, isothiazolyl group, oxadiazolyl group, and thiadiazolyl group); a six-membered aromatic heterocyclic group (such as pyridyl group, pyridazinyl group, pyrimidinyl group, pyrazinyl group, triazinyl group and tetrazinyl group).

Examples of the "non-aromatic heterocyclic group" include aziridinyl group, oxiranyl group, thiranyl group, azetidinyl group, oxetanyl group, thietanyl group, pyrrolidinyl group, tetrahydrofuranyl group, tetrahydrothienyl group, pyrazolynyl group, pyrazolidinyl group, imidazolinyl group, imidazolidinyl group, oxazolinyl group, thiazolinyl group, oxazolidinyl group, thiazolidinyl group, isoxazolinyl group, isoxazolidinyl group, isothiazolynyl group, isothiazolidinyl group, dioxolanyl group, dioxalanyl group, piperidyl group, piperaziny group, morpholinyl group, thiomorpholinyl group, pyranyl group, dihydropyranyl group, tetrahydropyranyl group, tetrahydrothiopyranyl group, azepanyl group, oxepanyl group, and thiepanyl group).

The present compound Y or the compound of the present invention may be existed as one or more stereoisomers. Examples of the stereoisomer include enantiomer, diastereoisomer, atropisomer, and geometric isomer. Each stereoisomer, and stereoisomer mixture(s) in an arbitrary ratio thereof are included in the present invention.

The present compound X or the compound of the present invention or their N-oxides may form an acid addition salts thereof such as hydrochloride salt, sulfate, nitrate, phosphate, acetate, benzoate and the like by mixing it with an acid such as hydrochloric acid, sulfuric acid, nitric acid, phosphoric acid, acetic acid, benzoic acid and the like.

Specific examples of the present compound Y wherein the neighboring R³ and R⁴ may combine together with a carbon atom to which they are attached to form a C5-C6 alicyclic hydrocarbon, a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {each of the benzene ring, and the five (5)- to six (6)-membered aromatic heterocyclic ring may optionally have one or more substituents selected from Group E} include a compound represented by formula (IA): 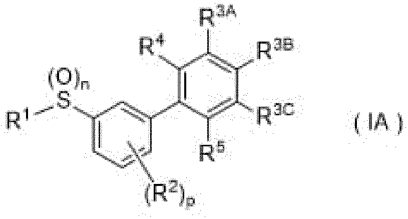 wherein
R¹, R², R³, n and p have the same definitions as above [1],
R^{3B} and R^{3C} are the identical to or different from each other, and each represents the same definitions as R³ in [1] or a hydrogen atom,
R^{3A} and R⁴ may combine together with a carbon atom to which they are attached to form a C5-C6 alicyclic hydrocarbon, a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {each of the benzene ring, and the five (5)- to six (6)- membered aromatic heterocyclic ring may optionally have one or more substituents selected from Group E}.

Specific examples of the present compound Y wherein the neighboring R³ and R⁴ may combine together with a carbon atom to which they are attached to form a C5-C6 alicyclic hydrocarbon, a five (5)- to six (6)- membered non-aromatic heterocyclic ring {each of the C5-C6 alicyclic hydrocarbon, and the five (5)- to six (6)- membered non-aromatic heterocyclic group may optionally have one or more substituents selected from Group B}, a benzene ring, or a five (5)- to six (6)- membered aromatic heterocyclic ring {each of the benzene ring, and the five (5)- to six (6)-membered aromatic heterocyclic ring may optionally have one or more substituents selected from Group E} include
1) the compound represented by formula (IA) wherein
   R¹, R², R⁴, R⁵, n and p have the same definitions as above [1],
   R^{3C} represents the same definitions as R³ in [1] or a hydrogen atom,
   R^{3A} and R^{3B} may combine together with a carbon atom to which they are attached to form a C5-C6 alicyclic hydrocarbon, a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {each of the benzene ring, and the five (5)- to six (6)- membered aromatic heterocyclic ring may optionally have one or more substituents selected from Group E}; or
2) the compound represented by formula (IA) wherein
   R¹, R², R⁴, R⁵, n and p have the same definitions as above [1],
   R^{3A} represents the same definitions as R³ in [1] or a hydrogen atom,
   R^{3B} and R^{3C} may combine together with a carbon atom to which they are attached to form a C5-C6 alicyclic hydrocarbon, a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {each of the benzene ring, and the five (5)- to six (6)- membered aromatic heterocyclic ring may optionally have one or more substituents selected from Group E}.

Embodiments of the present compound X include the following compounds.
[Embodiment X1] The present compound X wherein R¹ represents a C2-C6 chain hydrocarbon group which may optionally have one or more substituents selected from Group A.
[Embodiment X2] The present compound X wherein R¹ represents a C3-C8 alicyclic hydrocarbon group which may optionally have one or more substituents selected from Group B.
[Embodiment X3] The present compound X wherein R¹ represents a methyl group which may optionally have one or more substituents selected from Group C.
[Embodiment X4] The present compound X wherein R¹ represents a C2-C6 chain hydrocarbon group which may optionally have one or more substituents selected from Group A, or a C3-C8 alicyclic hydrocarbon group which may optionally have one or more substituents selected from Group B.
[Embodiment X5] The present compound X wherein R¹ represents a C2-C6 chain hydrocarbon group which may optionally have one or more substituents selected from Group A, or a methyl group which may optionally have one or more substituents selected from Group C.
[Embodiment X6] The present compound X wherein R² represents a C1-C6 chain hydrocarbon group which may optionally have one or more halogen atoms.
[Embodiment X7] The present compound X wherein R² represents a C3-C8 alicyclic hydrocarbon group which may optionally have one or more substituents selected from Group B.
[Embodiment X8] The present compound X wherein R² represents OR⁶, SR⁷, S(O)R⁸, S(O)₂R⁹, NR¹⁰R¹¹, C(O)R¹², a nitro group, or a cyano group.
[Embodiment X9] The present compound X wherein R² represents a halogen atom.
[Embodiment X10] The present compound X wherein R² represents a C1-C6 chain hydrocarbon group which may be optionally substituted with one or more halogen atoms, or a halogen atom.
[Embodiment X11] The present compound X wherein R³ represents a C1-C6 chain hydrocarbon group which may be optionally substituted with one or more substituents selected from Group A.
[Embodiment X12] The present compound X wherein R³ represents a C3-C8 alicyclic hydrocarbon group, or a three (3)- to eight (8)- membered non-aromatic heterocyclic group {each of the C3-C8 alicyclic hydrocarbon group, and the three (3)- to eight (8)- membered non-aromatic heterocyclic group may optionally have one or more substituents selected from Group B}.
[Embodiment X13] The present compound X wherein R³ represents a phenyl group, or a five (5)- to six (6)-membered aromatic heterocyclic group {each of the phenyl group, and the five (5)- to six (6)- membered aromatic heterocyclic group may optionally have one or substituents selected from Group D}.
[Embodiment X14] The present compound X wherein R³ represents OR¹³, SR¹⁴, S(O)R¹⁵, S (O)₂R¹⁶, NR¹⁷R¹⁸, C(O)R¹⁹, C (R²⁰)=NOR²¹, a cyano group, a nitro group, an amino group, or a hydroxy group.
[Embodiment X15] The present compound X wherein R³ represents a halogen atom.
[Embodiment X16] The present compound X wherein R³ represents a C1-C6 chain hydrocarbon group which may be optionally substituted with one or more substituents selected from Group A, OR¹³, SR¹⁴, S(O)R¹⁵, S (O)₂R¹⁶, NR¹⁷R¹⁸, C(O)R¹⁹, C (R²⁰)=NOR²¹, a cyano group, a nitro group, an amino group, a hydroxy group, or a halogen atom.
[Embodiment X17] The present compound X wherein R³ represents a C1-C6 chain hydrocarbon group which may be optionally substituted with one or more substituents selected from Group A, or a halogen atom.
[Embodiment X18] The present compound X wherein the neighboring R³ and R⁴ may combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}.
[Embodiment X19] The present compound X wherein the neighboring R³ and R⁴ may combine together with a carbon atom to which they are attached to form a benzene ring, or a five (5)- to six (6)- membered aromatic heterocyclic ring {each of the benzene ring, and the five (5)- to six (6)- membered aromatic heterocyclic ring may optionally have one or more substituents selected from Group E}.
[Embodiment X20] The present compound X wherein the neighboring R³ and R⁴ may combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}.
[Embodiment X21] The present compound X wherein two of the neighboring R³ and R³ may combine together with a carbon atom to which they are attached to form a benzene ring, or a five (5)- to six (6)- membered aromatic heterocyclic ring {each of the benzene ring, and the five (5)- to six (6)-membered aromatic heterocyclic ring may optionally have one or more substituents selected from Group E}.
[Embodiment X22] The present compound X wherein R⁴ and R⁵ are identical to or different from each other, and each represents a C1-C6 chain hydrocarbon group which may be optionally substituted with one or more halogen atoms, a halogen atom, or a hydrogen atom.
[Embodiment X23] The present compound X wherein R⁴ and R⁵ are identical to or different from each other, and each represents a C3-C8 alicyclic hydrocarbon group which may optionally have one or more substituents selected from Group B, a halogen atom, or a hydrogen atom.
[Embodiment X24] The present compound X wherein R⁴ and R⁵ are identical to or different from each other, and each represents OR²², SR²³, S(O)R²⁴, S (O)₂R²⁵, NR²⁶R²⁷, C(O)R²⁸, a nitro group, a cyano group, a halogen atom, or a hydrogen atom.
[Embodiment X25] The present compound X wherein R⁴ and R⁵ are identical to or different from each other, and each represents a halogen atom, or a hydrogen atom.
[Embodiment X26] The present compound X wherein R⁴ and R⁵ represent a hydrogen atom.
[Embodiment X27] The present compound X wherein n is 0.
[Embodiment X28] The present compound X wherein n is 1.
[Embodiment X29] The present compound X wherein n is 2.
[Embodiment X30] The present compound X wherein p is 0.
[Embodiment X31] The present compound X wherein p is 1.
[Embodiment X32] The present compound X wherein p is 2.
[Embodiment X33] The present compound X wherein q is 1.
[Embodiment X34] The present compound X wherein q is 2.
[Embodiment X35] The present compound X wherein q is 3.
[Embodiment X36] The present compound X wherein R² represents a C1-C6 chain hydrocarbon group which may be optionally substituted with one or more halogen atoms, or a halogen atom, R³ represents a C1-C6 chain hydrocarbon group which may be optionally substituted with one or more substituents selected from Group A, OR¹³, SR¹⁴, S(O)R¹⁵, S(O)₂R¹⁶, NR¹⁷R¹⁸, C(O)R¹⁹, C(R²⁰)=NOR²¹, a cyano group, a nitro group, an amino group, a hydroxy group, or a halogen atom, two of the neighboring R³ and R³ may combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, and R⁴ and R⁵ are identical to or different from each other, and each represents a halogen atom, or a hydrogen atom.
[Embodiment X37] The present compound X wherein p is 0, R³ represents a C1-C6 chain hydrocarbon group which may be optionally substituted with one or more substituents selected from Group A, or a halogen atom, two of the neighboring R³ and R³ may combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, and R⁴ and R⁵ are identical to or different from each other, and each represents a hydrogen atom.
[Embodiment X38] The present compound X wherein R¹ represents a C2-C6 chain hydrocarbon group, R² represents a C1-C6 chain hydrocarbon group which may be optionally substituted with one or more halogen atoms, or a halogen atom, R³ represents a C1-C6 chain hydrocarbon group which may be optionally substituted with one or more substituents selected from Group A, OR¹³, SR¹⁴, S(O)R¹⁵, S(O)₂R¹⁶, NR¹⁷R¹⁸, C(O)R¹⁹, C(R²⁰)=NOR²¹, a cyano group, a nitro group, an amino group, a hydroxy group, or a halogen atom, two of the neighboring R³ and R³ may combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, and R⁴ and R⁵ are identical to or different from each other, and each represents a halogen atom, or a hydrogen atom.
[Embodiment X39] A method for controlling soybean rust fungus, which comprises applying the present compound X to soybeans or soil for cultivating the soybeans.
[Embodiment X40] A method for controlling soybean rust fungus, which comprises applying the compound described in the Embodiment X38 to soybeans or soil for cultivating the soybeans.
[Embodiment X41] The present compound X wherein R¹ represents a three (3)- to seven (7)- membered non-aromatic heterocyclic group which may optionally have one or more substituents selected from Group B.
[Embodiment X42] The present compound X wherein R¹ represents a C6-C10 aryl group which may optionally have one or more substituents selected from Group E.
[Embodiment X43] The present compound X wherein R¹ represents a five (5)- to ten (10)- membered aromatic heterocyclic group which may optionally have one or more substituents selected from Group E.
[Embodiment X44] The present compound X wherein R¹ represents NR²⁹R³⁰.
[Embodiment X45] The present compound X wherein R¹ represents a C2-C6 chain hydrocarbon group, a C6-C10 aryl group, or a five (5)- to ten (10)- membered aromatic heterocyclic group {each of the C6-C10 aryl group, and the five (5)- to ten (10)- membered aromatic heterocyclic group may optionally have one or more substituents selected from Group E}, R² represents a C1-C6 chain hydrocarbon group which may be optionally substituted with one or more halogen atoms, or a halogen atom, R³ represents a C1-C6 chain hydrocarbon group which may be optionally substituted with one or more substituents selected from Group A, OR¹³, SR¹⁴, S(O)R¹⁵, S (O)₂R¹⁶, NR¹⁷R¹⁸, C(O)R¹⁹, C(R²⁰)=NOR²¹, a cyano group, a nitro group, an amino group, a hydroxy group, or a halogen atom, two of the neighboring R³ and R³ combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, and R⁴ and R⁵ are identical to or different from each other, and each represents a halogen atom, or a hydrogen atom.
[Embodiment X46] The present compound X wherein R¹ represents a C6-C10 aryl group which may optionally have one or more substituents selected from Group E, R² represents a C1-C6 chain hydrocarbon group which may be optionally substituted with one or more halogen atoms, or a halogen atom, R³ represents a C1-C6 chain hydrocarbon group which may be optionally substituted with one or more substituents selected from Group A, OR¹³, SR¹⁴, S(O)R¹⁵, S (O)₂R¹⁶, NR¹⁷R¹⁸, C(O)R¹⁹, C(R²⁰)=NOR²¹, a cyano group, a nitro group, an amino group, a hydroxy group, or a halogen atom, two of the neighboring R³ and R³ may combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, and R⁴ and R⁵ are identical to or different from each other, and each represents a halogen atom, or a hydrogen atom.
[Embodiment X47] The present compound X wherein R¹ represents a five (5)- to ten (10)- membered aromatic heterocyclic group which may optionally have one or more substituents selected from Group E, R² represents a C1-C6 chain hydrocarbon group which may be optionally substituted with one or more halogen atoms, or a halogen atom, R³ represents a C1-C6 chain hydrocarbon group which may be optionally substituted with one or more substituents selected from Group A, OR¹³, SR¹⁴, S(O)R¹⁵, S (O)₂R¹⁶, NR¹⁷R¹⁸, C(O)R¹⁹, C(R²⁰)=NOR²¹, a cyano group, a nitro group, an amino group, a hydroxy group, or a halogen atom, two of the neighboring R³ and R³ may combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, and R⁴ and R⁵ are identical to or different from each other, and each represents a halogen atom, or a hydrogen atom.
[Embodiment X48] A method for controlling soybean rust fungus, which comprises applying the compound described in the Embodiment X45 to soybeans or soil for cultivating the soybeans.
[Embodiment X49] A method for controlling soybean rust fungus, which applying any one of the compounds described in the Embodiments X1 to X38 or the Embodiments X41 to X47, or the present compound X, or their N-oxides or the salts thereof to soybeans or soil for cultivating the soybeans.

Examples of the Embodiments of the present compound X include the following compounds.
[Embodiment Z1] A compound represented by formula (IAA): 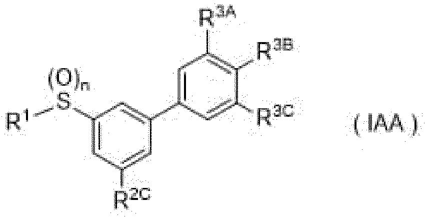 [wherein
   R¹ represents a C2-C6 chain hydrocarbon group which may optionally have one or more substituents selected from Group A¹, a C3-C6 cycloalkyl group which may optionally have one or more halogen atoms, a three (3)- to seven (7)- membered non-aromatic heterocyclic group which may optionally have one or more substituents selected from Group B¹, a methyl group which has one or more substituents selected from Group C¹, a phenyl group, a five (5)- to six (6)- membered aromatic heterocyclic group {each of the phenyl group, and the five (5)- to six (6)- membered aromatic heterocyclic group may optionally have one or more substituents selected from Group E1}, or NR29R30,
   n is 0, 1, or 2,
   R^{2c} represents a fluorine atom, or a hydrogen atom,
   R^{3A} and R^{3C} are identical to or different from each other, and each represents a C1-C3 alkyl group which may optionally have one or more halogen atoms, a halogen atom, or a hydrogen atom,
   R^{3B} represents a C1-C3 alkyl group which may optionally have one or more halogen atoms, or a halogen atom,
   R²⁹ and R³⁰ are identical to or different from each other, and each represents a C1-C6 alkyl group which may optionally have one or more halogen atoms,
   Group A¹ is a group consisting of a cyclopropyl group which may optionally have one or more halogen atoms, a phenyl group, a five (5)- to six (6)- membered aromatic heterocyclic group {each of the phenyl group, and the five (5)- to six (6)- membered aromatic heterocyclic group may optionally have one or more substituents selected from Group E1}, and a halogen atom.
   Group B¹ is a group consisting of a C1-C3 alkyl group which may optionally have one or more halogen atoms, and a halogen atom.
   Group C¹ is a group consisting of a cyclopropyl group which may optionally have one or more halogen atoms, a phenyl group, a five (5)- to six (6)- membered aromatic heterocyclic group {each of the phenyl group, and the five (5)- to six (6)- membered aromatic heterocyclic group may optionally have one or more substituents selected from Group E1}, and a halogen atom.
   Group E¹ is a group consisting of a C1-C3 alkyl group which may optionally have one or more halogen atoms, and a halogen atom}.
[Embodiment Z2] The compound described in the Embodiment Z¹ wherein R^{3A} and R^{3C} are identical to or different from each other, and each represents a methyl group which may optionally have one or more halogen atoms, a halogen atom, or a hydrogen atom, and R^{3B} represents a methyl group which may optionally have one or more halogen atoms or a halogen atom.
[Embodiment Z3] The compound described in the Embodiment Z1 wherein R^{3A} and R^{3C} are identical to or different from each other, and each represents a methyl group which may optionally have one or more halogen atoms, a fluorine atom, or a hydrogen atom, and R^{3B} represents a methyl group which may optionally have one or more halogen atoms or a halogen atom.
[Embodiment Z4] The compound described in the Embodiment Z1 wherein R^{3A} and R^{3C} are identical to or different from each other, and each represents a methyl group which may optionally have one or more halogen atoms, a fluorine atom, or a hydrogen atom, and R^{3B} represents a methyl group which may optionally have one or more halogen atoms, a fluorine atom, or a chlorine atom.
[Embodiment Z5] The compound described in the Embodiment Z1 wherein R^{3A} and R^{3C} are identical to or different from each other, and each represents a methyl group which may optionally have one or more halogen atoms, a fluorine atom, or a hydrogen atom, and R^{3B} represents a methyl group which may optionally have one or more halogen atoms, a fluorine atom, or a chlorine atom.
[Embodiment Z6] The compound described in the Embodiment Z1 wherein R^{3A} and R^{3C} are identical to or different from each other, and each represents a methyl group, a fluorine atom, or a hydrogen atom, and R^{3B} represents a methyl group which may optionally have one or more halogen atoms, a fluorine atom, or a chlorine atom.
[Embodiment Z7] The compound described in the Embodiment Z1 wherein R^{3A} represents a methyl group, a fluorine atom, or a hydrogen atom, R^{3B} represents a methyl group which may optionally have one or more halogen atoms, a fluorine atom, or a chlorine atom, and R^{3C} represents a fluorine atom, or a hydrogen atom.
[Embodiment Z8] The compound described in the Embodiment Z1 wherein R^{3A} and R^{3C} are identical to or different from each other, and each represents a fluorine atom, or a hydrogen atom, and R^{3B} represents a methyl group which may optionally have one or more halogen atoms, a fluorine atom, or a chlorine atom.
[Embodiment Z9] The compound described in the Embodiment Z1 wherein R^{3A} and R^{3C} are identical to or different from each other, and each represents a fluorine atom, or a hydrogen atom, and R^{3B} represents a methyl group, a fluorine atom, or a chlorine atom.
[Embodiment Z10] The compound described in the Embodiment Z1 wherein R^{3A} and R^{3C} are identical to or different from each other, and each represents a fluorine atom or a hydrogen atom, and R^{3B} represents a fluorine atom, or a chlorine atom.
[Embodiment Z11] The compound described in the Embodiment Z1 wherein R^{3A} and R^{3C} are identical to or different from each other, and each represents a fluorine atom, or a hydrogen atom, and R^{3B} represents a chlorine atom.
[Embodiment Z12] The compound described in the Embodiment Z1 wherein R^{2C} represents a hydrogen atom.
[Embodiment Z13] The compound described in the Embodiment Z2 wherein R^{2C} represents a hydrogen atom.
[Embodiment Z14] The compound described in the Embodiment Z3 wherein R^{2C} represents a hydrogen atom.
[Embodiment Z15] The compound described in the Embodiment Z4 wherein R^{2C} represents a hydrogen atom.
[Embodiment Z16] The compound described in the Embodiment Z5 wherein R^{2C} represents a hydrogen atom.
[Embodiment Z17] The compound described in the Embodiment Z6 wherein R^{2C} represents a hydrogen atom.
[Embodiment Z18] The compound described in the Embodiment Z7 wherein R^{2C} represents a hydrogen atom.
[Embodiment Z19] The compound described in the Embodiment Z8 wherein R^{2C} represents a hydrogen atom.
[Embodiment Z20] The compound described in the Embodiment Z9 wherein R^{2C} represents a hydrogen atom.
[Embodiment Z21] The compound described in the Embodiment Z10 wherein R^{2C} represents a hydrogen atom.
[Embodiment Z22] The compound described in the Embodiment Z11 wherein R^{2C} represents a hydrogen atom.
[Embodiment Z23] The compound described in any one of the Embodiments Z¹ to Z22, wherein R¹ represents a C2-C4 chain hydrocarbon group which may optionally have one or more substituents selected from Group A¹, a C3-C4 cycloalkyl group which may optionally have one or more halogen atoms, a three (3)- to seven (7)- membered non-aromatic heterocyclic group which may optionally have one or more substituents selected from Group B¹, a methyl group which has one or more substituents selected from Group C¹, a five (5)- to six (6)-membered aromatic heterocyclic group {the five (5)- to six (6)- membered aromatic heterocyclic group may optionally have one or more substituents selected from Group E1}, or NR²⁹R³⁰, and R²⁹ and R³⁰ are identical to or different from each other, and each represents a C1-C3 alkyl group.
[Embodiment Z24] The compound described in any one of the Embodiments Z1 to Z22, wherein R¹ represents a C2-C4 alkyl group which may optionally have one or more fluorine atoms, a C3-C4 cycloalkyl group which may optionally have one or more fluorine atoms, a methyl group which has one or more substituents selected from Group C¹, a five (5)- to six (6)-membered aromatic heterocyclic group {the five (5)- to six (6)- membered aromatic heterocyclic group may optionally have one or more substituents selected from Group E¹}, or NR²⁹R³⁰, and R²⁹ and R³⁰ are identical to or different from each other, and each represents a C1-C3 alkyl group.
[Embodiment Z25] The compound described in any one of the Embodiments Z¹ to Z22, wherein R¹ represents a C2-C4 alkyl group which may optionally have one or more fluorine atoms, a five (5)- to six (6)- membered non-aromatic heterocyclic group which may optionally have one or more substituents selected from Group B², a methyl group which has one or more substituents selected from Group C², a six (6)-membered aromatic heterocyclic group {the six (6)-membered aromatic heterocyclic group may optionally have one or more substituents selected from Group E²}, NR²⁹R³⁰, and R²⁹ and R³⁰ are identical to or different from each other, and each represents a C1-C3 alkyl group.
   Group B² is a group consisting of a methyl group which may optionally have one or more fluorine atoms, and a halogen atom.
   Group C² is a group consisting of a cyclopropyl group which may optionally have one or more fluorine atoms, a phenyl group, a six (6)-membered aromatic heterocyclic group {the six (6)-membered aromatic heterocyclic group may optionally have one or more substituents selected from Group E²}, and a halogen atom.
   Group E² is a group consisting of a methyl group which may optionally have one or more fluorine atoms, and a halogen atom.
[Embodiment Z26] The compound described in any one of the Embodiments Z1 to Z22, wherein R¹ represents a C2-C4 chain hydrocarbon group which may optionally have one or more substituents selected from Group A¹.
[Embodiment Z27] The compound described in any one of the Embodiments Z¹ to Z22, wherein R¹ represents a C3-C4 cycloalkyl group which may optionally have one or more halogen atoms.
[Embodiment Z28] The compound described in any one of the Embodiments Z¹ to Z22, wherein R¹ represents a three (3)- to seven (7)- membered non-aromatic heterocyclic group which may optionally have one or more substituents selected from Group B¹.
[Embodiment Z29] The compound described in any one of the Embodiments Z1 to Z22, wherein R¹ represents a methyl group which has one or more substituents selected from Group C¹.
[Embodiment Z30] The compound described in any one of the Embodiments Z1 to Z22, wherein R¹ represents a five (5)- to six (6)- membered aromatic heterocyclic group {the five (5)-to six (6)- membered aromatic heterocyclic group may optionally have one or more substituents selected from Group E¹}.
[Embodiment Z31] The compound described in any one of the Embodiments Z1 to Z22, wherein R¹ represents NR²⁹R³⁰, R²⁹ and R³⁰ are identical to or different from each other, and each represents a C1-C3 alkyl group.
[Embodiment Z32] The compound described in any one of the Embodiments Z1 to Z22, wherein R¹ represents a C2-C4 alkyl group which may optionally have one or more fluorine atoms.
[Embodiment Z33] The compound described in any one of the Embodiments Z1 to Z22, wherein R¹ represents a C3-C4 cycloalkyl group which may optionally have one or more fluorine atoms.
[Embodiment Z34] The compound described in any one of the Embodiments Z1 to Z22, wherein R¹ represents a five (5)- to six (6)- membered non-aromatic heterocyclic group which may optionally have one or more substituents selected from Group B².
[Embodiment Z35] The compound described in any one of the Embodiments Z1 to Z22, wherein R¹ represents a methyl group which has one or more substituents selected from Group C².
[Embodiment Z36] The compound described in any one of the Embodiments Z¹ to Z22, wherein R¹ represents a six (6)-membered aromatic heterocyclic group {the six (6)-membered aromatic heterocyclic group may optionally have one or more substituents selected from Group E²}.

Examples of the Embodiments of the present compound include the following compounds.
[Embodiment 1] The present compound wherein R¹ represents a C2-C6 chain hydrocarbon group which may optionally have one or more substituents selected from Group A.
[Embodiment 2] The present compound wherein R¹ represents a C3-C8 alicyclic hydrocarbon group may optionally have one or more substituents selected from Group B.
[Embodiment 3] The present compound wherein R¹ represents a methyl group which may optionally have one or more substituents selected from Group C.
[Embodiment 4] The present compound wherein R¹ represents a C2-C6 chain hydrocarbon group which may optionally have one or more substituents selected from Group A, or a C3-C8 alicyclic hydrocarbon group which may optionally have one or more substituents selected from Group B.
[Embodiment 5] The present compound wherein R¹ represents a C2-C6 chain hydrocarbon group which may optionally have one or more substituents selected from Group A, or a methyl group which may optionally have one or more substituents selected from Group C.
[Embodiment 6] The present compound wherein R² represents a C1-C6 chain hydrocarbon group which may optionally have one or more halogen atoms.
[Embodiment 7] The present compound wherein R² represents a C3-C8 alicyclic hydrocarbon group which may optionally have one or more substituents selected from Group B.
[Embodiment 8]The present compound wherein R² represents OR⁶, SR⁷, S(O)R⁸, S(O)₂R⁹, NR¹⁰R¹¹, C(O)R¹², a nitro group, or a cyano group.
[Embodiment 9] The present compound wherein R² represets a halogen atom.
[Embodiment 10] The present compound wherein R² represets a C1-C6 chain hydrocarbon group which may be optionally substituted with one or more halogen atoms, or a halogen atom.
[Embodiment 11] The present compound wherein R³ represets a C1-C6 chain hydrocarbon group which may be optionally substituted with one or more substituents selected from Group A.
[Embodiment 12] The present compound wherein R³ represets a C3-C8 alicyclic hydrocarbon group, or a three (3)- to eight (8)- membered non-aromatic heterocyclic group {each of the C3-C8 alicyclic hydrocarbon group, and the three (3)- to eight (8)- membered non-aromatic heterocyclic group may optionally have one or more substituents selected from Group B}.
[Embodiment 13] The present compound wherein R³ represets a phenyl group, or a five (5)- to six (6)- membered aromatic heterocyclic group {each of the phenyl group, and the five (5)- to six (6)- membered aromatic heterocyclic group may optionally have one or substituents selected from Group D}.
[Embodiment 14] The present compound wherein R³ represents OR¹³, SR¹⁴, S(O)R¹⁵, S(O)₂R¹⁶, NR¹⁷R¹⁸, C(O)R¹⁹, C(R²⁰)=NOR²¹, a cyano group, a nitro group, an amino group, or a hydroxy group.
[Embodiment 15] The present compound wherein R³ represents a halogen atom.
[Embodiment 16] The present compound wherein R³ represents a C1-C6 chain hydrocarbon group which may be optionally substituted with one or more substituents selected from Group A, OR¹³, SR¹⁴, S(O)R¹⁵, S (O)₂R¹⁶, NR¹⁷R¹⁸, C(O)R¹⁹, C(R²⁰)=NOR²¹, a cyano group, a nitro group, an amino group, a hydroxy group, or a halogen atom.
[Embodiment 17] The present compound wherein R³ represents a C1-C6 chain hydrocarbon group which may be optionally substituted with one or more substituents selected from Group A, or a halogen atom.
[Embodiment 18] The present compound wherein the neighboring R³ and R⁴ combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}.
[Embodiment 19] The present compound wherein the neighboring R³ and R⁴ combine together with a carbon atom to which they are attached to form a benzene ring, or a five (5)- to six (6)- membered aromatic heterocyclic ring {each of the benzene ring, and the a five (5)- to six (6)- membered aromatic heterocyclic ring may optionally have one or more substituents selected from Group E}.
[Embodiment 20] The present compound wherein two of the neighboring R³ and R³ combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}.
[Embodiment 21] The present compound wherein two of the neighboring R³ and R³ combine together with a carbon atom to which they are attached to form a benzene ring, or a five (5)- to six (6)- membered aromatic heterocyclic ring {each of the benzene ring, and the five (5)- to six (6)- membered aromatic heterocyclic ring may optionally have one or more substituents selected from Group E}.
[Embodiment 22] The present compound wherein R⁴ and R⁵ are identical to or different from each other, and each represents a C1-C6 chain hydrocarbon group which may be optionally substituted with one or more halogen atoms, a halogen atom, or a hydrogen atom.
[Embodiment 23] The present compound wherein R⁴ and R⁵ are identical to or different from each other, and each represents a C3-C8 alicyclic hydrocarbon group which may optionally have one or more substituents selected from Group B, a halogen atom, or a hydrogen atom.
[Embodiment 24] The present compound wherein R⁴ and R⁵ are identical to or different from each other, and each represents OR²², SR²³, S(O)R²⁴, S(O)₂R²⁵, NR²⁶R²⁷, C(O)R²⁸, a nitro group, a cyano group, a halogen atom, or a hydrogen atom.
[Embodiment 25] The present compound wherein R⁴ and R⁵ are identical to or different from each other, and each represents a halogen atom, or a hydrogen atom.
[Embodiment 26] The present compound wherein R⁴ and R⁵ represent a hydrogen atom.
[Embodiment 27] The present compound wherein n is 0.
[Embodiment 28] The present compound wherein n is 1.
[Embodiment 29] The present compound wherein n is 2.
[Embodiment 30] The present compound wherein p is 0.
[Embodiment 31] The present compound wherein p is 1.
[Embodiment 32] The present compound wherein p is 2.
[Embodiment 33] The present compound wherein q is 1.
[Embodiment 34] The present compound wherein q is 2.
[Embodiment 35] The present compound wherein q is 3.
[Embodiment 36] The present compound wherein R² represents a C1-C6 chain hydrocarbon group which may be optionally substituted with one or more halogen atoms, or a halogen atom, R³ represents a C1-C6 chain hydrocarbon group which may be optionally substituted with one or more substituents selected from Group A, OR¹³, SR¹⁴, S(O)R¹⁵, S(O)₂R¹⁶, NR¹⁷R¹⁸, C(O)R¹⁹, C(R²⁰)=NOR²¹, a cyano group, a nitro group, an amino group, a hydroxy group, or a halogen atom, two of the neighboring R³ and R³ may combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, and R⁴ and R⁵ are identical to or different from each other, and each represents a halogen atom, or a hydrogen atom.
[Embodiment 37] The present compound wherein p is 0, R³ represents a C1-C6 chain hydrocarbon group which may be optionally substituted with one or more substituents selected from Group A, or a halogen atom, two of the neighboring may combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, and R⁴ and R⁵ are identical to or different from each other, and each represents a hydrogen atom.
[Embodiment 38] The present compound wherein R¹ represents a C2-C6 chain hydrocarbon group, R² represents a C1-C6 chain hydrocarbon group which may be optionally substituted with one or more halogen atoms, or a halogen atom, R³ represents a C1-C6 chain hydrocarbon group which may be optionally substituted with one or more substituents selected from Group A, OR¹³, SR¹⁴, S(O)R¹⁵, S(O)₂R¹⁶, NR¹⁷R¹⁸, C(O)R¹⁹, C(R²⁰)=NOR²¹, a cyano group, a nitro group, an amino group, a hydroxy group, or a halogen atom, two of the neighboring R³ and R³ may combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, and R⁴ and R⁵ are identical to or different from each other, and each represents a halogen atom, or a hydrogen atom.
[Embodiment 39] A method for controlling soybean rust fungus which comprises applying the present compound to soybeans or soil for cultivating the soybeans.
[Embodiment 40] A method for controlling soybean rust fungus which comprises applying the compound described in the Embodiment 38 to soybeans or soil for cultivating the soybeans.

Examples of the Embodiment of the compound D of the present invention includes the following compounds.
[Embodiment D1] The compound D of the present invention wherein R¹³ represents a C2-C3 chain hydrocarbon group which may be optionally substituted with one or more halogen atoms.
[Embodiment D2] The compound D of the present invention wherein R^{1a} represents a cyclopropyl group which may be optionally substituted with one or more halogen atoms.
[Embodiment D3] The compound D of the present invention wherein R^{1a} represents a phenyl group which may be optionally substituted with one or more halogen atoms.
[Embodiment D4] The compound D of the present invention wherein R^{1a} represents a five (5)- to six (6)- membered aromatic heterocyclic group which may be optionally substituted with one or more halogen atoms.
[Embodiment D5] The compound D of the present invention wherein R^{3a} represents a C1-C3 alkyl group.
[Embodiment D6] The compound D of the present invention wherein R^{3a} represents a C1-C3 alkoxy group.
[Embodiment D7] The compound D of the present invention wherein R^{3a} represents a cyclopropyl group which may be optionally substituted with one or more halogen atoms.
[Embodiment D8] The compound D of the present invention wherein R^{3a} represents a halogen atom.
[Embodiment D9] The compound D of the present invention wherein qa is 1.
[Embodiment D10] The compound D of the present invention wherein qa is 2.
[Embodiment D11] The compound D of the present invention wherein qa is 3.
[Embodiment D12] The compound D of the present invention wherein R^{1a} represents a C2-C3 alkyl group.
[Embodiment D13] The compound D of the present invention wherein R^{1a} represents a cyclopropyl group.
[Embodiment D14] The compound D of the present invention wherein R^{1a} represents a phenyl group.
[Embodiment D15] The compound D of the present invention wherein R^{1a} represents a five (5)- to six (6)- membered aromatic heterocyclic group.
[Embodiment D16] The compound D of the present invention wherein R^{1a} represents a C2-C3 alkyl group, a phenyl group, or a five (5)- to six (6)- membered aromatic heterocyclic group.
[Embodiment D17] The compound D of the present invention wherein R^{1a} represents a C2-C3 alkyl group, or a phenyl group.
[Embodiment D18] The compound D of the present invention wherein R^{1a} represents a C2-C3 alkyl group, or a five (5)-to six (6)- membered aromatic heterocyclic group.
[Embodiment D19] The compound D of the present invention wherein R^{1a} represents a phenyl group, or a five (5)- to six (6)- membered aromatic heterocyclic group.
[Embodiment D20] The compound D of the present invention wherein R^{3a} represents a C1-C3 alkyl group, or a halogen atom.
[Embodiment D21] The compound D of the present invention wherein R^{1a} represents a C2-C3 alkyl group, a phenyl group, or a five (5)- to six (6)- membered aromatic heterocyclic group, and R^{3a} represents a C1-C3 alkyl group, or a halogen atom.
[Embodiment D22] The compounds described in the Embodiments D1 to D4, D12 to D19, or the compound D of the present invention, wherein R^{3a} represents a C1-C3 alkyl group, or a halogen atom.
[Embodiment D23] The compounds described in the Embodiments D1 to D4, D12 to D19, or the compound D of the present invention, wherein R^{3a} represents a C1-C3 alkyl group.
[Embodiment D24] The compounds described in the Embodiments D1 to D4, D12 to D19, or the compound D of the present invention, wherein R^{3a} represents a halogen atom.
[Embodiment D25] A method for controlling soybean rust fungus which comprises applying the compound D of the present invention to soybeans or soil for cultivating the soybeans.
[Embodiment D26] A method for controlling soybean rust fungus which comprises applying the compound described in the Embodiment D21 to soybeans or soil for cultivating the soybeans.

Examples of the Embodiments of the compound D of the present invention include the following compounds.
[Embodiment C1] A compound represented by formula (IIA): 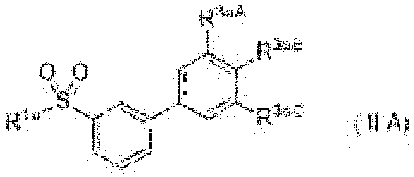 [wherein
   R^{1a} represents a C2-C3 alkyl group which may be optionally substituted with one or more halogen atoms, a cyclopropyl group which may be optionally substituted with one or more halogen atoms,
   R^{3aA} and R^{3aC} are identical to or different from each other, and each represents a C1-C3 alkyl group, a halogen atom, or a hydrogen atom, and R^{3aB} represents a C1-C3 alkyl group or a halogen atom.].
[Embodiment C2] The compound described in the Embodiment C1 wherein R^{3aA} and R^{3aC} are identical to or different from each other, and each represents a methyl group, a halogen atom, or a hydrogen atom, and R^{3aB} represents a methyl group or a halogen atom.
[Embodiment C3] The compound described in the Embodiment C1 wherein R^{3aA} and R^{3aC} are identical to or different from each other, and each represents a methyl group, a fluorine atom, or a hydrogen atom, and R^{3aB} represents a methyl group or a halogen atom.
[Embodiment C4] The compound described in the Embodiment C1 wherein R^{3aA} and R^{3aC} are identical to or different from each other, and each represents a methyl group, a fluorine atom, or a hydrogen atom, and R^{3aB} represents a methyl group, a fluorine atom, or a chlorine atom.
[Embodiment C5] The compound described in the Embodiment C1 wherein R^{3aA} and R^{3aC} are identical to or different from each other, and each represents a methyl group, a fluorine atom, or a hydrogen atom, and R^{3aB} represents a methyl group or a chlorine atom.
[Embodiment C6] The compound described in the Embodiment C1 wherein R^{3aA} and R^{3aC} are identical to or different from each other, and each represents a fluorine atom, or a hydrogen atom, and R^{3aB} represents a methyl group or a chlorine atom.
[Embodiment C7] The compound described in the Embodiment C1 wherein R^{3aA} represents a fluorine atom, or a hydrogen atom, R^{3aB} represents a methyl group or a chlorine atom, and R^{3aC} represents a hydrogen atom.
[Embodiment C8] The compound described in any one of the Embodiments C1 to C7 wherein R^{1a} represents a C2-C3 alkyl group which may be optionally substituted with one or more halogen atoms.
[Embodiment C9] The compound described in any one of the Embodiments C1 to C7 wherein R^{1a} represents an ethyl group or a propyl group.
[Embodiment C10] The compound described in any one of the Embodiments C1 to C7 wherein R^{1a} represents a cyclopropyl group.

Next, a process for preparing the present compound Y (including the compound of the present invention) is explained.

### Process A

A compound represented by formula (I-b) (hereinafter, referred to as "Compound (I-b)") or a compound represented by formula (I-c) (hereinafter, referred to as "Compound (I-c)") can be prepared by reacting a compound represented by formula (I-a) (hereinafter, referred to as "Compound (I-a)") with an oxidizing agent. 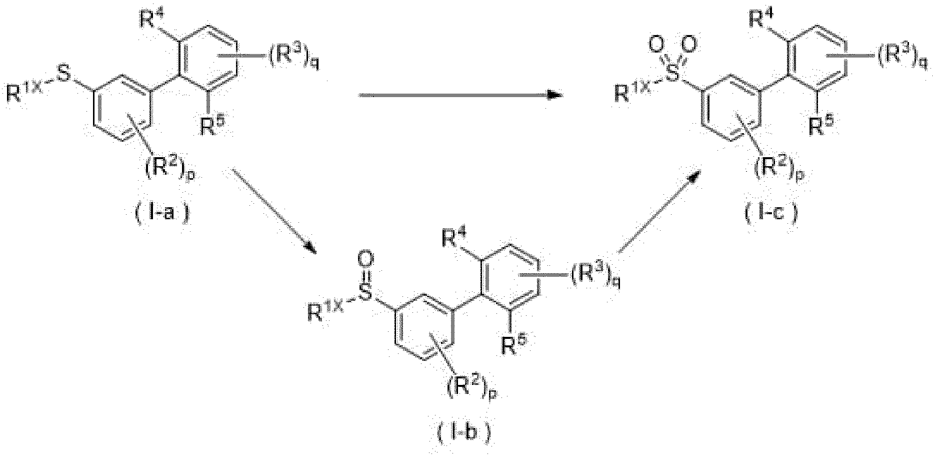 [wherein R^{1X} represents a C2-C6 chain hydrocarbon group which may optionally have one or more substituents selected from Group A, a C3-C8 alicyclic hydrocarbon group which may optionally have one or more substituents selected from Group B, a three (3)- to seven (7)- membered non-aromatic heterocyclic group which may optionally have one or more substituents selected from Group B, a methyl group which may optionally have one or more substituents selected from Group C, a C6-C10 aryl group, or a five (5)- to ten (10)- membered aromatic heterocyclic group {each of the C6-C10 aryl group, and the five (5)- to ten (10)- membered aromatic heterocyclic group may optionally have one or more substituents selected from Group E}, and the other symbols have the same definitions as the above.]

First, a process for preparing the compound (I-b) from the compound (I-a) is described.

The reaction is usually carried out in a solvent. Examples of the solvent to be used in the reaction include halogenated hydrocarbons such as dichloromethane, chloroform and chlorobenzene (hereinafter, collectively referred to as "halogenated hydrocarbons"); nitriles such as acetonitrile and propionitrile (hereinafter, collectively referred to as "nitriles"); alcohols such as methanol and ethanol (hereinafter, collectively referred to as "alcohols"); acetic acid; water; and mixed solvents comprising two or more of these solvents.

Examples of the oxidizing agent to be used in the reaction include sodium periodate, m-chloroperoxybenzoic acid (hereinafter, abbreviated as "mCPBA"), and hydrogen peroxide.

When a hydrogen peroxide is used as the oxidizing agent, if necessary, a base or a catalyst may be added.

Examples of the base to be used in the reaction include sodium carbonate. When the base is used in the reaction, the base is used usually within a range of 0.01 to 1 molar ratios, as opposed to 1 mole of the compound (I-a).

Examples of the catalysts to be used in the reaction include sodium tungstate.

When the catalyst is used in the reaction, the catalyst is used within a range of 0.01 to 0.5 molar ratios as opposed to 1 mole of the compound (I-a).

In the reaction, the oxidizing agent is used usually within a range of 1 to 1.2 molar ratio(s) as opposed to 1 mole of the compound (I-a).

The reaction temperature is usually within a range of -20 to 80°C. The reaction period of the reaction is usually within a range of 0.1 to 12 hours.

When the reaction is completed, water is added to the reaction mixtures, and the mixtures are extracted with organic solvent(s), and if necessary, the resulting organic layer is washed with an aqueous solution of a reducing agent (such as sodium sulfite, sodium thiosulfate) and an aqueous solution of a base (such as sodium hydrogen carbonate). The organic layers can be then dried and concentrated to obtain the compound (I-b).

Next, a process for preparing the compound (I-c) from the compound (I-b) is described.

The reaction is usually carried out in a solvent. Examples of the solvent to be used in the reaction include halogenated hydrocarbons, nitriles, alcohols, acetic acid, water, and mixed solvents comprising two or more of these solvents.

Examples of the oxidizing agent to be used in the reaction include mCPBA and hydrogen peroxide.

When the hydrogen peroxide is used as the oxidizing agent, if necessary, a base or a catalyst may be added.

Examples of the base to be used in the reaction include sodium carbonate.

When the base is used in the reaction, the base is used usually within a range of 0.01 to 1 molar ratio(s) as opposed to 1 mole of the compound (I-b).

Examples of the catalyst to be used in the reaction include sodium tungstate.

When the catalyst is used in the reaction, the catalyst is usually used within a range of 0.01 to 0.5 molar ratios as opposed to 1 mole of the compound (I-b).

In the reaction, the oxidizing agent is usually used within a range of 1 to 2 molar ratio(s) as opposed to 1 mole of the compound (I-b).

The reaction temperature is usually within a range of -20 to 120°C. The reaction period of the reaction is usually within a range of 0.1 to 12 hours.

When the reaction is completed, water is added to the reaction mixtures, and the mixtures are extracted with organic solvent(s), and if necessary, the resulting organic layer is washed with an aqueous solution of a reducing agent (such as sodium sulfite, sodium thiosulfate) and an aqueous solution of a base (such as sodium hydrogen carbonate). The organic layers can be then dried and concentrated to obtain the compound (I-c).

Also, the compound (I-c) can be prepared by reacting the compound (I-a) with an oxidizing agent in one step reaction (that is, one-pot reaction).

The reaction can be carried out by using the oxidizing agent within a ratio of 2 to 5 molar ratios as opposed to 1 mol of the compound (I-a) according to the process for preparing the compound (I-c) from the compound (I-b).

### Process B

The compound (I-a) can be prepared by reacting a compound represented by formula (B1) (hereinafter, referred to as "Compound (B1)") with a compound represented by formula (M1) (hereinafter, referred to as "Compound (M1)") in the presence of a base. 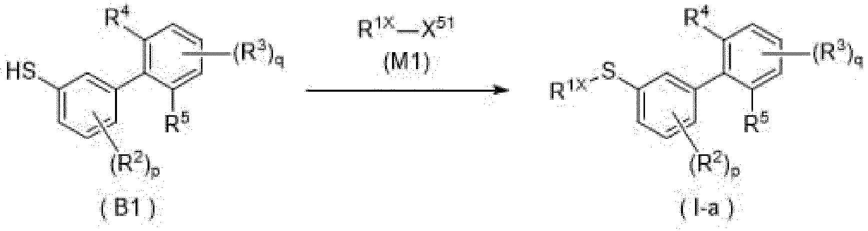 [wherein X⁵¹ represents a chlorine atom, a bromine atom, an iodine atom, a mesyloxy group, or a trifuryloxy group, and the other symbols have the same definitions as the above.]

The reaction is usually carried out in a solvent. Examples of the solvent to be used in the reaction include hydrocarbon such as hexane, toluene, xylene (hereinafter, collectively referred to as "hydrocarbons"); ethers such as methyl tert-butyl ether (hereinafter, referred to as "MTBE"), tetrahydrofuran (hereinafter, referred to as "THF"), and dimethoxyethane hereinafter, referred to as "DME") (hereinafter, collectively referred to as "ethers"); halogenated hydrocarbons; amides such as dimethylformamide (hereinafter, referred to as "DMF") and N-methyl pyrrolidone (hereinafter, referred to as "NMP") (hereinafter, collectively referred to as "amides"); esters such as methyl acetate and ethyl acetate (hereinafter, collectively referred to as "esters"); nitriles; water; and mixed solvents comprising two or more of these solvents.

Examples of the bases include organic bases such as triethylamine and pyridine (hereinafter, collectively referred to as "organic bases"); alkali metal carbonates such as sodium carbonate and potassium carbonate (hereinafter, collectively referred to as "alkali metal carbonates"); alkali metal hydrocarbonates such as sodium hydrogen carbonate, potassium hydrocarbonate (hereinafter, collectively referred to as "alkali metal hydrocarbonates"); sodium hydride and tripotassium phosphate.

If necessary, a metal catalyst, and/or a ligand may be used in the reaction.

Examples of the metal catalyst include copper catalysts (such as copper (I) iodide, copper (I) bromide, copper (I) chloride, copper (I) oxide, copper (I) trifluoromethane sulfonate benzene complex, tetrakis(acetonitrile) copper (I) hexafluorophosphate, copper (I) 2-thiophenecarboxylate; nickel catalysts (such as bis(cyclooctadiene) nickel (0), nickel(II) chloride); palladium catalysts (such as palladium (II) acetate, tetrakis(triphenylphosphine) palladium (0), and [1,1'-bis(diphenylphosphino)ferrocene palladium (II) dichloride]). When the metal catalyst is used in the reaction, the metal catalyst is usually used within a range of 0.01 to 1 molar ratio(s) as opposed to 1 mole of the compound (B1).

Examples of the ligand include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenlnyl, 1,2-bis (diphenylphosphino)ethane, 2,2'-bipyridine, 2-aminoethanol, 8-hydroxyqunoline, 1,10-phenanthroline, trans-1,2-cyclohexane diamine, trans-N,N'-dimethylcyclohexane-1,2-diamine, N,N'-dimethylethylenediamine, and N,N-dimethylglycine hydrochloride salt. When the ligand is used in the reaction, the ligand is usually used within a range of 0.01 to 1 molar ratio(s) as opposed to 1 mole of the compound (B1).

In the reaction, the compound (M1) is usually used within a range of 1 to 10 molar ratio(s), and the base is usualy used within a range of 1 to 10 molar ratio(s), as opposed to 1 mole of the compound (B1).

The reaction temperature in the reaction is usually within a range of 0 to 150°C. The reaction period in the reaction is usually within a range of 0.1 to 48 hours.

When the reaction is completed, water is added to the reaction mixture, and the resulting mixture was extracted with organic solvent(s), and the organic layer is worked up (for example, drying and concentration) to isolate the compound (I-a).

The compound (M1) is publicly known, or can be prepared according to a publicly known method.

### Process C

The compound represented by formula (I) can be prepared by reacting a compound represented by formula (B2) (hereinafter, referred to as "Compound (B2)") with a compound represented by formula (M2) (hereinafter, referred to as "Compound (M2)") in the presence of a palladium catalyst and a base. 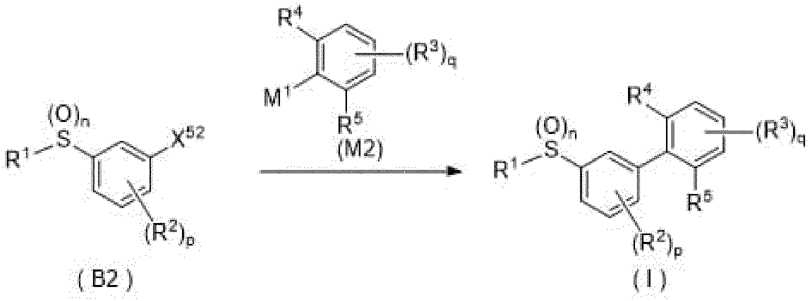 [wherein X⁵² represents a chlorine atom, a bromine atom, an iodine atom, and a trifuryloxy group, M¹ represents B(OH)₂, or 4,4,5,5-tetamethyl-1,3,2-dioxaborolan-2-yl group, and the other symbols have the same definitions as the above.]

The reaction is carried out in a solvent. Examples of the solvent to be used in the reaction include hydrocarbons, ethers, halogenated hydrocarbons, amides, esters, nitriles, dimethyl sulfoxide (hereinafter, referred to as "DMSO"), water, and mixed solvents of two or more of these solvents.

Examples of the palladium catalyst to be used in the reaction include palladium (II) acetate, and [1,1'-bis(diphenylphosphino)ferrocene] palladium (II) dichloride.

Examples of the base to be used in the reaction include organic bases, alkali metal carbonates, alkali metal hydrocarbonates, sodium fluoride, and tripotassium phosphate.

In the reaction, the compound (M) is usually used within a range of 0.5 to 2 molar ratios, and the palladium catalyst is usually used within a range of 0.01 to 0.3 molar ratios, and the base is usually used within a range of 1 to 10 molar ratio(s), as opposed to 1 mole of the compound (B2).

The reaction temperature in the reaction is usually within a range of 0 to 150°C. The reaction period in the reaction is usually within a range of 0.1 to 120 hours.

When the reaction is completed, water is added to the reaction mixture, and the organic layer is worked up (for exmaple, drying and concentration) to isolate the compound represented by formula (I).

The compound (M2) is publicly known, or can be prepared according to a publicly known method.

### Process D

The compound represented by formula (I) can be prepared by reacting a compound represented by formula (B3) (hereinafter, referred to as "Compound (B3)") with a compound represented by formula (M3) (hereinafter, referred to as "Compound (M3)") in the presence of a palladium catalyst and a base. 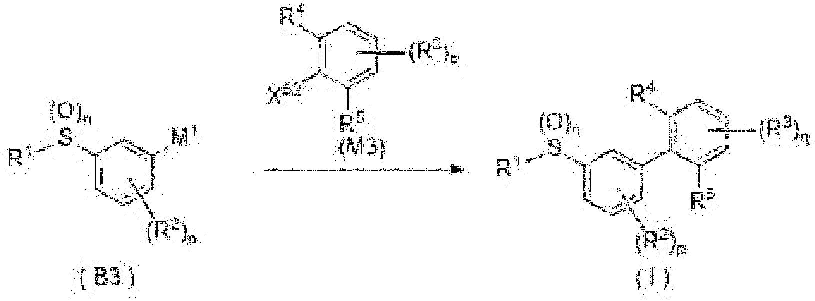 [wherein the symbols have the same definitions as the above.]

The reaction can be carried out by using the compound (B3) in place of the compound (B2) and using the compound (M3) in place of the compound (M2) according to the process C.

The compound (M3) is publicly known, or can be prepared according to a publicly known method.

### Process E

The compound (I-a) can be prepared by reacting a compound represented by formula (B4) (hereinafter, referred to as "Compound (B4)") with a compound represented by formula (M4) (hereinafter, referred to as "Compound (M4)") in the presence of a base. 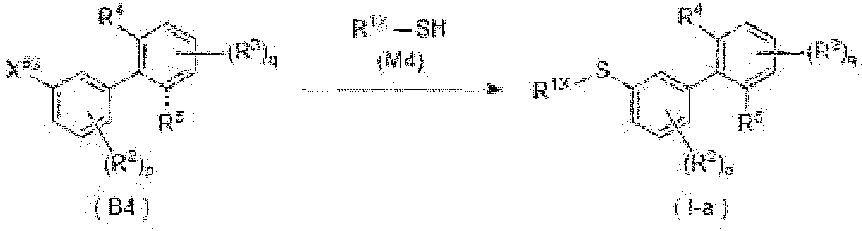 [wherein X⁵³ represents a fluorine atom, a chlorine atom, a bromine atom, an iodine atom, a mesyloxy group or a trifuryloxy group, and the other symbols have the same definitions as the above.]

The reaction is usually carried in a solvent. Examples of the solvent to be used in the reaction include alcohols, nitriles, ethers, aromatic hydrocarbons, amides, water, and mixed solvents comprising two or more of these solvents.

Examples of the base to be used in the reaction include alkali metal hydrides, alkali metal carbonates, organic bases, sodium hydride, and tripotassium phosphate.

When X⁵³ represents a bromine atom, an iodine atom, or a trifuryloxy group, if necessary, a metal catalyst and a ligand may be added.

Examples of the metal catalyst to be used in the reaction include palladium catalysts such as palladium (II) acetate, and [1,1'-bis(diphenylphosphino)ferrocene] palladium (II) dichloride); nickel catalysts such as bis(cyclooctadiene)nickel (0) and nickel (II) chloride); and copper catalysts such as copper (I) iodide, and copper (I) chloride. When the metal catalyst is used in the reaction, the metal catalyst is usually used within a range of 0.01 to 1 molar(s) as opposed to 1 mole of the compound (B4).

Examples of the ligand to be used in the reaction include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenlnyl, 1,2-bis (diphenylphosphino)ethane, 2,2'-bipyridine, 2-aminoethanol, 8-hydroxyqunoline, and 1,10-phenanthroline.

When the ligand is used in the reaction, the ligand is usually used within a range of 0.01 to 1 molar ratio(s) as opposed to 1 mole of the compound (B4).

In the reaction, the compound (M4) is usualy used within a range of 1 to 10 molar ratio(s), and the base is usually used within a range of 1 to 10 molar(s), as opposed to 1 mole of the compound (B4).

The reaction temperature in the reaction is usually within a range of -20 to 200°C. The reaction period in the reaction is usually within a range of 0.1 to 72 hours.

When the reaction is completed, water is added to the reaction mixture, and the organic layer is extracted with organic solvent(s), and the organic layer is worked up (for example, drying and concentration) to obtain the compound (I-a).

The compound is publicly known, or can be prepared according to a publicly known method.

### Process F

A compound represented by formula (I-e) (hereinafter, referred to as "Compound (I-e)") can be prepared by reacting a compound represented by formula (B6) (hereinafter, referred to as "Compound (B6)") with a compound represented by formula (M6) (hereinafter, referred to as "Compound (M6)") in the presence of base. 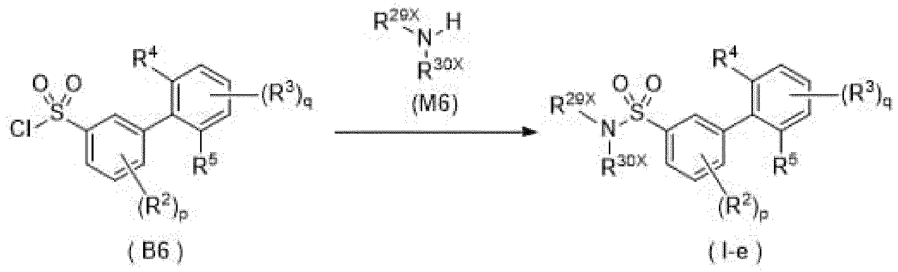 [wherein R^{29X} represents a C1-C6 chain hydrocarbon group which may be optionally substituted with one or more halogen atoms, or a hydrogen atom, R^{30X} represents a C1-C6 chain hydrocarbon group which may be optionally substituted with one or more halogen atoms, R^{29X} and R^{30X} may combine together with a nitrogen atom to which they are attached to form a three (3)- to seven (7)- membered non-aromatic heterocyclic group which may optionally have one or more substituents selected from Group B, or a five (5)- to ten (10)- membered aromatic heterocyclic ring which may optionally have one or more substituents selected from Group E, and the other symbols have the same definitions as the above.]

The reaction is usually carried out in a solvent. Examples of the solvents to be used in the reaction include hydrocarbons, ethers, halogenated hydrocarbons, amides, esters, nitriles, water, and mixed solvents of two or more of these solvents.

Examples of the base to be used in the reaction include organic bases, alkali metal carbonates, alkali metal hydrocarbonates, sodium hydride, and tripotassium phosphate.

In the reaction, the compound (M6) is usually used within a range of 1 to 10 molar ratio(s), and the bae is usually used within a range of 1 to 10 molar ratio(s), as opposed to 1 mole of the compound (B6).

The reaction temperature in the reaction is usually within a range of 0 to 150°C. The reaction period in the reaction is usually within a range of 0.1 to 48 hours.

When the reaction is completed, water is added to the reaction mixture, and the resulting mixture is extracted with organic solvent(s), and the organic layer is worked up (for example, drying and concentration) to isolate the compound (I-e).

The compound (M6) is publicly known, or can be prepared according to a publicly known method.

### Reference Process A

The compound (B1) can be prepared by reacting the compound (B4) with a sulfurizing agent. 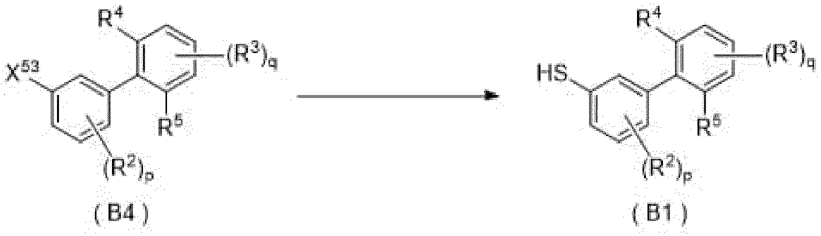 [wherein the symbols have the same definitions as the above.]

The reaction can be carried out by using the sulfurizing agent (such as hydrogen sulfide) in place of the compound (M4) according to the process E.

### Reference Process B

The compound (B4) can be prepared by reacting a compound represented by formula (C1) (hereinafter, referred to as "Compound (C1)") with the compound (M2) in the presence of a palladium catalyst and a base. 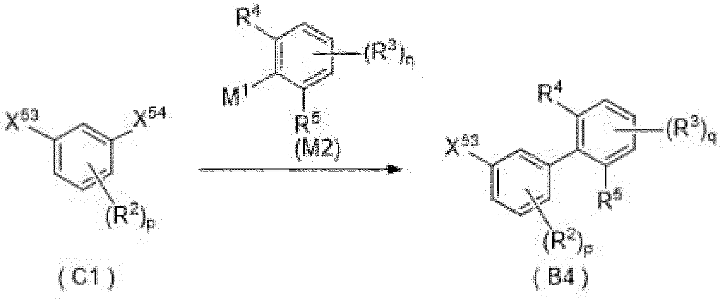 [wherein X⁵⁴ represents a bromine atom, an iodine atom or a trifuryloxy group, and the other symbols have the same definitions as the above.]

The reaction can be carried out by using the compound (C1) in place of the compound (B2) according to the process C.

The compound (C1) is publicly known, or can be prepared according to a publicly known method.

### Reference process C

A compound represented by formula (C3) (hereinafter, referred to as "Compound (C3)") can be prepared by reacting a compound represented by formula (C2) (hereinafter, referred to as "Compound (C2)") with the compound (M1) in the presence of a base. 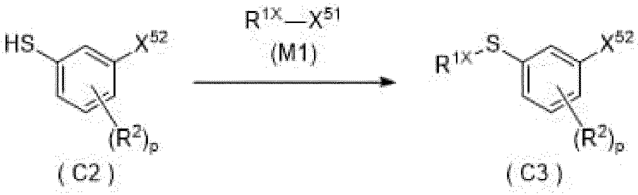 [wherein the symbols have the same definitions as the above.]

The reaction can be carried out by using the compound (C2) in place of the compound (B1) according to the process B.

The compound (C2) is publicly known, or can be prepared according to a publicly known method.

### Reference process D

A compound represented by formula (C4) (hereinafter, referred to as "Compound (C4)") or a compound represented by formula (C5) (hereinafter, referred to as "Compound (C5)") can be prepared by reacting the compound (C3) with an oxidizing agent. 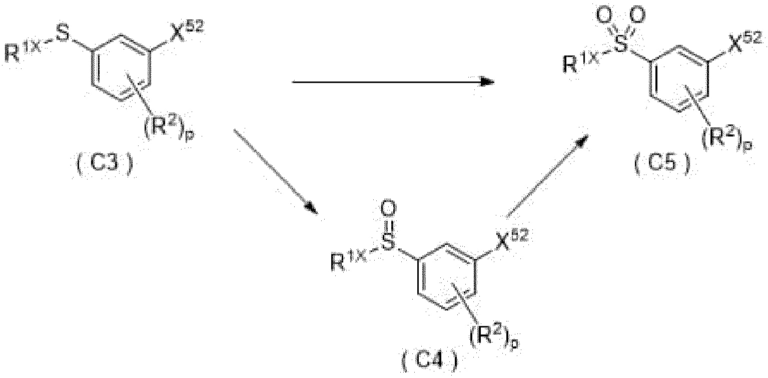 [wherein the symbols have the same definitions as the above.]

The reaction can be carried out by using the compound (C3) in place of the compound (I-a) and using the compound (C4) in place of the compound (I-b) according to the process A.

### Reference process E

A compound represented by formula (C6) (hereinafter, referred to as "Compound (C6)") with bis(pinacolato)diboron in the presence of a base and a palladium catalyst. 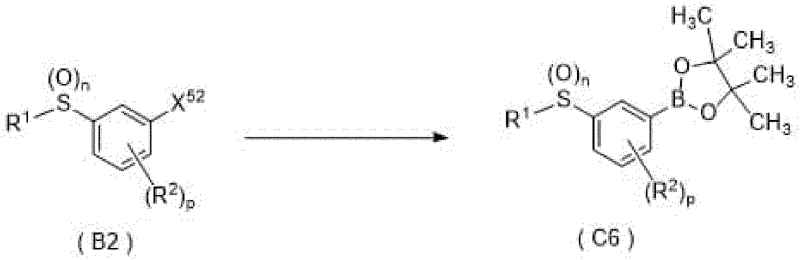 [wherein the symbols have the same definitions as the above.]

The reaction can be carried out by using bis(pinacolato)diboron in place of the compound (M2) according to the process C.

### Reference Process F

The compound (B6) can be prepared from a compound represented by formula (C10) (hereinafter, referred to as "Compound (C10)"). 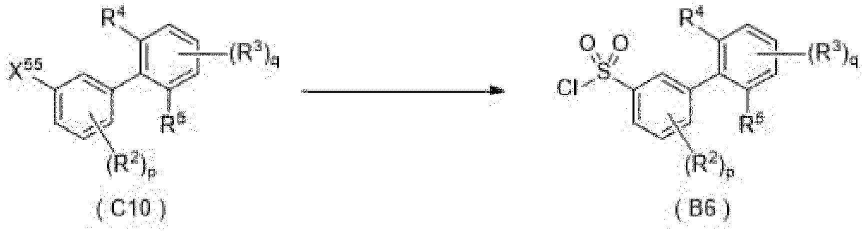 [wherein X⁵⁵ represents SH or S(O)₂OH, and the other symbols have the same definitions as the above.]

The reaction can be carried out according to the method described in WO 2016/204096 or Tetrahedron Letters, 2017, 58, 2244-2247.

The compound (C10) is publicly known, or can be prepared according to a publicly known method.

The compound of the present invention can be mixed or combined with one or more ingredients selected from the group consisting of the following Group (a), Group (b), Group (c), and Group (d) (hereinafter, referred to as "Present ingredient").

The above-described defined mixing or combining represents that the compound of the present invention and the present ingredient are used concurrently, separately, or at an interval.

When the compound of the present invention and the present ingredient are concurrently used, the compound of the present invention and the present ingredient may be incorporated as a separate formulation or one formulation.

One aspect of the present invention relates to a composition comprising one or more ingredients selected from the group consisting of the Group (a), the Group (b), the Group (c) and the Group (d) (that is, the present ingredient) as well as the compound of the present invention.

One aspect of the present invention relates to a composition comprising one or more ingredients selected from the group consisting of the Group (a), the Group (b), the Group (c) and the Group (d) (that is, the present ingredient) as well as the compound of the present invention (hereinafter, referred to as "Composition A").

The Group (a) represents insecticidal active ingredients, miticidal active ingredients and nematicidal active ingredients that are a group consisting of acetylcholinesterase inhibitors (for example, carbamate insecticides and organophosphate insecticides), GABA-gated chloride ion channel blockers (for example, phenylpyrazole insecticides), sodium channel modulators (for example, pyrethroid insecticides), nicotinic acetylcholine receptor antagonist modulators (for example, neonicotinoid insecticides), nicotinic acetylcholine receptor allosteric modulators, glutamate-gated chloride ion channel competitive modulators (for example, macrolide insecticides), juvenile hormone mimics, multisite inhibitors, chordotonal organ TRPV channel modulators, mites growth regulators, microbial disruptors of insect midgut membranes, mitochondrial ATP synthase inhibitors, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, Inhibitors of mitochondrial electron transport chain complex I, II, III, and IV, voltage-dependent sodium channel blockers, Inhibitors of acetyl CoA carboxylase, ryanodine receptor modulators (for example, diamide insecticides), chordotonal organ modulators, microbial fungicides, as well as other insecticidal active ingredients, miticidal active ingredients and nematicidal active ingredients. These agents are described in the classification based on the IRAC mode of action.

The Group (b) is a group consisting of nucleic acid synthesis inhibitors (for example, phenylamide fungicides and acylamino acid fungicides), cytostatic and cytoskeletal inhibitors (for example, MBC fungicides), respiration inhibitors (for example, QoI fungicides and QiI fungicides), amino-acid synthesis and protein synthesis inhibitors (for example, anilinopyridine fungicides), signal-transduction inhibitors, lipid synthesis and membrane synthesis inhibitors, sterol biosynthesis inhibitors (for example, DMI fungicides such as triazoles), cell wall synthesis inhibitors, melanin synthesis inhibitors, plant defense inducer, multisite fungicides, microbial fungicides, and other fungicidal active ingredients. These agents are described in the classification based on the FRAC mode of action.

The Group (c) represents a group of plant growth modulating ingredients (including mycorrhizal fungus and rhizobia).

The Group (d) represents a group of repellent active ingredients.

Examples of combinations of the present ingredient and the compound of the present invention are recited as follows. For example, the "alanycarb + SX" indicates a combination of alanycarb and SX. The abbreviation "SX" means to any one of the compounds of the present invention selected from SX1 to SX54. Further, all of the present ingredients as described below are known ingredients, and can be obtained from commercially available formulations or prepared by known methods. When the present ingredient represents a microorganism, the present ingredient can be obtained from a microorganism depositary authority. The number in parentheses represents CAS RN (registered trademark).

A combination of the present ingredient in the above-mentioned Group (a) and the compound of the present invention:
abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat) + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC(2-methyl-4,6-dinitrophenol) + SX, doramectin + SX, dried leaves of Dryopteris filix-mas + SX, emamectin-benzoate + SX, empenthrin + SX, endosulfan + SX, EPN(O-ethyl-O-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, flupyroxystrobin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hv1a peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, ledprona + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, 2-({2-fluoro-4-methyl-5-[(2,2,2-trifluoroethyl)sulfinyl]phenyl}imino)-3-(2,2,2-trifluoroethyl)-1,3-thiazolidin-4-one (1445683-71-5) + SX, (2Z)-2-({2-fluoro-4-methyl-5-[(R)-(2,2,2-trifluoroethyl)sulfinyl]phenyl}imino)-3-(2,2,2-trifluoroethyl)-1,3-thiazolidin-4-one (2377084-09-6) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, 1,4-dimethyl-2-[2-(3-pyridinyl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, BT crop protein Cry1Ab + SX, BT crop protein Cry1Ac + SX, BT crop protein Cry1Fa + SX, BT crop protein Cry1A.105 + SX, BT crop protein Cry2Ab + SX, BT crop protein Vip3A + SX, BT crop protein mCry3A + SX, BT crop protein Cry3Ab + SX, BT crop protein Cry3Bb + SX, BT crop protein Cry34Ab1/Cry35Ab1 + SX, Adoxophyes orana granulosis virus strain BV-0001 + SX, Anticarsia gemmatalis mNPV + SX, Autographa californica mNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua mNPV + SX, Spodoptera littoralis mNPV + SX, Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306) + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, and Wolbachia pipientis + SX.

A combination of the present ingredient in the above-mentioned Group (b) and the compound of the present invention:
acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper (II) acetate + SX, copper (II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Melaleuca alternifolia + SX, extract of Reynoutria sachalinensis + SX, extract of the cotyledons of lupine plantlets ("BLAD") + SX, extract of Allium sativum + SX, extract of Equisetum arvense + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin +SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl]ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({4-[5-(trifluoromethyl)-1,2,4-oxadiazol-3-yl]phenyl}methyl)urea + SX, N,N-diethyl-N'-({4-[5-(trifluoromethyl)-1,2,4-oxadiazol-3-yl]phenyl}methyl)urea + SX, N-methyl-N'-({4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl) (2,6-difluoropyridin-4-yl)amino]-N-(2,2-dimethylcyclobutyl)-5-methylthiazole-4-carboxamide + SX, 2-[(2-methylpropanoyl) (2,6-difluoropyridin-4-yl)amino]-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2-methylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2,2-dimethylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(5-fluoro-2-methoxyphenyl)-2-(2-cyanoethoxy)ethyl]-5-[1-(isopropoxyimino)ethyl]-2,6-dioxo-3,6-dihydropyrimidin-1(2H)-yl}propan-2-yl)-2-methylpropanamide + SX, Agrobacterium radiobactor strain K1026 + SX, Agrobacterium radiobactor strain K84 + SX, Bacillus amyloliquefaciens strain PTA-4838 (Aveo (trade mark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, and Harpin protein + SX.

A combination of the present ingredient in the above-mentioned Group (c) and the compound of the present invention:
1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufenethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butyic acid + SX, methyl [5-(trifluoromethyl)benzo[b]thiophen-2-carboxylate + SX, and 3-[(6-chloro-4-phenylquinazolin-2-yl)amino)propane-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, and Zucchini Yellow Mosaik Virus weak strain + SX.

A combination of the present ingredient in the above-mentioned Group (d) and the compound of the present invention:
anthraquinone + SX, deet + SX, and icaridin + SX.

Examples of a ratio of the compound of the present invention to the present ingredient include, but are not particularly limited to 1000 : 1 to 1 : 1000, 500 : 1 to 1 : 500, 100 : 1 to 1 : 100, 50 : 1, 20 : 1, 10 : 1, 9 : 1, 8 : 1, 7 : 1, 6 : 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9, 1 : 10, 1 : 20, and 1 : 50 in the weight ratio (the compound of the present invention : the present ingredient).

The present compound, the present compound **Y,** the compound of the present invention, or the composition A can control plant diseases caused by phytopathogenic microorganisms (such as fungi, Oomycete, Phytomyxea, and bacteria). Examples of the fungi include Ascomycota, Basidiomycota, Blasocladiomycota, Chytridiomycota, Mucoromycota, and Olpidiomycota. Specific examples of plant diseases include the followings. The descriptions in a parenthesis indicates an academic name of phytopathogenic microorganism that causes each of the disease.

### Rice diseases:

blast (Pyricularia oryzae), brown spot (Cochliobolus miyabeanus), sheath blight (Rhizoctonia solani), bakanae disease (Gibberella fujikuroi), downy mildew (Sclerophthora macrospora), false blast and head blight (Epicoccum nigrum), seedling blight (Trichoderma viride, Rhizopus oryzae), bordered sheath spot (Waitea circinata), brown sclerotium disease (Ceratobasidium setariae), and brown sheath blight (Thanatephorus cucumeris);

### Wheat diseases:

powdery mildew (Blumeria graminis), fusarium blight (Fusarium graminearum, Fusarium avenaceum, Fusarium culmorum, Microdochium nivale), stripe rust (Puccinia striiformis), stem rust (Puccinia graminis), leaf rust (Puccinia recondita), snow mould (Microdochium nivale, Microdochium majus), typhula snow blight (Typhula incarnata, Typhula ishikariensis), Sclerotinia snow blight (Sclerotinia borealis), Pythium snow blight (Pythium spp.), loose smut (Ustilago tritici), stinking smut (Tilletia caries, Tilletia controversa), eyespot (Pseudocercosporella herpotrichoides), leaf blotch (Septoria tritici), glume blotch (Stagonospora nodorum), tan spot (Pyrenophora tritici-repentis), rhizoctonia seeding blight (Rhizoctonia solani), take-all disease (Gaeumannomyces graminis), and blast (Pyricularia graminis-tritici);

### Barley diseases:

powdery mildew (Blumeria graminis), fusarium head blight (Fusarium graminearum, Fusarium avenaceum, Fusarium culmorum, Microdochium nivale), stripe rust (Puccinia striiformis), stem rust (Puccinia graminis), dwarf leaf rust (Puccinia hordei), loose smut (Ustilago nuda), scald (Rhynchosporium secalis), net blotch (Pyrenophora teres), spot blotch (Cochliobolus sativus), stripe (Pyrenophora graminea), Ramularia disease (Ramularia collo-cygni), and rhizoctonia seeding blight (Rhizoctonia solani);

### Corn diseases:

rust (Puccinia sorghi), southern rust (Puccinia polysora), northern leaf blight (Setosphaeria turcica), tropical rust (Physopella zeae), southern leaf blight (Cochliobolus heterostrophus), anthracnose (Colletotrichum graminicola), gray leaf spot (Cercospora zeae-maydis), eyespot (Kabatiella zeae), phaeosphaeria leaf spot (Phaeosphaeria maydis), diplodia rot (Stenocarpella maydis, Stenocarpella macrospora), stalk rot (Fusarium graminearum, Fusarium verticilioides, Colletotrichum graminicola), smut (Ustilago maydis), and Physoderma brown spot and Physoderma stalk rot (Physoderma maydis);

### Cotton diseases:

anthracnose (Colletotrichum gossypii), grey mildew (Ramularia areola), alternaria leaf spot (Alternaria macrospora, Alternaria gossypii), and black root rot (Thielaviopsis basicola);

### Coffee diseases:

rust (Hemileia vastatrix), and leaf spot (Cercospora coffeicola);

### Rape seed diseases:

sclerotinia rot (Sclerotinia sclerotiorum), gray leaf spot (Alternaria brassicae), root rot (Phoma lingam), and light leaf spot (Pyrenopeziza brassicae);

### Sugar cane diseases:

rust (Puccinia melanocephela, Puccinia kuehnii), and smut (Ustilago scitaminea);

### Sunflower diseases:

rust (Puccinia helianthi), and downy mildew (Plasmopara halstedii);

### Citrus diseases:

melanose (Diaporthe citri), scab (Elsinoe fawcetti), green mold (Penicillium digitatum), blue mold (Penicillium italicum), Phytophthora rot (Phytophthora parasitica, Phytophthora citrophthora), and aspergillus rot (Aspergillus niger) ;

### Apple diseases:

blossom blight (Monilinia mali), valsa canker (Valsa ceratosperma), powdery mildew (Podosphaera leucotricha), alternaria leaf spot (Alternaria alternata apple pathotype), scab (Venturia inaequalis), bitter rot (Glomerella cingulata, Colletotrichum acutatum), blotch (Diplocarpon mali), ring rot (Botryosphaeria berengeriana), crown rot (Phytophtora cactorum), and rust (Gymnosporangium juniperi-virginianae, Gymnosporangium yamadae);

### Pear diseases:

scab (Venturia nashicola, Venturia pirina), black spot (Alternaria alternata Japanese pear pathotype), and rust (Gymnosporangium haraeanum);

### Peach diseases:

brown rot (Monilinia fructicola), scab (Cladosporium carpophilum), Phomopsis rot (Phomopsis sp.), and leaf curl (Taphrina deformans);

### Grapes diseases:

anthracnose (Elsinoe ampelina), ripe rot (Glomerella cingulata, Colletotrichum acutatum), powdery mildew (Uncinula necator), rust (Phakopsora ampelopsidis), black rot (Guignardia bidwellii), and downy mildew (Plasmopara viticola);

### Japanese persimmon diseases:

anthracnose (Gloeosporium kaki, Colletotrichum acutatum), and leaf spot (Cercospora kaki, Mycosphaerella nawae);

### Diseases of gourd family:

anthracnose (Colletotrichum lagenarium), powdery mildew (Sphaerotheca fuliginea), gummy stem blight (Didymella bryoniae), Corynespora leaf spot (Corynespora cassiicola), fusarium wilt (Fusarium oxysporum), downy mildew (Pseudoperonospora cubensis), phytophthora rot (Phytophthora capsici), damping-off (Pythium sp.), and Phomopsis root rot (Phomopsis sp.);

### Tomato diseases:

early blight (Alternaria solani), leaf mold (Cladosporium fulvum), Cercospora leaf mold (Pseudocercospora fuligena), late blight (Phytophthora infestans), and powdery mildew (Leveillula taurica);

### Eggplant diseases:

brown spot (Phomopsis vexans), and powdery mildew (Erysiphe cichoracearum);

### Cruciferous vegetables diseases:

alternaria leaf spot (Alternaria japonica), white spot (Cercosporella brassicae), clubroot (Plasmodiophora brassicae), downy mildew (Peronospora parasitica), and white rust (Albugo candida);

Welsh onion disease:

rust (Puccinia allii), alternaria leaf spot (Alternaria porri), white rot (Sclerotium cepivorum), and neck rot (Botrytis squamosa);

### Soybean diseases:

purple stain (Cercospora kikuchii), sphaceloma scab (Elsinoe glycines), pod and stem blight (Diaporthe phaseolorum var. sojae), rust (Phakopsora pachyrhizi), target spot (Corynespora cassiicola), anthracnose (Colletotrichum glycines, Colletotrichum truncatum), Rhizoctonia rot (Rhizoctonia solani), septoria brown spot (Septoria glycines), Cercospora leaf spot (Cercospora sojina), stem rot (Sclerotinia sclerotiorum), powdery mildew (Microsphaera diffusa), phytophthora stem and root rot (Phytophthora sojae), downy mildew (Peronospora manshurica), sudden death syndrome (Fusarium virguliforme), red crown rot (Calonectria ilicicola), and Diaporthe/Phomopsis complex (Diaporthe longicolla);

### Kidney bean diseases:

stem rot (Sclerotinia sclerotiorum), rust (Uromyces appendiculatus), angular leaf spot (Phaeoisariopsis griseola), and anthracnose (Colletotrichum lindemuthianum), and Fusarium root-rot (Fusarium solani);

### Peanut diseases:

leaf spot (Cercospora personata), brown leaf spot (Cercospora arachidicola), southern blight (Sclerotium rolfsii), and Cylindrocladium black rot (Calonectria ilicicola);

### Garden pea disease:

powdery mildew (Erysiphe pisi), and root rot (Fusarium solani);

### Potato diseases:

early blight (Alternaria solani), late blight (Phytophthora infestans), Pink rot (Phytophthora erythroseptica), powdery scab (Spongospora subterranea f. sp. subterranea), verticillium wilt (Verticillium albo-atrum, Verticillium dahliae, Verticillium nigrescens), dry rot (Fusarium solani), and potato wart (Synchytrium endobioticum);

### Strawberry disease:

powdery mildew (Sphaerotheca humuli), and strawberry anthracnose (Glomerella cingulata, Colletotrichum acutatum);

### Tea diseases:

net blister blight (Exobasidium reticulatum), white scab (Elsinoe leucospila), gray blight (Pestalotiopsis sp.), and anthracnose (Colletotrichum theae-sinensis);

### Tobacco diseases:

brown spot (Alternaria longipes), anthracnose (Colletotrichum tabacum), blue mold (Peronospora tabacina), and black shank (Phytophthora nicotianae);

### Sugar beet diseases:

cercospora leaf spot (Cercospora beticola), leaf blight (Thanatephorus cucumeris), root rot (Thanatephorus cucumeris), and aphanomyces root rot (Aphanomyces cochlioides), and rust (Uromyces betae);

### Rose diseases:

black spot (Diplocarpon rosae), and powdery mildew (Sphaerotheca pannosa);

### Chrysanthemum diseases:

leaf blight (Septoria chrysanthemi-indici), and white rust (Puccinia horiana);

### Onion diseases:

botrytis leaf blight (Botrytis cinerea, Botrytis byssoidea, Botrytis squamosa), gray-mold neck rot (Botrytis allii), and small sclerotial neck rot (Botrytis squamosa);

### Various crops diseases:

Botrytis rot (Botrytis cinerea), sclerotinia rot (Sclerotinia sclerotiorum), seedling blight (Pythium aphanidermatum, Pythium irregulare, Pythium ultimum);

### Japanese radish disease:

alternaria leaf spot (Alternaria brassicicola);

### Turfgrass diseases:

dollar spot (Sclerotinia homoeocarpa), brown patch and large patch (Rhizoctonia solani), and pythium bligt (Pythium aphanidermatum);

### Banana disease:

Sigatoka disease (Mycosphaerella fijiensis, Mycosphaerella musicola);

### Lentils disease:

ascochyta blight (Ascochyta lentis);

### Chickpea disease:

ascochyta blight (Ascochyta rabiei);

### Green pepper disease:

anthracnose (Colletotrichum scovillei);

### Mango disease:

anthracnose (Colletotrichum acutatum);

### Sweet potato disease:

foot rot (Diaporthe destruens);

### Fruit trees diseases:

white root rot (Rosellinia necatrix), and violet root rot (Helicobasidium mompa);

### Postharvest disease of fruits (for example, apple and pear):

Mucor rot diseases (Mucor piriformis);

Seed diseases or diseases in the early stages of the growth of various plants caused by Aspergillus spp., Penicillium spp., Fusarium spp., Gibberella spp., Tricoderma spp., Thielaviopsis spp., Rhizopus spp., Mucor spp., Corticium spp., Phoma spp., Rhizoctonia spp. or Diplodia spp.; and the like;

### Viral diseases:

Lettuce big-vein disease transmitted by Olpidium brassicae, and viral diseases of several crops transmitted by Polymixa spp. (e.g. Polymyxa betae and Polymyxa graminis);

### Diseases caused by bacteria:

bacterial seedling blight of rice (Burkholderia plantarii), bacterial palea browning of rice (Pantoea ananatis), bacterial leaf blight of rice (Xanthomonas oryzae pv. oryzae.), bacterial spot of cucumber (Pseudomonas syringae pv. lachrymans), bacterial wilt of eggplant (Ralstonia solanacearum), canker of citrus (Xanthomonas citri), bacterial soft rot of Chinese cabbage (Erwinia carotovora), scab of potato (Streptomyces scabiei), Goss's wilt of corn (Clavibacter michiganensis), Pierce's disease of grapes, olive and peach (Xylella fastidiosa), andcrown gall of Rosaceae plants such as apple, peach, cherries (Agrobacterium tumefaciens).

The term of "soybean rust fungus having an amino acid substitution of F129L on mitochondrial cytochrome b protein" represents soybean rust fungus (scientific name: *Phakopsora pachyrhizi*) which shows a resistance against QoI fungicide by having a mutation in the mitochondrial cytochrome b gene encoding mitochondrial cytochrome protein and as a result of the mutation, causing amino acid substitution of F129L.

The method for controlling plant diseases of the present invention is carried out by applying an effective amount of the present compound, the present compound **Y,** the compound of the present invention, or the composition A to a plant or soil for cultivating a plant. Examples of the application method include foliar application, soil application, and seed application.

The present compound, the present compound **Y,** the compound of the present invention, or the composition A is usually used by mixing it with inert carrier (s) such as solid carrier(s) and liquid carrier(s), and surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, or the like. In addition to these formulations, the Present compound or the Composition A may be used by formulating it into a dosage form described in Manual on development and use of FAO and WHO Specifications for pesticides, FAO Plant Production and Protection Papers-271-276, prepared by the FAO/WHO Joint Meeting on Pesticide Specifications, 2016, ISSN:0259-2517.

These formulations usually comprise 0.0001 to 99% by weight ratio of the present compound, the present compound Y, the compound of the present invention, or the composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Moreover, some adjuvants can be employed to improve or help the efficacy of the present compound, the present compound Y, the compound of the present invention, or the composition A. The specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark).

The plant as used herein include whole plant, and a certain part of the plant. Examples of the certain part of the plant include stem and leaf, flower, ear, fruit, tree stem, branch, crown, seed, vegetative reproductive organ, and seedling.

A vegetative reproduction organ means a part of plant such as root, stem, and leaf which has a growth capability even when said part is separated from the plant body and placed into soil. Examples of the vegetative reproduction organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

The seedling as used herein include a seedling and a sapling.

Examples of a method for controlling plant diseases by applying an effective amount of the present compound, the present compound Y, the compound of the present invention, or the composition A to soils include a method of applying an effective amount of the present compound, the present compound Y, the compound of the present invention, or the composition A to soils before planting plants or after planting plants. Specific examples of the application method include planting hole treatment (spraying into planting holes, soil mixing after planting hole treatment), plant foot treatment (plant foot spraying, soil mixing after plant foot treatment, irrigation at plant foot, plant foot treatment at a later seeding raising stage), planting furrow treatment (planting furrow spraying, soil mixing after planting furrow treatment), planting row treatment (planting row spraying, soil mixing after planting row treatment, planting row spraying at a growing stage), planting row treatment at the time of sowing (planting row spraying at the time of sowing, soil mixing after planting row treatment at the time of sowing), broadcast treatment (overall soil surface spraying, soil mixing after broadcast treatment), side-article treatment, treatment of water surface (application to water surface, application to water surface after flooding), other soil spraying treatment (spraying of a granular formulation on leaves at a growing stage, spraying under a canopy or around a tree stem, spraying on the soil surface, mixing with surface soil, spraying into seed holes, spraying on the ground surfaces of furrows, spraying between plants), other irrigation treatment (soil irrigation, irrigation at a seedling raising stage, chemical solution injection treatment, irrigation of a plant part just above the ground, chemical solution drip irrigation, chemigation), seedling raising box treatment (spraying into a seedling raising box, irrigation of a seedling raising box, flooding into a seedling raising box with chemical solution), seedling raising tray treatment (spraying on a seedling raising tray, irrigation of a seedling raising tray, flooding into a seedling raising tray with chemical solution), seedbed treatment (spraying on a seedbed, irrigation of a seedbed, spraying on a lowland rice nursery, immersion of seedlings), seedbed soil incorporation treatment (mixing with seedbed soil, mixing with seedbed soil before sowing, spraying at sowing before covering with soils, spraying at sowing after covering with soils, mixing with covering with soils), and other treatment (mixing with culture soil, plowing under, mixing with surface soil, mixing with soil at the place where raindrops fall from a canopy, treatment at a planting position, spraying of a granule formulation on flower clusters, mixing with a paste fertilizer).

Examples of the application to seeds (or seed treatments) include an application of the present compound, the present compound **Y,** the compound of the present invention, or the composition A to seeds or vegetative reproductive organs, and specific examples thereof include spraying treatment in which a suspension of the present compound, the present compound **Y,** the compound of the present invention, or the composition A is sprayed onto seed surface or the vegetative reproductive organ surface in the form of mist; smearing treatment in which the Present compound, the present compound **Y,** the compound of the present invention, or the composition A is coated a surface of seeds or the vegetative reproductive organ; a soaking treatment in which the seeds are soaked into the solution of the present compound, the present compound **Y,** the compound of the present invention, or the composition A for a certain time; and a method for coating the seeds or the vegetative reproductive organ with a carrier containing the present compound, the present compound **Y,** the compound of the present invention, or the composition A (film coating treatment, pellet coating treatment). Examples of the above-described vegetative reproductive organ include particularly seed potato.

As used herein, seeds or vegetative reproductive organs carrying the present compound, the present compound **Y,** the compound of the present invention, or the composition A means seeds or vegetative reproductive organs in the state where the present compound, the present compound **Y,** the compound of the present invention, or the composition A is adhered to a surface of the seeds or the vegetative reproductive organ. The above-described seeds or vegetative reproductive organs carrying the present compound, the present compound **Y,** the compound of the present invention, or the composition A may be adhered by any other materials that are different from the present compound, the present compound **Y,** the compound of the present invention, or the composition A before or after being adhered the present compound, the present compound Y, the compound of the present invention, or the composition A to the seeds or vegetative reproductive organs.

When the composition A is applied to seeds or vegetative reproductive organs, the composition A may be also applied to seeds or vegetative reproductive organs as a single formulation, or the Composition A may be applied to seeds or vegetative reproductive organs as multiple different formulations by multiple times. Examples of the method in which the Composition A is applied as multiple different formulations by multiple times include, for example, a method in which the formulations comprising as an active component the Present compound only are applied, and seeds or vegetative reproductive organs are air dried, followed by applying the formulations comprising the Present ingredient: and a method in which the formulations comprising as an active component the present compound and the Present ingredients are applied, and seeds or vegetative reproductive organs are air dried, followed by applying the formulations comprising the Present ingredients other than the already-applied Present ingredients, are included.

Also, when the composition A is adhered in a form of layer(s) to a surface of seeds or vegetative reproductive organ, the layer(s) is/are composed of one layer or multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs carrying the present compound, the present compound Y, the compound of the present invention, or the composition A can be obtained, for example, by applying the formulations comprising the present compound, the present compound Y, the compound of the present invention, or the composition A by the above-described application method to seeds to seeds or vegetative reproductive organs.

The application dose of the present compound, the present compound Y, the compound of the present invention, or the composition A may be varied depending on the climate condition, the formulation forms, the application timing, the application method, the application area, the target diseases, or the target crops, and the like, and in the case of the foliar application or soil application, a dose thereof is within a range of 1 to 500 g per 1,000 m². In the case of being applied to seeds, the dose of application dose of the present compound, the present compound Y, the compound of the present invention, or the composition A is usually within a range of 0.001 to 100 g of the Present compound per 1 Kg of seeds. When the present compound, the present compound Y, the compound of the present invention, or the composition A is formulated into an emulsifiable concentrate, a wettable powder or a flowable etc., they are usually applied by diluting them with water so as to make an effective concentration of the active ingredients 0.01 to 10,000 ppm, and the dust formulation or the granular formulation, etc., is usually applied as itself without diluting them.

The present compound, the present compound Y, the compound of the present invention, or the composition A can be used as an agent for controlling plant diseases in the agricultural land such as field, paddy, lawn and orchard. Examples of the plants include the followings.

corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

### EXAMPLES

Hereinafter, the present invention is explained in more detail by using Preparation Examples, Reference Preparation Examples, Formulation Examples, and Test Examples, etc., however, the present invention should not be limited to these examples.

As used herein, "Me" represents a methyl group, "Et" represents an ethyl group, "Pr" represents a propyl group, "i-Pr" represents an isopropyl group, "c-Pr" represents a cyclopropyl group, "Bu" represents a butyl group, "i-Bu" represents an isobutyl group, "s-Bu" represents a sec-butyl group, "Ph" represents a phenyl group, "Bn" represents a benzyl group, "N-pyrrolidinyl" represents a N-pyrrolidinyl group, "2-pyrimidyl" represents a 2-pyrimidyl group, "2-pyrazinyl" represents a 2-pyrazinyl group, "4-pyrimidyl" represents 4-pyrimidyl group, "2-Py" represents a 2-pyridyl group, "3-Py" represents a 3-pyridyl group, "4-Py" represents a 4-pyridyl group, "2-furyl" represents a 2-furyl group, "3-furyl" represents a 3-furyl group, "2-thienyl" represents a 2-thienyl group, and "3-thienyl" represents a 3-thienyl group. When the phenyl group has a substituent, the substituent is written with its substituted position before the symbol. For example, "3,5-diF-Ph" represents a 3,5-difluorophenyl.

In the case where a physical property value of a compound is measured by gas chromatography (GC) / Mass analysis (MS) (hereinafter, referred to as "GCMS"), the measured molecular ion value [M]⁺ and a retention time (hereinafter, referred to as "RT") is described. The condition for GC is shown below.

### [GC Analysis condition]

Device: HP 7890A (manufactured by Agilent Technologies, Ltd.)
Column: HP-5MS (0.25 µm × 0.25 mm × 30 m) (manufactured by Agilent Technologies, Ltd.)
Inlet temperature: 250 °C
Control mode: linear velocity, Pressure: 14.7 psi, Linear velocity: 23.98 cm/sec
Carrier gas: helium
Flow volume: 1 mL/min
Injection volume: 1.0 µL (split ratio 200 : 1)
Oven temperature: 70°C (2 min) → 15 °C/min → 300 °C (2.67 min)

### [MS analysis condition]

Detector: 5875C inert XL MSD (manufactured by Agilent Technologies, Ltd.)
Ionization method: EI
Ionization voltage: 69.9 eV, Ion source temperature: 230 °C

When a physical property value of a compound is measured by a liquid chromatography / Mass spectrometry (hereinafter, referred to as "LCMS"), the measured molecular ion value [M + H]⁺, [M + H + MeCN]⁺, or [M - H]⁻, and a retention time (hereinafter, referred to as "RT") are described. The condition for the liquid chromatography (hereinafter, referred to as "LC") and mass spectrometry (hereinafter, referred to as "MS") are described below.

### [LC condition]

Column: L-column2 ODS, inner diameter 4.6 mm, length 30 mm, particle size 3 µm (Chemicals Evaluation and Research Institute, Japan)
UV measurement wavelength: 254 nm
Mobile phase: A solution: 0.1 % aqueous solution of formic acid, B solution: 0.1 % acetonitrile solution of formic acid
Flow rates: 2.0 mL/min.
Pump: LC-20 AD (manufactured by Shimadzu Corporation) two machines (high pressure gradient)
Gradient condition: a solution for elution was transferred at a concentration gradient indicated in [Table LC1].

**[Table LC1]**

| Time (min) | A solution (%) | B solution (%) |
|---|---|---|
| 0.01 | 90 | 10 |
| 2.00 | 0 | 100 |
| 4.00 | 0 | 100 |
| 4.01 | 90 | 10 |

### [MS condition]

Detector: LCMS-2020 (manufactured by Shimadzu Corporation) Ionization Methods: DUIS

First, a preparation example of the Present compounds Y (including the compounds of the present invention) is shown.

### Preparation Example 1

A mixture of 1-bromo-3-(ethylsulfanyl)benzene 0.43 g, 4-chloropheyl boronic acid 0.33 g, [1,1'-bis(diphenylphosphino)ferrocene] dichloropalladium (II) 0.15 g, tripotassium phosphate 1.28 g, DME 4 mL, and water 0.1 mL was stirred under reflux for 8 hours. Water was added to the resulting mixture, and the mixture was extracted with ethyl acetate. The resulting organic layer was washed with water and saturated brine successively, dried over anhydrous sodium sulfate, and concentrated under reduced pressure. The resulting residue was subjected to a silica gel column chromatography (hexane : ethyl acetate = 10 : 1) to obtain the present compound 1 represented by the following formula 0.22 g. 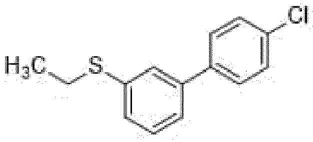 Present compound 1: ¹H-NMR (CDCl₃) δ: 7.52-7.48 (3H, m), 7.43-7.39 (2H, m), 7.37-7.34 (2H, m), 7.33-7.29 (1H, m), 3.00 (2H, q), 1.35 (3H, t).

### Preparation Example 1-1

The compounds which were prepared according to the Preparation Example 1 and their physical property values are shown.

A compound represented by formula (A-1): 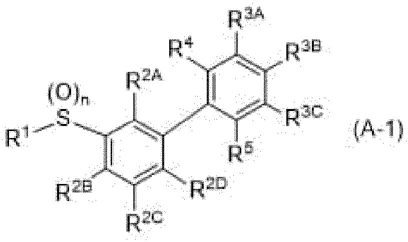 (hereinafter, referred to as "Compound (A-1)") wherein a combination of n, R¹, R^{2A}, R^{2B}, R^{2C}, R^{2D}, R^{3A}, R^{3B}, R^{3C}, R⁴ and R⁵ represents any combination described in [Table A-1] and [Table A-1A]).

**[Table A-1]**

| Present compoun d | n | R¹ | R^{2A} | R^{2B} | R^{2C} | R^{2D} | R^{3A} | R^{3B} | R^{3C} | R⁴ | R⁵ |
|---|---|---|---|---|---|---|---|---|---|---|---|
| 2 | 0 | Et | H | H | H | H | F | Cl | F | H | H |
| 5 | 0 | Et | H | H | H | H | F | Me | H | H | H |
| 6 | 0 | Et | H | H | H | H | H | Me | H | H | F |
| 7 | 0 | Et | H | H | H | H | H | Me | H | H | H |
| 8 | 0 | Et | H | H | H | H | F | Cl | H | H | H |
| 9 | 0 | Et | H | H | H | H | OCH₂O | | H | H | H |
| 10 | 0 | Et | H | H | H | H | H | CF₃ | H | H | H |
| 13 | 0 | Pr | H | H | H | H | F | Cl | F | H | H |
| 14 | 0 | Pr | H | H | H | H | F | Me | H | H | H |
| 19 | 0 | Pr | H | H | H | H | Me | H | Me | H | H |
| 33 | 0 | Pr | H | H | H | H | F | Me | F | H | H |
| 34 | 0 | Pr | H | H | H | H | Me | Me | H | H | H |
| 35 | 0 | Pr | H | H | H | H | CH₂CH₂CH ₂ | | H | H | H |
| 41 | 0 | Bu | H | H | H | H | F | Cl | F | H | H |
| 42 | 0 | s-Bu | H | H | H | H | F | Cl | F | H | H |
| 43 | 0 | Bn | H | H | H | H | F | Cl | F | H | H |
| 44 | 0 | c-Pr | H | H | H | H | F | Cl | F | H | H |
| 45 | 2 | N-pyrrolidiny 1 | H | H | H | H | F | Cl | F | H | H |
| 46 | 2 | NMe₂ | H | H | H | H | F | Cl | F | H | H |

**[Table A-1A]**

| Present compound | n | R¹ | R^{2A} | R^{2B} | R^{2C} | R^{2D} | R^{3A} | R^{3B} | R^{3C} | R⁴ | R⁵ |
|---|---|---|---|---|---|---|---|---|---|---|---|
| 55 | 0 | CF₃ | H | H | H | H | H | Cl | H | H | H |
| 56 | 0 | CF₃ | H | H | H | H | F | Cl | F | H | H |
| 60 | 0 | CH₂ (3, 5-diF-Ph) | H | H | H | H | F | Cl | F | H | H |
| 61 | 0 | CH₂CH₂Ph | H | H | H | H | F | Cl | F | H | H |
| 62 | 0 | CH₂ (c-Pr) | H | H | H | H | F | Cl | F | H | H |
| 66 | 0 | Pr | H | H | H | H | F | F | F | H | H |
| 67 | 0 | Pr | H | H | H | H | F | H | F | H | H |
| 72 | 0 | Pr | H | H | F | H | F | Me | H | H | H |
| 75 | 2 | 2-pyrimidyl | H | H | H | H | F | Cl | F | H | H |
| 76 | 2 | 2-pyrazinyl | H | H | H | H | F | Cl | F | H | H |
| 77 | 2 | 4-pyrimidyl | H | H | H | H | F | Cl | F | H | H |
| 78 | 2 | 2-Py | H | H | H | H | F | Cl | F | H | H |
| 79 | 2 | 3-Py | H | H | H | H | F | Cl | F | H | H |
| 80 | 2 | 4-Py | H | H | H | H | F | Cl | F | H | H |

Present compound 2: ¹H-NMR (CDCl₃) δ: 7.45 (1H, br s), 7.39-7.30 (3H, m), 7.19 (2H, d), 3.01 (2H, q), 1.35 (3H, t).

Present compound 5: ¹H-NMR (CDCl₃) δ: 7.51-7.50 (1H, m), 7.37-7.33 (2H, m), 7.33-7.20 (4H, m), 3.00 (2H, q), 2.31 (3H, d), 1.35 (3H, t).

Present compound 6: ¹H-NMR (CDCl₃) δ: 7.50-7.47 (1H, m), 7.36-7.28 (4H, m), 7.02 (1H, dq), 6.97 (1H, dd), 2.98 (2H, q), 2.39 (3H, s), 1.34 (3H, t).

Present compound 7: ¹H-NMR (CDCl₃) δ: 7.53 (1H, td), 7.47 (2H, dt), 7.40-7.23 (5H, m), 2.99 (2H, q), 2.40 (3H, s), 1.34 (3H, t).

Present compound 8: ¹H-NMR (CDCl₃) δ: 7.49-7.27 (7H, m), 3.00 (2H, q), 1.35 (3H, t).

Present compound 9: ¹H-NMR (CDCl₃) δ: 7.46 (1H, t), 7.33-7.26 (3H, m), 7.06-7.02 (2H, m), 6.88 (1H, d), 6.01 (2H, s), 2.99 (2H, q), 1.34 (3H, t).

Present compound 10: ¹H-NMR (CDCl₃) δ: 7.72-7.65 (4H, m), 7.54-7.53 (1H, m), 7.41-7.33 (3H, m), 3.01 (2H, q), 1.36 (3H, t).

Present compound 13: ¹H-NMR (CDCl₃) δ: 7.45 (1H, td), 7.39-7.32 (2H, m), 7.30 (1H, dt), 7.21-7.16 (2H, m), 2.96 (2H, t), 1.71 (2H, dq), 1.05 (3H, t).

Present compound 14: ¹H-NMR (CDCl₃) δ: 7.50 (1H, br s), 7.36-7.27 (3H, m), 7.27-7.19 (3H, m), 2.95 (2H, t), 2.31 (3H, d), 1.71 (2H, dq), 1.04 (3H, t).

Present compound 19: ¹H-NMR (CDCl₃) δ: 7.52 (1H, dd), 7.36 (1H, dt), 7.33 (1H, td), 7.29 (1H, dt), 7.19 (2H, br s), 7.01 (1H, br s), 2.95 (2H, dd), 2.38 (6H, br s), 1.70 (2H, td), 1.04 (3H, t).

Present compound 33: ¹H-NMR (CDCl₃) δ: 7.47 (1H, td), 7.38-7.29 (3H, m), 7.10-7.01 (2H, m), 2.95 (2H, t), 2.23 (3H, br s), 1.71 (2H, td), 1.05 (3H, t).

Present compound 34: ¹H-NMR (CDCl₃) δ: 7.53-7.52 (1H, m), 7.39-7.17 (6H, m), 2.94 (2H, t), 2.33 (3H, s), 2.31 (3H, s), 1.70 (2H, td), 1.04 (3H, t).

Present compound 35: ¹H-NMR (CDCl₃) δ: 7.53 (1H, t), 7.43 (1H, br s), 7.38-7.26 (5H, m), 3.00-2.82 (6H, m), 2.16-2.08 (2H, m), 1.70 (2H, td), 1.03 (3H, t).

Present compound 41: ¹H-NMR (CDCl₃) δ: 7.45-7.44 (1H, m), 7.39-7.29 (3H, m), 7.19 (2H, d), 2.98 (2H, t), 1.71-1.63 (2H, m), 1.53-1.43 (2H, m), 0.94 (3H, t).

Present compound 42: ¹H-NMR (CDCl₃) δ: 7.53-7.52 (1H, m), 7.43-7.34 (3H, m), 7.19 (2H, d), 3.24 (1H, td), 1.74-1.51 (2H, m), 1.31 (3H, d), 1.03 (3H, t).

Present compound 43: ¹H-NMR (CDCl₃) δ: 7.36-7.25 (9H, m), 7.09 (2H, d), 4.15 (2H, s).

Present compound 44: ¹H-NMR (CDCl₃) δ: 7.49 (1H, td), 7.41 (1H, dt), 7.37 (1H, td), 7.27 (1H, dt), 7.19 (2H, d), 2.27-2.20 (1H, m), 1.15-1.10 (2H, m), 0.76-0.71 (2H, m).

Present compound 45: GCMS: 357 [M]⁺, RT = 19.4 min

Present compound 46: GCMS: 331 [M]⁺, RT = 17.4 min

Present compound 55: GCMS: 288 [M]⁺, RT = 13.1 min

Present compound 56: GCMS: 324 [M]⁺, RT = 12.8 min

Present compound 60: GCMS: 382 [M]⁺, RT = 18.1 min

Present compound 61: GCMS: 360 [M]⁺, RT = 19.6 min

Present compound 62: GCMS: 310 [M]⁺, RT = 16.6 min

Present compound 66: ¹H-NMR (CDCl₃) δ: 7.43-7.41 (1H, m), 7.38-7.31 (2H, m), 7.29-7.25 (1H, m), 7.19-7.14 (2H, m), 2.98-2.93 (2H, m), 1.77-1.66 (2H, m), 1.05 (3H, t).

Present compound 67: ¹H-NMR (CDCl₃) δ: 7.49-7.47 (1H, m), 7.40-7.30 (3H, m), 7.12-7.04 (2H, m), 6.83-6.76 (1H, m), 2.96 (2H, t), 1.76-1.66 (2H, m), 1.05 (3H, t).

Present compound 72: ¹H-NMR (CDCl₃) δ: 7.28-7.18 (4H, m), 7.05-6.95 (2H, m), 2.95 (2H, t), 2.31 (3H, d), 1.78-1.66 (2H, m), 1.05 (3H, t).

Present compound 75: LCMS: 367 [M+H]⁺, RT = 2.00 min

Present compound 76: GCMS: 366 [M]⁺, RT = 17.8 min

Present compound 77: LCMS: 367 [M+H]⁺, RT = 2.06 min

Present compound 78: LCMS: 366 [M+H]⁺, RT = 2.10 min

Present compound 79: LCMS: 366 [M+H]⁺, RT = 2.07 min

Present compound 80: LCMS: 366 [M+H]⁺, RT = 2.08 min

### Preparation Example 2

To a mixture of the present compound 2 0.44 g and chloroform 4 mL was added mCPBA (purity 75 %, containing 25 % water) 0.50 g at 0 °C, and the mixture was stirred at room temperature for 3 hours. Saturated aqueous sodium thiosulfate solution 10 mL and saturated aqueous sodium hydrogen carbonate solution 10 mL were added to the resulting mixture, and the resulting mixture was stirred for 1 hour, and then extracted with chloroform. The resulting organic layer was washed with saturated brine, dried over anhydrous sodium sulfate, and concentrated under reduced pressure. The resulting residue was subjected to a silica gel column chromatography (hexane : ethyl acetate = 2 : 1) to obtain the present compound 4 represented by the following formula 0.23 g and the present compound 26 represented by the following formula 0.15 g. 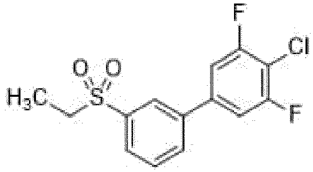 Present compound 4: ¹H-NMR (CDCl₃) δ: 8.07 (1H, t), 7.95 (1H, dt), 7.82 (1H, ddd), 7.69 (1H, t), 7.28-7.23 (2H, m), 3.18 (2H, q), 1.33 (3H, t). 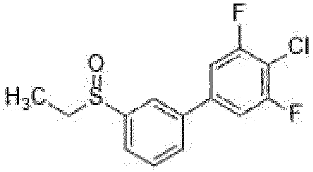 Present compound 26: ¹H-NMR (CDCl₃) δ: 7.83-7.81 (1H, m), 7.67-7.57 (3H, m), 7.29-7.23 (2H, m), 3.01-2.93 (1H, m), 2.85-2.77 (1H, m), 1.25 (3H, t).

### Preparation Example 2-1

The compounds which were prepared according to the Preparation Example 2 and their physical property values are shown.

The compound represented by formula (A-1) wherein the combination of n, R¹, R^{2A}, R^{2B}, R^{2C}, R^{2D}, R^{3A}, R^{3B}, R^{3C}, R⁴ and R⁵ represents any combination described in [Table A-2] and [Table A-2A].

**[Table A-2]**

| Present compound | n | R¹ | R^{2A} | R^{2B} | R^{2C} | R^{2D} | R^{3A} | R^{3B} | R^{3C} | R⁴ | R⁵ |
|---|---|---|---|---|---|---|---|---|---|---|---|
| 3 | 2 | Et | H | H | H | H | H | Cl | H | H | H |
| 11 | 2 | Et | H | H | H | H | F | Me | H | H | H |
| 12 | 1 | Et | H | H | H | H | F | Me | H | H | H |
| 15 | 2 | Et | H | H | H | H | H | Me | H | H | F |
| 16 | 1 | Et | H | H | H | H | H | Me | H | H | F |
| 17 | 2 | Et | H | H | H | H | H | Me | H | H | H |
| 18 | 1 | Et | H | H | H | H | H | Me | H | H | H |
| 20 | 2 | Pr | H | H | H | H | F | Cl | F | H | H |
| 21 | 1 | Pr | H | H | H | H | F | Cl | F | H | H |
| 22 | 2 | Et | H | H | H | H | F | Cl | H | H | H |
| 23 | 1 | Et | H | H | H | H | F | Cl | H | H | H |
| 24 | 2 | Pr | H | H | H | H | F | Me | H | H | H |
| 25 | 1 | Pr | H | H | H | H | F | Me | H | H | H |
| 27 | 2 | Et | H | H | H | H | H | CF₃ | H | H | H |
| 28 | 1 | Et | H | H | H | H | H | CF₃ | H | H | H |
| 29 | 2 | Et | H | H | H | H | OCH₂O | | H | H | H |
| 30 | 1 | Et | H | H | H | H | OCH₂O | | H | H | H |
| 31 | 2 | Pr | H | H | H | H | Me | H | Me | H | H |
| 32 | 1 | Pr | H | H | H | H | Me | H | Me | H | H |
| 36 | 2 | Pr | H | H | H | H | F | Me | F | H | H |
| 37 | 1 | Pr | H | H | H | H | F | Me | F | H | H |
| 38 | 2 | Pr | H | H | H | H | Me | Me | H | H | H |
| 39 | 2 | Pr | H | H | H | H | CH₂CH₂CH₂ | | H | H | H |
| 40 | 1 | Pr | H | H | H | H | CH₂CH₂CH₂ | | H | H | H |
| 47 | 2 | Bu | H | H | H | H | F | Cl | F | H | H |
| 48 | 1 | Bu | H | H | H | H | F | Cl | F | H | H |
| 49 | 2 | s-Bu | H | H | H | H | F | Cl | F | H | H |
| 50 | 1 | **S-**Bu | H | H | H | H | F | Cl | F | H | H |
| 51 | 2 | Bn | H | H | H | H | F | Cl | F | H | H |
| 52 | 1 | Bn | H | H | H | H | F | Cl | F | H | H |
| 53 | 2 | c-Pr | H | H | H | H | F | Cl | F | H | H |
| 54 | 1 | c-Pr | H | H | H | H | F | Cl | F | H | H |

**[Table A-2A]**

| Present compound | n | R¹ | R^{2A} | R^{2B} | R^{2C} | R^{2D} | R^{3A} | R^{3B} | R^{3C} | R⁴ | R⁵ |
|---|---|---|---|---|---|---|---|---|---|---|---|
| 57 | 2 | CF₃ | H | H | H | H | H | Cl | H | H | H |
| 58 | 2 | CF₃ | H | H | H | H | F | Cl | F | H | H |
| 59 | 1 | CF₃ | H | H | H | H | F | Cl | F | H | H |
| 63 | 2 | CH₂ (3, 5-diF-Ph) | H | H | H | H | F | Cl | F | H | H |
| 64 | 2 | CH₂ CH₂Ph | H | H | H | H | F | Cl | F | H | H |
| 65 | 2 | CH₂ (c-Pr) | H | H | H | H | F | Cl | F | H | H |
| 68 | 1 | Pr | H | H | H | H | F | H | F | H | H |
| 69 | 2 | Pr | H | H | H | H | F | H | F | H | H |
| 70 | 1 | Pr | H | H | H | H | F | F | F | H | H |
| 71 | 2 | Pr | H | H | H | H | F | F | F | H | H |
| 73 | 2 | Pr | H | H | F | H | F | Me | H | H | H |
| 74 | 1 | Pr | H | H | F | H | F | Me | H | H | H |

Present compound 3: ¹H-NMR (CDCl₃) δ: 8.09 (1H, t), 7.90 (1H, ddd), 7.84 (1H, ddd), 7.66 (1H, t), 7.55 (2H, ddd), 7.46 (2H, ddd), 3.17 (2H, q), 1.32 (3H, t).

Present compound 11: ¹H-NMR (CDCl₃) δ: 8.09 (1H, t), 7.88 (1H, dt), 7.84 (1H, dt), 7.64 (1H, t), 7.32-7.28 (3H, m), 3.17 (2H, q), 2.34 (3H, d), 1.32 (3H, t).

Present compound 12: ¹H-NMR (CDCl₃) δ: 7.82-7.82 (1H, m), 7.68 (1H, dt), 7.60-7.52 (2H, m), 7.33-7.24 (3H, m), 3.01-2.91 (1H, m), 2.88-2.76 (1H, m), 2.33 (3H, d), 1.24 (3H, t).

Present compound 15: ¹H-NMR (CDCl₃) δ: 8.07 (1H, q), 7.89 (1H, dq), 7.83 (1H, dq), 7.63 (1H, t), 7.35 (1H, t), 7.06 (1H, dd), 7.01 (1H, dd), 3.16 (2H, q), 2.41 (3H, s), 1.32 (3H, t).

Present compound 16: ¹H-NMR (CDCl₃) δ: 7.76 (1H, br s), 7.69-7.64 (1H, m), 7.61-7.55 (2H, m), 7.35 (1H, t), 7.04 (1H, dt), 6.99 (1H, dd), 3.00-2.90 (1H, m), 2.86-2.77 (1H, m), 2.40 (3H, s), 1.24 (3H, t).

Present compound 17: ¹H-NMR (CDCl₃) δ: 8.11 (1H, t), 7.88-7.84 (2H, m), 7.62 (1H, t), 7.52 (2H, d), 7.29 (2H, d), 3.16 (2H, q), 2.42 (3H, s), 1.31 (3H, t).

Present compound 18: ¹H-NMR (CDCl₃) δ: 7.83 (1H, td), 7.70 (1H, dt), 7.59-7.50 (4H, m), 7.27 (2H, d), 3.00-2.90 (1H, m), 2.86-2.76 (1H, m), 2.41 (3H, s), 1.23 (3H, t).

Present compound 20: ¹H-NMR (CDCl₃) δ: 8.06 (1H, t), 7.95 (1H, dq), 7.81 (1H, dq), 7.68 (1H, td), 7.28-7.23 (2H, m), 3.14-3.10 (2H, m), 1.83-1.75 (2H, m), 1.03 (3H, t).

Present compound 21: ¹H-NMR (CDCl₃) δ: 7.84-7.83 (1H, m), 7.67-7.62 (1H, m), 7.62-7.58 (2H, m), 7.29-7.24 (2H, m), 2.85-2.79 (2H, m), 1.91-1.79 (1H, m), 1.76-1.65 (1H, m), 1.08 (3H, t).

Present compound 22: GCMS: 298 [M]⁺, RT = 17.6 min

Present compound 23: ¹H-NMR (CDCl₃) δ: 7.84-7.34 (7H, m), 3.01-2.89 (1H, m), 2.86-2.73 (1H, m), 1.24 (3H, t).

Present compound 24: ¹H-NMR (CDCl₃) δ: 8.09 (1H, t), 7.88 (1H, dq), 7.83 (1H, dq), 7.63 (1H, t), 7.32-7.25 (3H, m), 3.13-3.09 (2H, m), 2.34 (3H, d), 1.83-1.73 (2H, m), 1.02 (3H, t).

Present compound 25: ¹H-NMR (CDCl₃) δ: 7.83 (1H, t), 7.68 (1H, dt), 7.61-7.55 (2H, m), 7.34-7.24 (3H, m), 2.89-2.75 (2H, m), 2.33 (3H, d), 1.89-1.80 (1H, m), 1.76-1.65 (1H, m), 1.07 (3H, t).

Present compound 27: ¹H-NMR (CDCl₃) δ: 8.14 (1H, t), 7.95 (1H, dq), 7.89 (1H, dq), 7.77-7.67 (5H, m), 3.18 (2H, q), 1.33 (3H, t).

Present compound 28: ¹H-NMR (CDCl₃) δ: 7.89 (1H, td), 7.75-7.71 (5H, m), 7.63 (1H, td), 7.59 (1H, dt), 3.01-2.93 (1H, m), 2.87-2.78 (1H, m), 1.25 (3H, t).

Present compound 29: ¹H-NMR (CDCl₃) δ: 8.04 (1H, t), 7.84 (1H, dq), 7.79 (1H, dq), 7.61 (1H, t), 7.12-7.07 (2H, m), 6.92 (1H, d), 6.04 (2H, s), 3.16 (2H, q), 1.31 (3H, t).

Present compound 30: ¹H-NMR (CDCl₃) δ: 7.77 (1H, td), 7.63 (1H, dt), 7.55 (1H, td), 7.51 (1H, dt), 7.11-7.08 (2H, m), 6.90 (1H, d), 6.02 (2H, s), 3.00-2.90 (1H, m), 2.85-2.76 (1H, m), 1.24 (3H, t).

Present compound 31: ¹H-NMR (CDCl₃) δ: 8.11 (1H, t), 7.86 (2H, dd), 7.61 (1H, t), 7.24 (2H, br s), 7.06 (1H, s), 3.14-3.08 (2H, m), 2.40 (6H, s), 1.82-1.73 (2H, m), 1.01 (3H, t).

Present compound 32: ¹H-NMR (CDCl₃) δ: 7.85 (1H, t), 7.70 (1H, dt), 7.58-7.52 (2H, m), 7.24 (2H, br s), 7.04 (1H, br s), 2.89-2.75 (2H, m), 2.39 (6H, s), 1.90-1.78 (1H, m), 1.78-1.66 (1H, m), 1.06 (3H, t).

Present compound 36: ¹H-NMR (CDCl₃) δ: 8.06 (1H, t), 7.91 (1H, dq), 7.81 (1H, dq), 7.65 (1H, t), 7.12 (2H, d), 3.13-3.09 (2H, m), 2.25 (3H, t), 1.84-1.74 (2H, m), 1.02 (3H, t).

Present compound 37: ¹H-NMR (CDCl₃) δ: 7.82 (1H, dd), 7.67-7.56 (3H, m), 7.13 (2H, d), 2.87-2.76 (2H, m), 2.24 (3H, s), 1.90-1.79 (1H, m), 1.77-1.66 (1H, m), 1.07 (3H, t).

Present compound 38: ¹H-NMR (CDCl₃) δ: 8.10 (1H, t), 7.88-7.82 (2H, m), 7.64-7.58 (1H, m), 7.41 (1H, d), 7.36 (1H, dd), 7.24 (1H, d), 3.12-3.08 (2H, m), 2.35 (3H, s), 2.32 (3H, s), 1.82-1.73 (2H, m), 1.01 (3H, t).

Present compound 39: ¹H-NMR (CDCl₃) δ: 8.10 (1H, t), 7.87-7.83 (2H, m), 7.61 (1H, t), 7.49 (1H, s), 7.39 (1H, dd), 7.33 (1H, d), 3.13-3.08 (2H, m), 2.98 (4H, q), 2.18-2.10 (2H, m), 1.82-1.73 (2H, m), 1.00 (3H, t).

Present compound 40: ¹H-NMR (CDCl₃) δ: 7.84 (1H, td), 7.70 (1H, dt), 7.58-7.53 (2H, m), 7.49 (1H, br s), 7.40 (1H, dd), 7.31 (1H, d), 2.96 (4H, q), 2.89-2.75 (2H, m), 2.17-2.09 (2H, m), 1.88-1.65 (2H, m), 1.06 (3H, t).

Present compound 47: GCMS: 344 [M]⁺, RT = 18.2 min

Present compound 48: LCMS: 329 [M+H]⁺, RT = 2.16 min

Present compound 49: GCMS: 344[M]⁺, RT = 17.9 min

Present compound 50: LCMS: 329 [M+H]⁺, RT = 2.14 min

Present compound 51: LCMS: 379 [M+H]⁺, RT = 2.22 min

Present compound 52: LCMS: 363 [M+H]⁺, RT = 2.17 min

Present compound 53: GCMS 328 [M]⁺, RT = 18.1 min

Present compound 54: LCMS: 313 [M+H]⁺, RT = 1.98 min

Present compound 57: GCMS: 320 [M]⁺, RT = 14.5 min

Present compound 58: GCMS: 356 [M]⁺, RT = 14.2 min

Present compound 59: GCMS: 340 [M]⁺, RT = 14.3 min

Present compound 63: GCMS 414 [M]⁺, RT = 19.5 min

Present compound 64: LCMS: 393 [M+H]⁺, RT = 2.29 min

Present compound 65] LCMS: 384 [M+H+MeCN]⁺, RT = 2.15 min

Present compound 68: LCMS: 281 [M+H]⁺, RT = 1.91 min

Present compound 69: GCMS: 296 [M]⁺, RT = 16.2 min

Present compound 70: LCMS: 299 [M+H]⁺, RT = 1.96 min

Present compound 71: GCMS: 314 [M]⁺, RT = 16.3 min

Present compound 73: GCMS: 310 [M]⁺, RT = 16.8 min

Present compound 74: LCMS: 295 [M+H]⁺, RT = 2.08 min

### Reference Preparation Example 1

Under argon atmosphere, a mixture of 1-bromo-3-(propylsulfanyl)benzene 10.0 g, [1,1'-bis(diphenylphosphino)ferrocene] dichloropalladium (II) 1.6 g, bis(pinacolato)diboron 13.2 g, potassium acetate 8.5 g, and DMSO 100 mL was stirred at 90 °C for 8 hours. After the resulting mixture was cooled to room temperature, water was added thereto, and the mixture was filtered. MTBE was added to the resulting filtrates, and the mixture was washed with water and saturated brine successively. The resulting organic layer was dried over anhydrous sodium sulfate, and concentrated under reduced pressure, and the obtained residue was subjected to a silica gel column chromatography (hexane : ethyl acetate = 20 : 1) to obtain the intermediate compound 1 represented by the following formula 10.2 g. 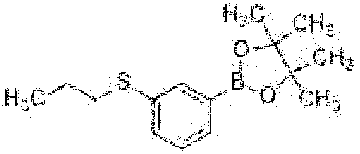 Intermediate compound 1: ¹H-NMR (CDCl₃) δ: 7.77 (1H, br s), 7.60 (1H, ddd), 7.42 (1H, ddd), 7.29 (1H, dd), 2.92 (2H, t), 1.66 (2H, td), 1.34 (12H, s), 1.02 (3H, t).

The compounds which were prepared according to the above Process and Preparation Example are shown below.

The compound represented by formula (A-1) wherein n is 0, each of R^{2A}, R^{2B}, R^{2C} and R^{2D} represents a hydrogen atom, and a combination of R¹, R^{3A}, R^{3B}, R^{3C}, R⁴ and R⁵ represents any combination described in the Combination A (hereinafter, referred to as "Compound class SX1").

The combination A consists of the Substituent Nos. A1 to A660. The substituent Nos. A1 to A660 represent the compound represented by formula (A-1) wherein the substituent No. represents a combination of R¹, R^{3A}, R^{3B}, R^{3C}, R⁴ and R⁵, which is described as [Substituent No.; R¹, R^{3A}, R^{3B}, R^{3C}, R⁴, R⁵] below.

For example, the Substituent No. A5 represents the combination in which R¹ represents an ethyl group, each of R^{3A}, R^{3B} and R^{3C} represents a methyl group, and each of R⁴ and R⁵ represents a hydrogen atom.

### Combination A

[Substituent No. ; R¹ , R^{3A}, R^{3B}, R^{3C}, R⁴, R⁵]:
[A1;Et,Me,H,H,H,H], [A2;Et,H,Me,H,H,H], [A3;Et,Me,Me,H,H,H], [A4;Et,Me,H,Me,H,H], [A5;Et,Me,Me,Me,H,H], [A6;Et,F,H,H,H,H], [A7;Et,H,F,H,H,H], [A8;Et,F,F,H,H,H], [A9;Et,F,H,F,H,H], [A10;Et,F,F,F,H,H], [A11;Et,Cl,H,H,H,H], [A12;Et,H,Cl,H,H,H], [A13;Et,Cl,Cl,H,H,H], [A14;Et,Cl,H,Cl,H,H], [A15;Et,Cl,Cl,Cl,H,H], [A16;Et,Br,H,H,H,H], [A17;Et,H,Br,H,H,H], [A18;Et,Br,Br,H,H,H], [A19;Et,Br,H,Br,H,H], [A20;Et,Br,Br,Br,H,H], [A21;Et,I,H,H,H,H], [A22;Et,H,I,H,H,H], [A23;Et,I,I,H,H,H], [A24;Et,I,H,I,H,H], [A25;Et,I,I,I,H,H], [A26;Et,Et,H,H,H,H], [A27;Et,H,Et,H,H,H], [A28;Et,Et,H,Et,H,H], [A29;Et,CF₃,H,H,H,H], [A30;Et,H,CF₃,H,H,H], [A31;Et,CF₃,H,CF₃,H,H], [A32;Et,c-Pr,H,H,H,H], [A33;Et,H,c-Pr,H,H,H], [A34;Et,OMe,H,H,H,H], [A35;Et,H,OMe,H,H,H], [A36;Et,OMe,H,OMe,H,H], [A37;Et,SMe,H,H,H,H], [A38;Et,H,SMe,H,H,H], [A39;Et,S(O)Me,H,H,H,H], [A40;Et,H,S(O)Me,H,H,H], [A41;Et,S(O)₂Me,H,H,H,H], [A42;Et,H,S(O)₂Me,H,H,H], [A43;Et,C(O)OMe,H,H,H,H], [A44;Et,H,C(O)OMe,H,H,H], [A45;Et,C(O)Me,H,H,H,H], [A46;Et,H,C(O)Me,H,H,H], [A47;Et,CN,H,H,H,H], [A48;Et,H,CN,H,H,H], [A49;Et,NO₂,H,H,H,H], [A50;Et,H,NO₂,H,H,H], [A51;Et,NH₂,H,H,H,H], [A52;Et,H,NH₂,H,H,H], [A53;Et,NMe₂,H,H,H,H], [A54;Et,H,NMe₂,H,H,H], [A55;Et,Ph,H,H,H,H], [A56;Et,H,Ph,H,H,H], [A57;Et,OPh,H,H,H,H], [A58;Et,H,OPh,H,H,H], [A59;Et,Me,F,H,H,H], [A60;Et,Me,H,F,H,H], [A61;Et,Me,F,F,H,H], [A62;Et,Me,Cl,H,H,H], [A63;Et,Me,H,Cl,H,H], [A64;Et,Me,Cl,Cl,H,H], [A65;Et,Me,Br,H,H,H], [A66;Et,Me,H,Br,H,H], [A67;Et,Me,Br,Br,H,H], [A68;Et,Me,I,H,H,H], [A69;Et,Me,H,I,H,H], [A70;Et,Me,I,I,H,H], [A71;Et,Me,F,Me,H,H], [A72;Et,Me,Cl,Me,H,H], [A73;Et,Me,Br,Me,H,H], [A74;Et,Me,I,Me,H,H], [A75;Et,Me,Me,F,H,H], [A76;Et,Me,Me,Cl,H,H], [A77;Et,Me,Me,Br,H,H], [A78;Et,Me,Me,I,H,H], [A79;Et,F,Me,H,H,H], [A80;Et,F,Me,F,H,H], [A81;Et,Cl,Me,H,H,H], [A82;Et,Cl,Me,Cl,H,H], [A83;Et,Br,Me,H,H,H], [A84;Et,Br,Me,Br,H,H], [A85;Et,I,Me,H,H,H], [A86;Et,I,Me,I,H,H], [A87;Et,F,Cl,H,H,H], [A88;Et,F,H,Cl,H,H], [A89;Et,F,Cl,Cl,H,H], [A90;Et,F,Br,H,H,H], [A91;Et,F,H,Br,H,H], [A92;Et,F,Br,Br,H,H], [A93;Et,F,I,H,H,H], [A94;Et,F,H,I,H,H], [A95;Et,F,I,I,H,H], [A96;Et,F,F,F,H,H], [A97;Et,F,Cl,F,H,H], [A98;Et,F,Br,F,H,H], [A99;Et,F,I,F,H,H], [A100;Et,F,F,Cl,H,H], [A101;Et,F,F,Br,H,H], [A102;Et,F,F,I,H,H], [A103;Et,Cl,F,H,H,H], [A104;Et,Cl,F,Cl,H,H], [A105;Et,Br,F,H,H,H], [A106;Et,Br,F,Br,H,H], [A107;Et,I,F,H,H,H], [A108;Et,I,F,I,H,H], [A109;Et,Cl,Br,H,H,H], [A110;Et,Cl,H,Br,H,H], [A111;Et,Cl,Br,Br,H,H], [A112;Et,Cl,I,H,H,H], [A113;Et,Cl,H,I,H,H], [A114;Et,Cl,I,I,H,H], [A115;Et,Br,Cl,H,H,H], [A116;Et,Br,Cl,Br,H,H], [A117;Et,I,Cl,H,H,H], [A118;Et,I,Cl,I,H,H], [A119;Et,Cl,Cl,Br,H,H], [A120;Et,Cl,Cl,I,H,H], [A121;Et,Cl,Br,Cl,H,H], [A122;Et,Cl,I,Cl,H,H], [A123;Et,Br,I,H,H,H], [A124;Et,Br,H,I,H,H], [A125;Et,Br,I,I,H,H], [A126;Et,Br,Br,I,H,H], [A127;Et,Br,I,Br,H,H], [A128;Et,Me,Me,H,F,H], [A129;Et,Me,H,Me,F,H], [A130;Et,Me,Me,Me,F,H], [A131;Et,F,F,H,F,H], [A132;Et,F,H,F,F,H], [A133;Et,Cl,Cl,H,F,H], [A134;Et,Cl,H,Cl,F,H], [A135;Et,Br,Br,H,F,H], [A136;Et,Br,H,Br,F,H], [A137;Et,I,I,H,F,H], [A138;Et,I,H,I,F,H], [A139;Et,F,Me,F,F,H], [A140;Et,F,F,F,F,H], [A141;Et,F,Cl,F,F,H], [A142;Et,F,Br,F,F,H], [A143;Et,F,I,F,F,H], [A144;Pr,Me,H,H,H,H], [A145;Pr,H,Me,H,H,H], [A146;Pr,Me,Me,H,H,H], [A147;Pr,Me,H,Me,H,H], [A148;Pr,Me,Me,Me,H,H], [A149;Pr,F,H,H,H,H], [A150;Pr,H,F,H,H,H], [A151;Pr,F,F,H,H,H], [A152;Pr,F,H,F,H,H], [A153;Pr,F,F,F,H,H], [A154;Pr,Cl,H,H,H,H], [A155;Pr,H,Cl,H,H,H], [A156;Pr,Cl,Cl,H,H,H], [A157;Pr,Cl,H,Cl,H,H], [A158;Pr,Cl,Cl,Cl,H,H], [A159;Pr,Br,H,H,H,H], [A160;Pr,H,Br,H,H,H], [A161;Pr,Br,Br,H,H,H], [A162;Pr,Br,H,Br,H,H], [A163;Pr,Br,Br,Br,H,H], [A164;Pr,I,H,H,H,H], [A165;Pr,H,I,H,H,H], [A166;Pr,I,I,H,H,H], [A167;Pr,I,H,I,H,H], [A168;Pr,I,I,I,H,H], [A169;Pr,Et,H,H,H,H], [A170;Pr,H,Et,H,H,H], [A171;Pr,Et,H,Et,H,H], [A172;Pr,CF₃,H,H,H,H], [A173;Pr,H,CF₃,H,H,H], [A174;Pr,CF₃,H,CF₃,H,H], [A175;Pr,c-Pr,H,H,H,H], [A176;Pr,H,c-Pr,H,H,H], [A177;Pr,OMe,H,H,H,H], [A178;Pr,H,OMe,H,H,H], [A179;Pr,OMe,H,OMe,H,H], [A180;Pr,SMe,H,H,H,H], [A181;Pr,H,SMe,H,H,H], [A182;Pr,S(O)Me,H,H,H,H], [A183;Pr,H,S(O)Me,H,H,H], [A184;Pr,S(O)₂Me,H,H,H,H], [A185;Pr,H,S(O)₂Me,H,H,H], [A186;Pr,C(O)OMe,H,H,H,H], [A187;Pr,H,C(O)OMe,H,H,H], [A188;Pr,C(O)Me,H,H,H,H], [A189;Pr,H,C(O)Me,H,H,H], [A190;Pr,CN,H,H,H,H], [A191;Pr,H,CN,H,H,H], [A192;Pr,NO₂,H,H,H,H], [A193;Pr,H,NO₂,H,H,H], [A194;Pr,NH₂,H,H,H,H], [A195;Pr,H,NH₂,H,H,H], [A196;Pr,NMe₂,H,H,H,H], [A197;Pr,H,NMe₂,H,H,H], [A198;Pr,Ph,H,H,H,H], [A199;Pr,H,Ph,H,H,H], [A200;Pr,OPh,H,H,H,H], [A201;Pr,H,OPh,H,H,H], [A202;Pr,Me,F,H,H,H], [A203;Pr,Me,H,F,H,H], [A204;Pr,Me,F,F,H,H], [A205;Pr,Me,Cl,H,H,H], [A206;Pr,Me,H,Cl,H,H], [A207;Pr,Me,Cl,Cl,H,H], [A208;Pr,Me,Br,H,H,H], [A209;Pr,Me,H,Br,H,H], [A210;Pr,Me,Br,Br,H,H], [A211;Pr,Me,I,H,H,H], [A212;Pr,Me,H,I,H,H], [A213;Pr,Me,I,I,H,H], [A214;Pr,Me,F,Me,H,H], [A215;Pr,Me,Cl,Me,H,H], [A216;Pr,Me,Br,Me,H,H], [A217;Pr,Me,I,Me,H,H], [A218;Pr,Me,Me,F,H,H], [A219;Pr,Me,Me,Cl,H,H], [A220;Pr,Me,Me,Br,H,H], [A221;Pr,Me,Me,I,H,H], [A222;Pr,F,Me,H,H,H], [A223;Pr,F,Me,F,H,H], [A224;Pr,Cl,Me,H,H,H], [A225;Pr,Cl,Me,Cl,H,H], [A226;Pr,Br,Me,H,H,H], [A227;Pr,Br,Me,Br,H,H], [A228;Pr,I,Me,H,H,H], [A229;Pr,I,Me,I,H,H], [A230;Pr,F,Cl,H,H,H], [A231;Pr,F,H,Cl,H,H], [A232;Pr,F,Cl,Cl,H,H], [A233;Pr,F,Br,H,H,H], [A234;Pr,F,H,Br,H,H], [A235;Pr,F,Br,Br,H,H], [A236;Pr,F,I,H,H,H], [A237;Pr,F,H,I,H,H], [A238;Pr,F,I,I,H,H], [A239;Pr,F,F,F,H,H], [A240;Pr,F,Cl,F,H,H], [A241;Pr,F,Br,F,H,H], [A242;Pr,F,I,F,H,H], [A243;Pr,F,F,Cl,H,H], [A244;Pr,F,F,Br,H,H], [A245;Pr,F,F,I,H,H], [A246;Pr,Cl,F,H,H,H], [A247;Pr,Cl,F,Cl,H,H], [A248;Pr,Br,F,H,H,H], [A249;Pr,Br,F,Br,H,H], [A250;Pr,I,F,H,H,H], [A251;Pr,I,F,I,H,H], [A252;Pr,Cl,Br,H,H,H], [A253;Pr,Cl,H,Br,H,H], [A254;Pr,Cl,Br,Br,H,H], [A255;Pr,Cl,I,H,H,H], [A256;Pr,Cl,H,I,H,H], [A257;Pr,Cl,I,I,H,H], [A258;Pr,Br,Cl,H,H,H], [A259;Pr,Br,Cl,Br,H,H], [A260;Pr,I,Cl,H,H,H], [A261;Pr,I,Cl,I,H,H], [A262;Pr,Cl,Cl,Br,H,H], [A263;Pr,Cl,Cl,I,H,H], [A264;Pr,Cl,Br,Cl,H,H], [A265;Pr,Cl,I,Cl,H,H], [A266;Pr,Br,I,H,H,H], [A267;Pr,Br,H,I,H,H], [A268;Pr,Br,I,I,H,H], [A269;Pr,Br,Br,I,H,H], [A270;Pr,Br,I,Br,H,H], [A271;Pr,Me,Me,H,F,H], [A272;Pr,Me,H,Me,F,H], [A273;Pr,Me,Me,Me,F,H], [A274;Pr,F,F,H,F,H], [A275;Pr,F,H,F,F,H], [A276;Pr,Cl,Cl,H,F,H], [A277;Pr,Cl,H,Cl,F,H], [A278;Pr,Br,Br,H,F,H], [A279;Pr,Br,H,Br,F,H], [A280;Pr,I,I,H,F,H], [A281;Pr,I,H,I,F,H], [A282;Pr,F,Me,F,F,H], [A283;Pr,F,F,F,F,H], [A284;Pr,F,Cl,F,F,H], [A285;Pr,F,Br,F,F,H], [A286;Pr,F,I,F,F,H], [A287;CH=CH₂,Me,Me,H,H,H], [A288;CH=CH₂,Me,H,Me,H,H], [A289;CH=CH₂,Me,Me,Me,H,H], [A290;CH=CH₂,F,F,H,H,H], [A291;CH=CH₂,F,H,F,H,H], [A292;CH=CH₂,Cl,Cl,H,H,H], [A293;CH=CH₂,Cl,H,Cl,H,H], [A294;CH=CH₂,Br,Br,H,H,H], [A295;CH=CH₂,Br,H,Br,H,H], [A296;CH=CH₂,I,I,H,H,H], [A297;CH=CH₂,I,H,I,H,H], [A298;CH=CH₂,F,Me,F,H,H], [A299;CH=CH₂,F,F,F,H,H], [A300;CH=CH₂,F,Cl,F,H,H], [A301;CH=CH₂,F,Br,F,H,H], [A302;CH=CH₂,F,I,F,H,H], [A303;CH=CH₂,Me,Me,H,H,H], [A304;CH=CHCH₃,Me,Me,H,H,H], [A305;CH=CHCH₃,Me,H,Me,H,H], [A306;CH=CHCH₃,Me,Me,Me,H,H], [A307;CH=CHCH₃,F,F,H,H,H], [A308;CH=CHCH₃,F,H,F,H,H], [A309;CH=CHCH₃,Cl,Cl,H,H,H], [A310;CH=CHCH₃,Cl,H,Cl,H,H], [A311;CH=CHCH₃,Br,Br,H,H,H], [A312;CH=CHCH₃,Br,H,Br,H,H], [A313;CH=CHCH₃,I,I,H,H,H], [A314;CH=CHCH₃,I,H,I,H,H], [A315;CH=CHCH₃,F,Me,F,H,H], [A316;CH=CHCH₃,F,F,F,H,H], [A317;CH=CHCH₃,F,Cl,F,H,H], [A318;CH=CHCH₃,F,Br,F,H,H], [A319;CH=CHCH₃,F,I,F,H,H], [A320;CH=CHCH₃,Me,Me,H,H,H], [A321;CH₂CH=CH₂,Me,Me,H,H,H], [A322;CH₂CH=CH₂,Me,H,Me,H,H], [A323;CH₂CH=CH₂,Me,Me,Me,H,H], [A324;CH₂CH=CH₂,F,F,H,H,H], [A325;CH₂CH=CH₂,F,H,F,H,H], [A326;CH₂CH=CH₂,Cl,Cl,H,H,H], [A327;CH₂CH=CH₂,Cl,H,Cl,H,H], [A328;CH₂CH=CH₂,Br,Br,H,H,H], [A329;CH₂CH=CH₂,Br,H,Br,H,H], [A330;CH₂CH=CH₂,I,I,H,H,H], [A331;CH₂CH=CH₂,I,H,I,H,H], [A332;CH₂CH=CH₂,F,Me,F,H,H], [A333;CH₂CH=CH₂,F,F,F,H,H], [A334;CH₂CH=CH₂,F,Cl,F,H,H], [A335;CH₂CH=CH₂,F,Br,F,H,H], [A336;CH₂CH=CH₂,F,I,F,H,H], [A337;CH₂CH=CH₂,Me,Me,H,H,H], [A338;i-Pr,Me,Me,H,H,H], [A339;i-Pr,Me,H,Me,H,H], [A340;i-Pr,Me,Me,Me,H,H], [A341;i-Pr,F,F,H,H,H], [A342;i-Pr,F,H,F,H,H], [A343;i-Pr,Cl,Cl,H,H,H], [A344;i-Pr,Cl,H,Cl,H,H], [A345;i-Pr,Br,Br,H,H,H], [A346;i-Pr,Br,H,Br,H,H], [A347;i-Pr,I,I,H,H,H], [A348;i-Pr,I,H,I,H,H], [A349;i-Pr,F,Me,F,H,H], [A350;i-Pr,F,F,F,H,H], [A351;i-Pr,F,Cl,F,H,H], [A352;i-Pr,F,Br,F,H,H], [A353;i-Pr,F,I,F,H,H], [A354;i-Pr,Me,Me,H,H,H], [A355;Bu,Me,Me,H,H,H], [A356;Bu,Me,H,Me,H,H], [A357;Bu,Me,Me,Me,H,H], [A358;Bu,F,F,H,H,H], [A359;Bu,F,H,F,H,H], [A360;Bu,Cl,Cl,H,H,H], [A361;Bu,Cl,H,Cl,H,H], [A362;Bu,Br,Br,H,H,H], [A363;Bu,Br,H,Br,H,H], [A364;Bu,I,I,H,H,H], [A365;Bu,I,H,I,H,H], [A366;Bu,F,Me,F,H,H], [A367;Bu,F,F,F,H,H], [A368;Bu,F,Cl,F,H,H], [A369;Bu,F,Br,F,H,H], [A370;Bu,F,I,F,H,H], [A371;Bu,Me,Me,H,H,H], [A372;i-Bu,Me,Me,H,H,H], [A373;i-Bu,Me,H,Me,H,H], [A374;i-Bu,Me,Me,Me,H,H], [A375;i-Bu,F,F,H,H,H], [A376;i-Bu,F,H,F,H,H], [A377;i-Bu,Cl,Cl,H,H,H], [A378;i-Bu,Cl,H,Cl,H,H], [A379;i-Bu,Br,Br,H,H,H], [A380;i-Bu,Br,H,Br,H,H], [A381;i-Bu,I,I,H,H,H], [A382;i-Bu,I,H,I,H,H], [A383;i-Bu,F,Me,F,H,H], [A384;i-Bu,F,F,F,H,H], [A385;i-Bu,F,Cl,F,H,H], [A386;i-Bu,F,Br,F,H,H], [A387;i-Bu,F,I,F,H,H], [A388;i-Bu,Me,Me,H,H,H], [A389;s-Bu,Me,Me,H,H,H], [A390;s-Bu,Me,H,Me,H,H], [A391;s-Bu,Me,Me,Me,H,H], [A392;s-Bu,F,F,H,H,H], [A393;s-Bu,F,H,F,H,H], [A394;s-Bu,Cl,Cl,H,H,H], [A395;s-Bu,Cl,H,Cl,H,H], [A396;s-Bu,Br,Br,H,H,H], [A397;s-Bu,Br,H,Br,H,H], [A398;s-Bu,I,I,H,H,H], [A399;s-Bu,I,H,I,H,H], [A400;s-Bu,F,Me,F,H,H], [A401;s-Bu,F,F,F,H,H], [A402;s-Bu,F,Cl,F,H,H], [A403;s-Bu,F,Br,F,H,H], [A404;s-Bu,F,I,F,H,H], [A405;s-Bu,Me,Me,H,H,H], [A406;c-Pr,Me,Me,H,H,H], [A407;c-Pr,Me,H,Me,H,H], [A408;c-Pr,Me,Me,Me,H,H], [A409;c-Pr,F,F,H,H,H], [A410;c-Pr,F,H,F,H,H], [A411;c-Pr,Cl,Cl,H,H,H], [A412;c-Pr,Cl,H,Cl,H,H], [A413;c-Pr,Br,Br,H,H,H], [A414;c-Pr,Br,H,Br,H,H], [A415;c-Pr,I,I,H,H,H], [A416;c-Pr,I,H,I,H,H], [A417;c-Pr,F,Me,F,H,H], [A418;c-Pr,F,F,F,H,H], [A419;c-Pr,F,Cl,F,H,H], [A420;c-Pr,F,Br,F,H,H], [A421;c-Pr,F,I,F,H,H], [A422;c-Pr,Me,Me,H,H,H], [A423;CH₂(c-Pr),Me,Me,H,H,H], [A424;CH₂ (c-Pr),Me,H,Me,H,H], [A425;CH₂(c-Pr),Me,Me,Me,H,H], [A426;CH₂(c-Pr),F,F,H,H,H], [A427;CH₂(c-Pr),F,H,F,H,H], [A428;CH₂(c-Pr),Cl,Cl,H,H,H], [A429;CH₂(c-Pr),Cl,H,Cl,H,H], [A430;CH₂(c-Pr),Br,Br,H,H,H], [A431;CH₂(c-Pr),Br,H,Br,H,H], [A432;CH₂(c-Pr),I,I,H,H,H], [A433;CH₂(c-Pr),I,H,I,H,H], [A434;CH₂(c-Pr),F,Me,F,H,H], [A435;CH₂(c-Pr),F,F,F,H,H], [A436;CH₂(c-Pr),F,Cl,F,H,H], [A437;CH₂(c-Pr),F,Br,F,H,H], [A438;CH₂(c-Pr),F,I,F,H,H], [A439;CH₂(c-Pr),Me,Me,H,H,H], [A440;Bn,Me,Me,H,H,H], [A441;Bn,Me,H,Me,H,H], [A442;Bn,Me,Me,Me,H,H], [A443;Bn,F,F,H,H,H], [A444;Bn,F,H,F,H,H], [A445;Bn,Cl,Cl,H,H,H], [A446;Bn,Cl,H,Cl,H,H], [A447;Bn,Br,Br,H,H,H], [A448;Bn,Br,H,Br,H,H], [A449;Bn,I,I,H,H,H], [A450;Bn,I,H,I,H,H], [A451;Bn,F,Me,F,H,H], [A452;Bn,F,F,F,H,H], [A453;Bn,F,Cl,F,H,H], [A454;Bn,F,Br,F,H,H], [A455;Bn,F,I,F,H,H], [A456;Bn,Me,Me,H,H,H], [A457;Ph,Me,Me,H,H,H], [A458;Ph,Me,H,Me,H,H], [A459;Ph,Me,Me,Me,H,H], [A460;Ph,F,F,H,H,H], [A461;Ph,F,H,F,H,H], [A462;Ph,Cl,Cl,H,H,H], [A463;Ph,Cl,H,Cl,H,H], [A464;Ph,Br,Br,H,H,H], [A465;Ph,Br,H,Br,H,H], [A466;Ph,I,I,H,H,H], [A467;Ph,I,H,I,H,H], [A468;Ph,F,Me,F,H,H], [A469;Ph,F,F,F,H,H], [A470;Ph,F,Cl,F,H,H], [A471;Ph,F,Br,F,H,H], [A472;Ph,F,I,F,H,H], [A473;Ph,Me,Me,H,H,H], [A474;2-Py,Me,Me,H,H,H], [A475;2-Py,Me,H,Me,H,H], [A476;2-Py,Me,Me,Me,H,H], [A477;2-Py,F,F,H,H,H], [A478;2-Py,F,H,F,H,H], [A479;2-Py,Cl,Cl,H,H,H], [A480;2-Py,Cl,H,Cl,H,H], [A481;2-Py,Br,Br,H,H,H], [A482;2-Py,Br,H,Br,H,H], [A483;2-Py,I,I,H,H,H], [A484;2-Py,I,H,I,H,H], [A485;2-Py,F,Me,F,H,H], [A486;2-Py,F,F,F,H,H], [A487;2-Py,F,Cl,F,H,H], [A488;2-Py,F,Br,F,H,H], [A489;2-Py,F,I,F,H,H], [A490;2-Py,Me,Me,H,H,H], [A491;3-Py,Me,Me,H,H,H], [A492;3-Py,Me,H,Me,H,H], [A493;3-Py,Me,Me,Me,H,H], [A494;3-Py,F,F,H,H,H], [A495;3-Py,F,H,F,H,H], [A496;3-Py,Cl,Cl,H,H,H], [A497;3-Py,Cl,H,Cl,H,H], [A498;3-Py,Br,Br,H,H,H], [A499;3-Py,Br,H,Br,H,H], [A500;3-Py,I,I,H,H,H], [A501;3-Py,I,H,I,H,H], [A502;3-Py,F,Me,F,H,H], [A503;3-Py,F,F,F,H,H], [A504;3-Py,F,Cl,F,H,H], [A505;3-Py,F,Br,F,H,H], [A506;3-Py,F,I,F,H,H], [A507;3-Py,Me,Me,H,H,H], [A508;4-Py,Me,Me,H,H,H], [A509;4-Py,Me,H,Me,H,H], [A510;4-Py,Me,Me,Me,H,H], [A511;4-Py,F,F,H,H,H], [A512;4-Py,F,H,F,H,H], [A513;4-Py,Cl,Cl,H,H,H], [A514;4-Py,Cl,H,Cl,H,H], [A515;4-Py,Br,Br,H,H,H], [A516;4-Py,Br,H,Br,H,H], [A517;4-Py,I,I,H,H,H], [A518;4-Py,I,H,I,H,H], [A519;4-Py,F,Me,F,H,H], [A520;4-Py,F,F,F,H,H], [A521;4-Py,F,Cl,F,H,H], [A522;4-Py,F,Br,F,H,H], [A523;4-Py,F,I,F,H,H], [A524;4-Py,Me,Me,H,H,H], [A525;2-Pyrimidyl,Me,Me,H,H,H], [A526;2-Pyrimidyl,Me,H,Me,H,H], [A527;2-Pyrimidyl,Me,Me,Me,H,H], [A528;2-Pyrimidyl,F,F,H,H,H], [A529;2-Pyrimidyl,F,H,F,H,H], [A530;2-Pyrimidyl,Cl,Cl,H,H,H], [A531;2-Pyrimidyl,Cl,H,Cl,H,H], [AS32;2-Pyrimidyl,Br,Br,H,H,H], [AS33;2-Pyrimidyl,Br,H,Br,H,H], [A534;2-Pyrimidyl,I,I,H,H,H], [A535;2-Pyrimidyl,I,H,I,H,H], [A536;2-Pyrimidyl,F,Me,F,H,H], [A537;2-Pyrimidyl,F,F,F,H,H], [A538;2-Pyrimidyl,F,Cl,F,H,H], [A539;2-Pyrimidyl,F,Br,F,H,H], [A540;2-Pyrimidyl,F,I,F,H,H], [A541;2-Pyrimidyl,Me,Me,H,H,H], [A542;4-Pyrimidyl,Me,Me,H,H,H], [A543;4-Pyrimidyl,Me,H,Me,H,H], [A544;4-Pyrimidyl,Me,Me,Me,H,H], [A545;4-Pyrimidyl,F,F,H,H,H], [A546;4-Pyrimidyl,F,H,F,H,H], [A547;4-Pyrimidyl,Cl,Cl,H,H,H], [A548;4-Pyrimidyl,Cl,H,Cl,H,H], [A549;4-Pyrimidyl,Br,Br,H,H,H], [A550;4-Pyrimidyl,Br,H,Br,H,H], [A551;4-Pyrimidyl,I,I,H,H,H], [A552;4-Pyrimidyl,I,H,I,H,H], [A553;4-Pyrimidyl,F,Me,F,H,H], [A554;4-Pyrimidyl,F,F,F,H,H], [A555;4-Pyrimidyl,F,Cl,F,H,H], [A556;4-Pyrimidyl,F,Br,F,H,H], [A557;4-Pyrimidyl,F,I,F,H,H], [A558;4-Pyrimidyl,Me,Me,H,H,H], [A559;2-Pyradinyl,Me,Me,H,H,H], [A560;2-Pyradinyl,Me,H,Me,H,H], [A561;2-Pyradinyl,Me,Me,Me,H,H], [A562;2-Pyradinyl,F,F,H,H,H], [A563;2-Pyradinyl,F,H,F,H,H], [A564;2-Pyradinyl,Cl,Cl,H,H,H], [A565;2-Pyradinyl,Cl,H,Cl,H,H], [A566;2-Pyradinyl,Br,Br,H,H,H], [A567;2-Pyradinyl,Br,H,Br,H,H], [A568;2-Pyradinyl,I,I,H,H,H], [A569;2-Pyradinyl,I,H,I,H,H], [A570;2-Pyradinyl,F,Me,F,H,H], [A571;2-Pyradinyl,F,F,F,H,H], [A572;2-Pyradinyl,F,Cl,F,H,H], [A573;2-Pyradinyl,F,Br,F,H,H], [A574;2-Pyradinyl,F,I,F,H,H], [A575;2-Pyradinyl,Me,Me,H,H,H], [A576;2-furyl,Me,Me,H,H,H], [A577;2-furyl,Me,H,Me,H,H], [A578;2-furyl,Me,Me,Me,H,H], [A579;2-furyl,F,F,H,H,H], [A580;2-furyl,F,H,F,H,H], [A581;2-furyl,Cl,Cl,H,H,H], [A582;2-furyl,Cl,H,Cl,H,H], [A583;2-furyl,Br,Br,H,H,H], [A584;2-furyl,Br,H,Br,H,H], [A585;2-furyl,I,I,H,H,H], [A586;2-furyl,I,H,I,H,H], [A587;2-furyl,F,Me,F,H,H], [A588;2-furyl,F,F,F,H,H], [A589;2-furyl,F,Cl,F,H,H], [A590;2-furyl,F,Br,F,H,H], [A591;2-furyl,F,I,F,H,H], [A592;2-furyl,Me,Me,H,H,H], [A593;3-furyl,Me,Me,H,H,H], [A594;3-furyl,Me,H,Me,H,H], [A595;3-furyl,Me,Me,Me,H,H], [A596;3-furyl,F,F,H,H,H], [A597;3-furyl,F,H,F,H,H], [A598;3-furyl,Cl,Cl,H,H,H], [A599;3-furyl,Cl,H,Cl,H,H], [A600;3-furyl,Br,Br,H,H,H], [A601;3-furyl,Br,H,Br,H,H], [A602;3-furyl,I,I,H,H,H], [A603;3-furyl,I,H,I,H,H], [A604;3-furyl,F,Me,F,H,H], [A605;3-furyl,F,F,F,H,H], [A606;3-furyl,F,Cl,F,H,H], [A607;3-furyl,F,Br,F,H,H], [A608;3-furyl,F,I,F,H,H], [A609;3-furyl,Me,Me,H,H,H], [A610;2-thienyl,Me,Me,H,H,H], [A611;2-thienyl,Me,H,Me,H,H], [A612;2-thienyl,Me,Me,Me,H,H], [A613;2-thienyl,F,F,H,H,H], [A614;2-thienyl,F,H,F,H,H], [A615;2-thienyl,Cl,Cl,H,H,H], [A616;2-thienyl,Cl,H,Cl,H,H], [A617;2-thienyl,Br,Br,H,H,H], [A618;2-thienyl,Br,H,Br,H,H], [A619;2-thienyl,I,I,H,H,H], [A620;2-thienyl,I,H,I,H,H], [A621;2-thienyl,F,Me,F,H,H], [A622;2-thienyl,F,F,F,H,H], [A623;2-thienyl,F,Cl,F,H,H], [A624;2-thienyl,F,Br,F,H,H], [A625;2-thienyl,F,I,F,H,H], [A626;2-thienyl,Me,Me,H,H,H], [A627;3-thienyl,Me,Me,H,H,H], [A628;3-thienyl,Me,H,Me,H,H], [A629;3-thienyl,Me,Me,Me,H,H], [A630;3-thienyl,F,F,H,H,H], [A631;3-thienyl,F,H,F,H,H], [A632;3-thienyl,Cl,Cl,H,H,H], [A633;3-thienyl,Cl,H,Cl,H,H], [A634;3-thienyl,Br,Br,H,H,H], [A635;3-thienyl,Br,H,Br,H,H], [A636;3-thienyl,I,I,H,H,H], [A637;3-thienyl,I,H,I,H,H], [A638;3-thienyl,F,Me,F,H,H], [A639;3-thienyl,F,F,F,H,H], [A640;3-thienyl,F,Cl,F,H,H], [A641;3-thienyl,F,Br,F,H,H], [A642;3-thienyl,F,I,F,H,H], [A643;3-thienyl,Me,Me,H,H,H], [A644;NMe₂,Me,Me,H,H,H], [A645;NMe₂,Me,H,Me,H,H], [A646;NMe₂,Me,Me,Me,H,H], [A647;NMe₂,F,F,H,H,H], [A648;NMe₂,F,H,F,H,H], [A649;NMe₂,Cl,Cl,H,H,H], [A650;NMe₂,Cl,H,Cl,H,H], [A651;NMe₂,Br,Br,H,H,H], [A652;NMe₂,Br,H,Br,H,H], [A653;NMe₂,I,I,H,H,H], [A654;NMe₂,I,H,I,H,H], [A655;NMe₂,F,Me,F,H,H], [A656;NMe₂,F,F,F,H,H], [A657;NMe₂,F,Cl,F,H,H], [A658;NMe₂,F,Br,F,H,H], [A659;NMe₂,F,I,F,H,H], [A660;NMe₂,Me,Me,H,H,H].

The compound represented by formula (A-1) wherein n is 1, each of the R^{2A}, R^{2B}, R^{2C}, and R^{2D} represents a hydrogen atom, and the combination of R¹, R^{3A}, R^{3B}, R^{3C}, R⁴ and R⁵ represents any combination described in the Combination A (hereinafter, referred to as "compound class SX2").

The compound represented by formula (A-1) wherein n is 2, each of the R^{2A}, R^{2B}, R^{2C} and R^{2D} represents a hydrogen atom, and the combination of R¹, R^{3A}, R^{3B}, R^{3C}, R⁴ and R⁵ represents any combination described in the Combination A (hereinafter, referred to as "compound class SX3").

The compound represented by formula (A-1) wherein n is 0, R^{2A} represents a methyl group, each of the R^{2B}, R^{2C} and R^{2D} represents a hydrogen atom, and the combination of R¹, R^{3A}, R^{3B}, R^{3C}, R⁴ and R⁵ represents any combination described in the Combination A (hereinafter, referred to as "compound class SX4").

The compound represented by formula (A-1) wherein n is 1, R^{2A} represents a methyl group, each of the R^{2B}, R^{2C} and R^{2D} represents a hydrogen atom, and the combination of R¹, R^{3A}, R^{3B}, R^{3C}, R⁴ and R⁵ represents any combination described in the Combination A (hereinafter, referred to as "compound class SX5").

The compound represented by formula (A-1) wherein n is 2, R^{2A} represents a methyl group, each of the R^{2B}, R^{2C} and R^{2D} represents a hydrogen atom, and the combination of R¹, R^{3A}, R^{3B}, R^{3C}, R⁴ and R⁵ represents any combination described in the Combination A (hereinafter, referred to as "compound class SX6").

The compound represented by formula (A-1) wherein n is 0, R^{2B} represents a methyl group, each of the R^{2A}, R^{2C} and R^{2D} represents a hydrogen atom, and the combination of R¹, R^{3A}, R^{3B}, R^{3C}, R⁴ and R⁵ represents any combination described in the Combination A (hereinafter, referred to as "compound class SX7").

The compound represented by formula (A-1) wherein n is 1, R^{2B} represents a methyl group, each of the R^{2A}, R^{2C} and R^{2D} represents a hydrogen atom, and the combination of R¹, R^{3A}, R^{3B}, R^{3C}, R⁴ and R⁵ represents any combination described in the Combination A (hereinafter, referred to as "compound class SX8").

The compound represented by formula (A-1) wherein n is 2, R^{2B} represents a methyl group, each of the R^{2A}, R^{2C} and R^{2D} represents a hydrogen atom, and the combination of R¹, R^{3A}, R^{3B}, R^{3C}, R⁴ and R⁵ represents any combination described in the Combination A (hereinafter, referred to as "compound class SX9").

The compound represented by formula (A-1) wherein n is 0, R^{2C} represents a methyl group, each of the R^{2A}, R^{2B} and R^{2D} represents a hydrogen atom, and the combination of R¹, R^{3A}, R^{3B}, R^{3C}, R⁴ and R⁵ represents any combination described in the Combination A (hereinafter, referred to as "compound class SX10").

The compound represented by formula (A-1) wherein n is 1, R^{2C} represents a methyl group, each of the R^{2A}, R^{2B} and R^{2D} represents a hydrogen atom, and the combination of R¹, R^{3A}, R^{3B}, R^{3C}, R⁴ and R⁵ represents any combination described in the Combination A (hereinafter, referred to as "compound class SX11").

The compound represented by formula (A-1) wherein n is 2, R^{2C} represents a methyl group, each of the R^{2A}, R^{2B} and R^{2D} represents a hydrogen atom, and the combination of R¹, R^{3A}, R^{3B}, R^{3C}, R⁴ and R⁵ represents any combination described in the Combination A (hereinafter, referred to as "compound class SX12").

The compound represented by formula (A-1) wherein n is 0, R^{2D} represents a methyl group, each of the R^{2A}, R^{2B} and R^{2C} represents a hydrogen atom, and the combination of R¹, R^{3A}, R^{3B}, R^{3C}, R⁴ and R⁵ represents any combination described in the Combination A (hereinafter, referred to as "compound class SX13").

The compound represented by formula (A-1) wherein n is 1, R^{2D} represents a methyl group, each of the R^{2A}, R^{2B} and R^{2C} represents a hydrogen atom, and the combination of R¹, R^{3A}, R^{3B}, R^{3C}, R⁴ and R⁵ represents any combination described in the Combination A (hereinafter, referred to as "compound class SX14").

The compound represented by formula (A-1) wherein n is 2, R^{2D} represents a methyl group, each of the R^{2A}, R^{2B} and R^{2C} represents a hydrogen atom, and the combination of R¹, R^{3A}, R^{3B}, R^{3C}, R⁴ and R⁵ represents any combination described in the Combination A (hereinafter, referred to as "compound class SX15").

The compound represented by formula (A-1) wherein n is 0, R^{2A} represents a chlorine atom, each of the R^{2B}, R^{2C} and R^{2D} represents a hydrogen atom, and the combination of R¹, R^{3A}, R^{3B}, R^{3C}, R⁴ and R⁵ represents any combination described in the Combination A (hereinafter, referred to as "compound class SX16").

The compound represented by formula (A-1) wherein n is 1, R^{2A} represents a chlorine atom, each of the R^{2B}, R^{2C} and R^{2D} represents a hydrogen atom, and the combination of R¹, R^{3A}, R^{3B}, R^{3C}, R⁴ and R⁵ represents any combination described in the Combination A (hereinafter, referred to as "compound class SX17").

The compound represented by formula (A-1) wherein n is 2, R^{2A} represents a chlorine atom, each of the R^{2B}, R^{2C} and R^{2D} represents a hydrogen atom, and the combination of R¹, R^{3A}, R^{3B}, R^{3C}, R⁴ and R⁵ represents any combination described in the Combination A (hereinafter, referred to as "compound class SX18").

The compound represented by formula (A-1) wherein n is 0, R^{2B} represents a chlorine atom, each of the R^{2A}, R^{2C} and R^{2D} represents a hydrogen atom, and the combination of R¹, R^{3A}, R^{3B}, R^{3C}, R⁴ and R⁵ represents any combination described in the Combination A (hereinafter, referred to as "compound class SX19").

The compound represented by formula (A-1) wherein n is 1, R^{2B} represents a chlorine atom, each of the R^{2A}, R^{2C} and R^{2D} represents a hydrogen atom, and the combination of R¹, R^{3A}, R^{3B}, R^{3C}, R⁴ and R⁵ represents any combination described in the Combination A (hereinafter, referred to as "compound class SX20").

The compound represented by formula (A-1) wherein n is 2, R^{2B} represents a chlorine atom, each of the R^{2A}, R^{2C} and R^{2D} represents a hydrogen atom, and the combination of R¹, R^{3A}, R^{3B}, R^{3C}, R⁴ and R⁵ represents any combination described in the Combination A (hereinafter, referred to as "compound class SX21").

The compound represented by formula (A-1) wherein n is 0, R^{2C} represents a chlorine atom, each of the R^{2A}, R^{2B} and R^{2D} represents a hydrogen atom, and the combination of R¹, R^{3A}, R^{3B}, R^{3C}, R⁴ and R⁵ represents any combination described in the Combination A (hereinafter, referred to as "compound class SX22").

The compound represented by formula (A-1) wherein n is 1, R^{2C} represents a chlorine atom, each of the R^{2A}, R^{2B} and R^{2D} represents a hydrogen atom, and the combination of R¹, R^{3A}, R^{3B}, R^{3C}, R⁴ and R⁵ represents any combination described in the Combination A (hereinafter, referred to as "compound class SX23").

The compound represented by formula (A-1) wherein n is 2, R^{2C} represents a chlorine atom, each of the R^{2A}, R^{2B} and R^{2D} represents a hydrogen atom, and the combination of R¹, R^{3A}, R^{3B}, R^{3C}, R⁴ and R⁵ represents any combination described in the Combination A (hereinafter, referred to as "compound class SX24").

The compound represented by formula (A-1) wherein n is 0, R^{2D} represents a chlorine atom, each of the R^{2A}, R^{2B} and R^{2C} represents a hydrogen atom, and the combination of R¹, R^{3A}, R^{3B}, R^{3C}, R⁴ and R⁵ represents any combination described in the Combination A (hereinafter, referred to as "compound class SX25").

The compound represented by formula (A-1) wherein n is 1, R^{2D} represents a chlorine atom, each of the R^{2A}, R^{2B} and R^{2C} represents a hydrogen atom, and the combination of R¹, R^{3A}, R^{3B}, R^{3C}, R⁴ and R⁵ represents any combination described in the Combination A (hereinafter, referred to as "compound class SX26").

The compound represented by formula (A-1) wherein n is 2, R^{2D} represents a chlorine atom, each of the R^{2A}, R^{2B} and R^{2C} represents a hydrogen atom, and the combination of R¹, R^{3A}, R^{3B}, R^{3C}, R⁴ and R⁵ represents any combination described in the Combination A (hereinafter, referred to as "compound class SX27").

A compound represented by formula (B-1) 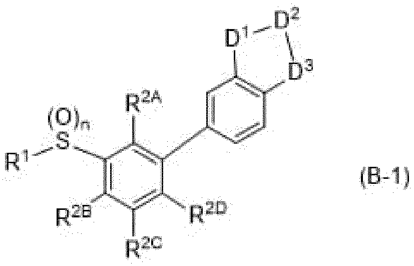 wherein n is 0, each of R^{2A}, R^{2B}, R^{2C} and R^{2D} represents a hydrogen atom, and a combination of R¹, D¹, D² and D³ represents a combination described in Combination B (hereinafter, referred to as "Compound class SX28").

The combination B consists of the Substituent Nos. B1 to B192. The substituent Nos. B1 to B192 represent the compound represented by formula (B-1) wherein the substituent No. represents a combination of R¹, D¹, D² and D³, which is described as [Substituent No.; R¹, D¹, D², D³] below.

For example, the Substituent No. B5 represents the combination in which R¹ represents an ethyl group, D1 represents C(CH₃)₂, and each of D² and D³ represents CH₂.

### Combination B

[Substituent No.;R¹,D¹,D²,D³]:
[B1;Et,CH₂,CH₂,CH₂], [B2;Et,O,CH₂,O], [B3;Et,O,CF₂,O], [B4;Et,CH₂,O,CH₂], [B5;Et,C(CH₃)₂,CH₂,CH₂], [B6;Et,CH₂,CH₂,C(CH₃)₂], [B7;Et,C(CH₃)₂,CH₂,CH(CH₃)], [B8;Et,CH(CH₃),CH₂,C(CH₃)₂], [B9;CH=CH₂,CH₂,CH₂,CH₂], [B10;CH=CH₂,O,CH₂,O], [B11;CH=CH₂,O,CF₂,O], [B12;CH=CH₂,CH₂,O,CH₂], [B13;CH=CH₂,C(CH₃)₂,CH₂,CH₂], [B14;CH=CH₂,CH₂,CH₂,C(CH₃)₂], [B15;CH=CH₂,C(CH₃)₂,CH₂,CH(CH₃)], [B16;CH=CH₂,CH(CH₃),CH₂,C(CH₃)2], [B17;Pr,CH₂,CH₂,CH₂], [B18;Pr,O,CH₂,O], [B19;Pr,O,CF₂,O], [B20;Pr,CH₂,O,CH₂], [B21;Pr,C(CH₃)₂,CH₂,CH₂], [B22;Pr,CH₂,CH₂,C(CH₃)₂], [B23;Pr,C(CH₃)₂,CH₂,CH(CH₃)], [B24;Pr,CH(CH₃),CH₂,C(CH₃)₂], [B25;CH=CHCH₃,CH₂,CH₂,CH₂], [B26;CH=CHCH₃,O,CH₂,O], [B27;CH=CHCH₃,O,CF₂,O], [B28;CH=CHCH₃,CH₂,O,CH₂], [B29;CH=CHCH₃,C(CH₃)₂,CH₂,CH₂], [B30;CH=CHCH₃,CH₂,CH₂,C(CH₃)₂], [B31;CH=CHCH₃,C(CH₃)₂,CH₂,CH(CH₃)], [B32;CH=CHCH₃,CH(CH₃),CH₂,C(CH₃)₂], [B33;CH₂CH=CH₂,CH₂,CH₂,CH₂], [B34;CH₂CH=CH₂,O,CH₂,O], [B35;CH₂CH=CH₂,O,CF₂,O], [B36;CH₂CH=CH₂,CH₂,O,CH₂], [B37;CH₂CH=CH₂,C(CH₃)₂,CH₂,CH₂], [B38;CH₂CH=CH₂,CH₂,CH₂,C(CH₃)₂], [B39;CH₂CH=CH₂,C(CH₃)₂,CH₂,CH(CH₃)], [B40;CH₂CH=CH₂,CH(CH₃),CH₂,C(CH₃)₂], [B41;i-Pr,CH₂,CH₂,CH₂], [B42;i-Pr,O,CH₂,O], [B43;i-Pr,O,CF₂,O], [B44;i-Pr,CH₂,O,CH₂], [B45;i-Pr,C(CH₃)₂,CH₂,CH₂], [B46;i-Pr,CH₂,CH₂,C(CH₃)₂], [B47;i-Pr,C(CH₃)₂,CH₂,CH(CH₃)], [B48;i-Pr,CH(CH₃),CH₂,C(CH₃)₂], [B49;Bu,CH₂,CH₂,CH₂], [B50;Bu,O,CH₂,O], [B51;Bu,O,CF₂,O], [B52;Bu,CH₂,O,CH₂], [B53;Bu,C(CH₃)₂,CH₂,CH₂], [B54;Bu,CH₂,CH₂,C(CH₃)₂], [B55;Bu,C(CH₃)₂,CH₂,CH(CH₃)], [B56;Bu,CH(CH₃),CH₂,C(CH₃)₂], [B57;i-Bu,CH₂,CH₂,CH₂], [B5S;i-Bu,O,CH₂,O], [B59;i-Bu,O,CF₂,O], [B60;i-Bu,CH₂,O,CH₂], [B61;i-Bu,C(CH₃)₂,CH₂,CH₂], [B62;i-Bu,CH₂,CH₂,C(CH₃)₂], [B63;i-Bu,C(CH₃)₂,CH₂,CH(CH₃)], [B64;i-Bu,CH(CH₃),CH₂,C(CH₃)₂], [B65;s-Bu,CH₂,CH₂,CH₂], [B66;s-Bu,O,CH₂,O], [B67;s-Bu,O,CF₂,O], [B68;s-Bu,CH₂,O,CH₂], [B69;s-Bu,C(CH₃)₂,CH₂,CH₂], [B70;s-Bu,CH₂,CH₂,C(CH₃)₂], [B71;s-Bu,C(CH₃)₂,CH₂,CH(CH₃)], [B72;s-Bu,CH(CH₃),CH₂,C(CH₃)₂], [B73;c-Pr,CH₂,CH₂,CH₂], [B74;c-Pr,O,CH₂,O], [B75;c-Pr,O,CF₂,O], [B76;c-Pr,CH₂,O,CH₂], [B77;c-Pr,C(CH₃)₂,CH₂,CH₂], [B78;c-Pr,CH₂,CH₂,C(CH₃)₂], [B79;c-Pr,C(CH₃)₂,CH₂,CH(CH₃)], [B80;c-Pr,CH(CH₃),CH₂,C(CH₃)₂], [B81;CH₂(c-Pr),CH₂,CH₂,CH₂], [B82;CH₂(c-Pr),O,CH₂,O], [B83;CH₂(c-Pr),O,CF₂,O], [BS4;CH₂(c-Pr),CH₂,O,CH₂], [B85;CH₂(c-Pr),C(CH₃)₂,CH₂,CH₂], [B86;CH₂(c-Pr),CH₂,CH₂,C(CH₃)₂], [B87;CH₂(c-Pr),C(CH₃)₂,CH₂,CH(CH₃)], [B88;CH₂(c-Pr),CH(CH₃),CH₂,C(CH₃)₂], [B89;Bn,CH₂,CH₂,CH₂], [B90;Bn,O,CH₂,O], [B91;Bn,O,CF₂,O], [B92;Bn,CH₂,O,CH₂], [B93;Bn,C(CH₃)₂,CH₂,CH₂], [B94;Bn,CH₂,CH₂,C(CH₃)₂], [B95;Bn,C(CH₃)₂,CH₂,CH(CH₃)], [B96;Bn,CH(CH₃),CH₂,C(CH₃)₂], [B97;Ph,CH₂,CH₂,CH₂], [B98;Ph,O,CH₂,O], [B99;Ph,O,CF₂,O], [B100;Ph,CH₂,O,CH₂], [B101;Ph,C(CH₃)₂,CH₂,CH₂], [B102;Ph,CH₂,CH₂,C(CH₃)₂], [B103;Ph,C(CH₃)₂,CH₂,CH(CH₃)], [B104;Ph,CH(CH₃),CH₂,C(CH₃)₂], [B105;2-Py,CH₂,CH₂,CH₂], [B106;2-Py,O,CH₂,O], [B107;2-Py,O,CF₂,O], [B108;2-Py,CH₂,O,CH₂], [B109;2-Py,C(CH₃)₂,CH₂,CH₂], [B110;2-Py,CH₂,CH₂,C(CH₃)₂], [B111;2-Py,C(CH₃)₂,CH₂,CH(CH₃)], [B112;2-Py,CH(CH₃),CH₂,C(CH₃)₂], [B113;3-Py,CH₂,CH₂,CH₂], [B114;3-Py,O,CH₂,O], [B115;3-Py,O,CF₂,O], [B116;3-Py,CH₂,O,CH₂], [B117;3-Py,C(CH₃)₂,CH₂,CH₂], [B118;3-Py,CH₂,CH₂,C(CH₃)₂], [B119;3-Py,C(CH₃)₂,CH₂,CH(CH₃)], [B120;3-Py,CH(CH₃),CH₂,C(CH₃)₂], [B121;4-Py,CH₂,CH₂,CH₂], [B122;4-Py,O,CH₂,O], [B123; 4-Py,O,CF₂,O], [B124; 4-Py,CH₂,O,CH₂], [B125;4-Py,C(CH₃)₂,CH₂,CH₂], [B126; 4-Py,CH₂,CH₂,C(CH₃)₂], [B127;4-Py,C(CH₃)₂,CH₂,CH(CH₃)], [B128;4-Py,CH(CH₃),CH₂,C(CH₃)₂], [B129;2-Pyrimidyl,CH₂,CH₂,CH₂], [B130;2-Pyrimidyl,O,CH₂,O], [B131;2-Pyrimidyl,O,CF₂,O], [B132;2-Pyrimidyl,CH₂,O,CH₂], [B133;2-Pyrimidyl, C(CH₃)₂,CH₂,CH₂], [B134;2-Pyrimidyl, CH₂,CH₂,C(CH₃)₂], [B135;2-Pyrimidyl, C(CH₃)₂,CH₂,CH(CH₃)], [B136;2-Pyrimidyl, CH(CH₃),CH₂,C(CH₃)₂], [B137;4-Pyrimidyl, CH₂,CH₂,CH₂], [B138;4-Pyrimidyl,O,CH₂,O], [B139; 4-Pyrimidyl,O,CF₂,O], [B140;4-Pyrimidyl,CH₂,O,CH₂], [B141;4-Pyrimidyl, C(CH₃)₂,CH₂,CH₂], [B142;4-Pyrimidyl, CH₂,CH₂,C(CH₃)₂], [B143;4-Pyrimidyl, C(CH₃)₂,CH₂,CH(CH₃)], [B144;4-Pyrimidyl,CH(CH₃),CH₂,C(CH₃)₂], [B145;2-Pyradinyl,CH₂,CH₂,CH₂], [B146;2-Pyradinyl,O,CH₂,O], [B147;2-Pyradinyl,O,CF₂,O], [B148;2-Pyradinyl,CH₂,O,CH₂], [B149;2-Pyradinyl,C(CH₃)₂,CH₂,CH₂], [B150;2-Pyradinyl,CH₂,CH₂,C(CH₃)₂], [B151;2-Pyradinyl,C(CH₃)₂,CH₂,CH(CH₃)], [B152;2-Pyradinyl,CH(CH₃),CH₂,C(CH₃)₂], [B153;2-furyl,CH₂,CH₂,CH₂], [B154;2-furyl,O,CH₂,O], [B155;2-furyl,O,CF₂,O], [B156;2-furyl, CH₂,O,CH₂], [B157;2-furyl,C (CH₃)₂,CH₂,CH₂], [B158;2-furyl, CH₂,CH₂,C(CH₃)₂], [B159;2-furyl,C (CH₃)₂,CH₂,CH(CH₃)], [B160;2-furyl,CH(CH₃),CH₂,C(CH₃)₂], [B161;3-furyl, CH₂,CH₂,CH₂], [B162;3-furyl,O,CH₂,O], [B163;3-furyl,O,CF₂,O], [B164;3-furyl,CH₂,O,CH₂], [B165;3-furyl,C(CH₃)₂,CH₂,CH₂], [B166;3-furyl,CH₂,CH₂,C(CH₃)₂], [B167;3-furyl,C(CH₃)₂,CH₂,CH(CH₃)], [B168;3-furyl,CH(CH₃),CH₂,C(CH₃)₂], [B169;2-thienyl,CH₂,CH₂,CH₂], [B170;2-thienyl,O,CH₂,O], [B171;2-thienyl,O,CF₂,O], [B172;2-thienyl, CH₂,O,CH₂], [B173;2-thienyl, C(CH₃)₂,CH₂,CH₂], [B174;2-thienyl,CH₂,CH₂,C(CH₃)₂], [B175;2-thienyl, C(CH₃)₂,CH₂,CH(CH₃)], [B176;2-thienyl, CH(CH₃),CH₂,C(CH₃)₂], [B177;3-thienyl,CH₂,CH₂,CH₂], [B178;3-thienyl,O,CH₂,O], [B179;3-thienyl,O,CF₂,O], [B180;3-thienyl, CH₂,O,CH₂], [B181;3-thienyl,C(CH₃)₂,CH₂,CH₂], [B182;3-thienyl,CH₂,CH₂,C(CH₃)₂], [B183;3-thienyl, C(CH₃)₂,CH₂,CH(CH₃)], [B184;3-thienyl, CH(CH₃),CH₂,C(CH₃)₂], [B185;NMe₂,CH₂,CH₂,CH₂], [B186;NMe₂,O,CH₂,O], [B187;NMe₂,O,CF₂,O], [B188;NMe₂,CH₂,O,CH₂], [B189;NMe₂,C(CH₃)₂,CH₂,CH₂], [B190;NMe₂,CH₂,CH₂,C (CH₃)₂], [B191;NMe₂,C (CH₃)₂,CH₂,CH (CH₃)], [B192;NMe₂,CH (CH₃), CH₂,C (CH₃)₂].

The compound represented by formula (B-1) wherein n is 1, each of R^{2A}, R^{2B}, R^{2C} and R^{2D} represents a hydrogen atom, and a combination of R¹, D¹, D² and D³ represents any combination described in the combination B (hereinafter, referred to "Compound class SX29").

The compound represented by formula (B-1) wherein n is 2, each of R^{2A}, R^{2B}, R^{2C} and R^{2D} represents a hydrogen atom, and a combination of R¹, D¹, D² and D³ represents any combination described in the combination B (hereinafter, referred to "Compound class SX30").

The compound represented by formula (B-1) wherein n is 0, R^{2A} represents a methyl group, each of R^{2B}, R^{2C} and R^{2D} represents a hydrogen atom, and a combination of R¹, D¹, D² and D³ represents any combination described in the combination B (hereinafter, referred to "Compound class SX31").

The compound represented by formula (B-1) wherein n is 1, R^{2A} represents a methyl group, each of R^{2B}, R^{2C} and R^{2D} represents a hydrogen atom, and a combination of R¹, D¹, D² and D³ represents any combination described in the combination B (hereinafter, referred to "Compound class SX32").

The compound represented by formula (B-1) wherein n is 2, R^{2A} represents a methyl group, each of R^{2B}, R^{2C} and R^{2D} represents a hydrogen atom, and a combination of R¹, D¹, D² and D³ represents any combination described in the combination B (hereinafter, referred to "Compound class SX33").

The compound represented by formula (B-1) wherein n is 0, R^{2B} represents a methyl group, each of R^{2A}, R^{2C} and R^{2D} represents a hydrogen atom, and a combination of R¹, D¹, D² and D³ represents any combination described in the combination B (hereinafter, referred to "Compound class SX34").

The compound represented by formula (B-1) wherein n is 1, R^{2B} represents a methyl group, each of R^{2A}, R^{2C} and R^{2D} represents a hydrogen atom, and a combination of R¹, D¹, D² and D³ represents any combination described in the combination B (hereinafter, referred to "Compound class SX35").

The compound represented by formula (B-1) wherein n is 2, R^{2B} represents a methyl group, each of R^{2A}, R^{2C} and R^{2D} represents a hydrogen atom, and a combination of R¹, D¹, D² and D³ represents any combination described in the combination B (hereinafter, referred to "Compound class SX36").

The compound represented by formula (B-1) wherein n is 0, R^{2C} represents a methyl group, each of R^{2A}, R^{2B} and R^{2D} represents a hydrogen atom, and a combination of R¹, D¹, D² and D³ represents any combination described in the combination B (hereinafter, referred to "Compound class SX37").

The compound represented by formula (B-1) wherein n is 1, R^{2C} represents a methyl group, each of R^{2A}, R^{2B} and R^{2D} represents a hydrogen atom, and a combination of R¹, D¹, D² and D³ represents any combination described in the combination B (hereinafter, referred to "Compound class SX38").

The compound represented by formula (B-1) wherein n is 2, R^{2C} represents a methyl group, each of R^{2A}, R^{2B} and R^{2D} represents a hydrogen atom, and a combination of R¹, D¹, D² and D³ represents any combination described in the combination B (hereinafter, referred to "Compound class SX39").

The compound represented by formula (B-1) wherein n is 0, R^{2D} represents a methyl group, each of R^{2A}, R^{2B} and R^{2C} represents a hydrogen atom, and a combination of R¹, D¹, D² and D³ represents any combination described in the combination B (hereinafter, referred to "Compound class SX40").

The compound represented by formula (B-1) wherein n is 1, R^{2D} represents a methyl group, each of R^{2A}, R^{2B} and R^{2C} represents a hydrogen atom, and a combination of R¹, D¹, D² and D³ represents any combination described in the combination B (hereinafter, referred to "Compound class SX41").

The compound represented by formula (B-1) wherein n is 2, R^{2D} represents a methyl group, each of R^{2A}, R^{2B} and R^{2C} represents a hydrogen atom, and a combination of R¹, D¹, D² and D³ represents any combination described in the combination B (hereinafter, referred to "Compound class SX42").

The compound represented by formula (B-1) wherein n is 0, R^{2A} represents a chlorine atom, each of R^{2B}, R^{2C} and R^{2D} represents a hydrogen atom, and a combination of R¹, D¹, D² and D³ represents any combination described in the combination B (hereinafter, referred to "Compound class SX43").

The compound represented by formula (B-1) wherein n is 1, R^{2A} represents a chlorine atom, each of R^{2B}, R^{2C} and R^{2D} represents a hydrogen atom, and a combination of R¹, D¹, D² and D³ represents any combination described in the combination B (hereinafter, referred to "Compound class SX44").

The compound represented by formula (B-1) wherein n is 2, R^{2A} represents a chlorine atom, each of R^{2B}, R^{2C} and R^{2D} represents a hydrogen atom, and a combination of R¹, D¹, D² and D³ represents any combination described in the combination B (hereinafter, referred to "Compound class SX45").

The compound represented by formula (B-1) wherein n is 0, R^{2B} represents a chlorine atom, each of R^{2A}, R^{2C} and R^{2D} represents a hydrogen atom, and a combination of R¹, D¹, D² and D³ represents any combination described in the combination B (hereinafter, referred to "Compound class SX46")

The compound represented by formula (B-1) wherein n is 1, R^{2B} represents a chlorine atom, each of R^{2A}, R^{2C} and R^{2D} represents a hydrogen atom, and a combination of R¹, D¹, D² and D³ represents any combination described in the combination B (hereinafter, referred to "Compound class SX47")

The compound represented by formula (B-1) wherein n is 2, R^{2B} represents a chlorine atom, each of R^{2A}, R^{2C} and R^{2D} represents a hydrogen atom, and a combination of R¹, D¹, D² and D³ represents any combination described in the combination B (hereinafter, referred to "Compound class SX48")

The compound represented by formula (B-1) wherein n is 0, R^{2C} represents a chlorine atom, each of R^{2A}, R^{2B} and R^{2D} represents a hydrogen atom, and a combination of R¹, D¹, D² and D³ represents any combination described in the combination B (hereinafter, referred to "Compound class SX49")

The compound represented by formula (B-1) wherein n is 1, R^{2C} represents a chlorine atom, each of R^{2A}, R^{2B} and R^{2D} represents a hydrogen atom, and a combination of R¹, D¹, D² and D³ represents any combination described in the combination B (hereinafter, referred to "Compound class SX50")

The compound represented by formula (B-1) wherein n is 2, R^{2C} represents a chlorine atom, each of R^{2A}, R^{2B} and R^{2D} represents a hydrogen atom, and a combination of R¹, D¹, D² and D³ represents any combination described in the combination B (hereinafter, referred to "Compound class SX51")

The compound represented by formula (B-1) wherein n is 0, R^{2D} represents a chlorine atom, each of R^{2A}, R^{2B} and R^{2D} represents a hydrogen atom, and a combination of R¹, D¹, D² and D³ represents any combination described in the combination B (hereinafter, referred to "Compound class SX52")

The compound represented by formula (B-1) wherein n is 1, R^{2D} represents a chlorine atom, each of R^{2A}, R^{2B} and R^{2D} represents a hydrogen atom, and a combination of R¹, D¹, D² and D³ represents any combination described in the combination B (hereinafter, referred to "Compound class SX53"),

The compound represented by formula (B-1) wherein n is 2, R^{2D} represents a chlorine atom, each of R^{2A}, R^{2B} and R^{2D} represents a hydrogen atom, and a combination of R¹, D¹, D² and D³ represents any combination described in the combination B (hereinafter, referred to "Compound class SX54")

Next, the Formulation Examples are shown below. The "parts" represents "part by weight" unless otherwise specified. Also "Present compound S" represents any compound described in the compound class SX1 to SX54.

### Formulation Example 1

Thirty five (35) parts of a mixture of ammonium polyoxyethylene alkyl ether sulfate and wet silica (weight ratio: 1:1), 10 parts of any one of the present compound S, and 55 parts of water are mixed, and the mixture is then finely-ground by a wet grinding method to obtain a formulation.

### Formulation Example 2

Fifty (50) parts of any one of the present compound S, 3 parts of calcium lignin sulfonate, 2 parts of sodium lauryl sulfate, and 45 parts of silica are well mixed-grinding to obtain a formulation.

### Formulation Example 3

Five (5) parts of any one of the present compound S, 9 parts of polyoxyethylene styryl phenyl ether, 5 parts of polyoxyethylene decyl ether (Number of ethylene oxide additions: 5), 6 parts of calcium dodecylbenzene sulfonate and 75 parts of xylene are well mixed to obtain a formulation.

### Formulation Example 4

Two (2) parts of any one of the present compound S, 1 part of silica, 2 parts of calcium lignin sulfonate, 30 parts of bentonite and 65 parts of kaolin clay are mixed-grinding, and thereto is added an appropriate amount of water, and the mixture is well kneaded and is then granulated with a granulator and dried to obtain a formulation.

### Formulation Example 5

Ten (10) parts of any one of the present compound S is mixed with a mixture of 18 parts of benzyl alcohol and 9 parts of DMSO, and thereto are added 6.3 parts of GERONOL (registered trademark) TE250, 2.7 parts of Ethylan (registered trademark) NS-500LQ, and 54 parts of Solvent naphtha, and the mixture is mixed to obtain a formulation.

Next, the test examples are shown below.

The untreated groups in the Test Example 3, the Test Example 5 and the Test Example 6 represents a treated group in which the procedures are carried out under the same conditions as those described in each of Test Examples except that DMSO is dispensed in place of a diluted DMSO solution containing the Present compound. The untreated groups in the Test Example 7 and the Test Example 15 represents a test in which the procedures are carried out under the same conditions as those described in each of the Test Examples except that DMSO is dispensed in place of a diluted DMSO solution containing the present compound. Also, the untreated groups in the Test Example 8, the Test Example 9, the Test Example 10 and the Test Exmaple 11 represents a test in which a diluted aqueous solution of the formulation containing the present compound isn't sprayed.

### Test Example 1: Control test against wheat septoria leaf blotch fungus (Septoria tritici)

The present compound is diluted with DMSO so as to contain 150 ppm, and 1 µL of the dilution solutions are dispensed into titer plate (96 well), and thereafter, thereto is then dispensed 150 µL of YBG medium to which conidia of Septoria tritici are inoculated in advance. This plate is cultured at 18°C for 3 days, thereby allowing Septoria tritici to undergo proliferation, and the absorbance at 550 nm of each well of the titer plate is then measured to determine a degree of growth of Septoria tritici. As a result, each of the present compounds shows an effect on controlling plant diseases.

### Test Example 2: Control test against Corn smut fungus (Ustilago maydis)

The present compound is diluted with DMSO so as to contain 150 ppm, and 1 µL of the dilution solutions are dispensed into titer plate (96 well), and thereafter, thereto is then dispensed 150 µL of a potato dextrose broth (PDB broth) to which conidia of Ustilago maydis are inoculated in advance. This plate is cultured at 18°C for 4 days, thereby allowing Ustilago maydis to undergo proliferation, and the absorbance at 550 nm of each well of the titer plate is then measured to determine a degree of growth of the Ustilago maydis. As a result, each of the present compounds shows an effect on controlling plant diseases.

### Test Example 3: Control test against Barley scald fungus (Rhynchosporium secalis)

The present compound 77 was diluted with DMSO so as to contain 150 ppm, and 1 µL of the dilution solutions were dispensed into titer plate (96 well), and thereafter, thereto was then dispensed 150 µL of a potato dextrose broth (PDB broth) to which conidia of Rhynchosporium secalis were inoculated in advance. This plate was cultured at 18°C for 7 days, thereby allowing Rhynchosporium secalis to undergo proliferation, and the absorbance at 550 nm of each well of the titer plate was then measured to determine a degree of growth of the Rhynchosporium secalis. As a result, the growth in the well in treated groups treated with the above present compound showed 50% or less compared to the growth in an untreated well.

### Test Example 4: Control test against Cucumber botrytis rot fungus (Botrytis cinerea)

The present compound is diluted with DMSO so as to contain 150 ppm, and 1 µL of the dilution solutions are dispensed into titer plate (96 well), and thereafter, thereto is then dispensed 150 µL of complete medium to which conidia of Botrytis cinerea are inoculated in advance. This plate is cultured at 18°C for 4 days, thereby allowing Botrytis cinerea to undergo proliferation, and the absorbance at 550 nm of each well of the titer plate is then measured to determine a degree of growth of the Botrytis cinerea. As a result, each of the present compounds shows an effect on controlling plant diseases.

### Test Example 5: Control test against Peach scab fungus (Cladosporium carpophilum)

The present compound 77 was diluted with DMSO so as to contain 150 ppm, and 1 µL of the dilution solutions were dispensed into titer plate (96 well), and thereafter, thereto was then dispensed 150 µL of a potato dextrose broth (PDB broth) to which conidia of Cladosporium carpophilum were inoculated in advance. This plate was cultured at 18°C for 5 days, thereby allowing Cladosporium carpophilum to undergo proliferation, and the absorbance at 550 nm of each well of the titer plate was then measured to determine a degree of growth of the Cladosporium carpophilum. As a result, the growth in the well in treated groups treated with the above present compound showed 50% or less compared to the growth in an untreated well.

### Test Example 6: Control test against rice brown spot fungus (Cochliobolus miyabeanus)

The present compound 77 was diluted with DMSO so as to contain 150 ppm, and 1 µL of the dilution solutions were dispensed into titer plate (96 well), and thereafter, thereto was then dispensed 150 µL of YB liquid medium to which conidia of Cochliobolus miyabeanus were inoculated in advance. This plate was cultured at 23°C for 3 days, thereby allowing Cochliobolus miyabeanus to undergo proliferation, and the absorbance at 550 nm of each well of the titer plate was then measured to determine a degree of growth of the Cochliobolus miyabeanus. As a result, the growth in the well in treated groups treated with the above present compound showed 50% or less compared to the growth in an untreated well.

### Test Example 7: Control test against soybean rust (Phakopsora pachyrhizi)

Soybean leaf (cv; Kurosengoku) was punched out to 1 cm diameter to prepare a leaf disk. Each 1 mL of an agar medium (agar concentration 1.2 %) was dispensed in each well of 24 well microplate. A piece of the leaf disk was placed on agar medium on each well. To a mixture of 0.5 µL of Sorpol (registered trademark) 1200KX, 4.5 µL of DMSO, and 5 µL of xylene was added 20 µL of a solution containing 10000 ppm of the test compounds in DMSO. The resulting mixture was diluted with ion exchange water to prepare a mixture containing a predetermined concentration of the test compounds. The resulting mixture was sprayed in 10 µL per one leaf disk. After 1 day, an aqueous suspension of conidia of *Phakopsora pachyrhizi* having an amino acid substitution of F129L on mitochondrial cytochrome b protein (1.0 × 10⁵/mL) was inoculated onto the leaf disks. After the inoculation, the microplate was placed in a growth chamber (light on for 6 hours, light off for 18 hours, 23°C temperature, 60% humidity). After 1 day, the leaf disks were air-dried to disappear water droplets on the surface of the leaf disk, and the microplate was placed again in the growth chamber for 12 days. Thereafter, a lesion area of soybean rust disease was assessed. As a result, lesion areas in the leaf disk treated with any one of the present compounds 1, 2, 3, 4, 5, 6, 7, 8, 10, 11, 12, 13, 14, 15, 17, 18, 20, 21, 22, 23, 24, 25, 26, 28, 29, 30, 33, 34, 36, 37, 38, 39, 40, 45, 46, 62, 66, 67, 68, 69, 70, 71, 73, 74, 76, or 78 as a tested compound at a prescribed concentration of 50 ppm showed 30% or less compared to the lesion areas in an untreated leaf disk.

### Test Example 8: Control test against barley net blotch (Pyrenophora teres)

Each of plastic pots was filled with soil and thereto barley (cv; NISHINOHOSHI) seeds were sown and the barleys were grown in a greenhouse for 7 days. Thereafter, the present compound 1, 3, 21, 41, or 44 each of which was made to a formulation according to the similar method to that of Formulation Example 1, was mixed with water so as to be a prescribed concentration (200 ppm). The resulting mixtures were sprayed to foliar parts so as to adhere adequately on the leaves of the above-mentioned barley. After spraying the mixtures, the barleys were air-dried and after 1 day, an aqueous suspension of the conidia of Pyrenophora teres was spraying-inoculated. After the inoculation, the barleys were placed at 23°C during daytime and 20°C during nighttime under a high humidity for 3 day and then cultivated in a greenhouse for 7 days, and a lesion area was observed. As a result, every of the lesion areas in barleys treated with each of the above present compounds showed 30% or less compared to the lesion area in an untreated barley.

### Test Example 9: Control test against wheat brown rust (Puccinia recondita)

Each of plastic pots was filled with soil and thereto wheat (cv; SHIROGANE) seeds were sown and the wheats were grown in a greenhouse for 9 days. The present compound 12, 17, or 39 each of which was made to a formulation according to the similar method to that of Formulation Example 1, was mixed with water so as to be 200 ppm, and the mixtures were sprayed to foliar parts so as to adhere adequately on the leaves of the above-mentioned wheat. After spraying the mixtures, the wheats were air-dried and were then cultivated at 20°C under lighting for 5 to 7 days. The conidia of Puccinia recondita were sprinkling-inoculated. After the inoculation, the wheats were placed under a dark and humid condition at 23°C for 1 day and were then cultivated at 20°C under lighting for 8 days, and a lesion area was observed. As a result, every of the lesion areas in wheats treated with each of the above present compounds showed 30% or less compared to the lesion area in an untreated wheat.

### Test Example 10: Control test against septoria leaf blotch (Septoria tritici)

Each of plastic pots was filled with soil and thereto wheat (cv; Apogee) seeds were sown and the wheats were grown in a greenhouse for 10 days. Thereafter, the present compound 1, 2, 3, 4, 12, 22, 23, 26, 28, 43, 44, 53, 54, 55, 61, 62, or 65 each of which was made to a formulation according to the similar method to that of Formulation Example 1, was mixed with water so as to be 200 ppm. The mixtures were sprayed to foliar parts so as to adhere adequately on the leaves of the above-mentioned wheat. After spraying the mixtures, the wheats were air-dried and after 4 days, an aqueous suspension of the conidia of Septoria tritici was spraying-inoculated. After the inoculation, the wheats were placed at 18°C under a high humidity for 3 days and then under lighting for 14 to 18 days, and a lesion area was observed. As a result, every of the lesion areas in wheats treated with each of the above present compounds showed 30% or less compared to the lesion area in an untreated wheat.

### Test Example 11: Control test against soybean rust (Phakopsora pachyrhizi)

Each of plastic pots was filled with soil and thereto soybean (cv: Kurosengoku) seeds were sown and the soybeans were grown in a greenhouse for 10 to 14 days. Thereafter, the present compound 3, 4, 11, 12, 13, 20, 21, 22, 23, 24, 25, 26, 36, 45, 46, 54, 62, 64, 65, 66, 67, 68, 69, 70, 71, 72, 73, 74, 75, 76, 77, 78, 79, or 80 each of which was made to a formulation according to the similar method to that of Formulation Example 1, was mixed with water so as to be 200 ppm. The resulting mixtures were sprayed to foliar parts so as to adhere adequately on the leaves of the above-mentioned soybean. After spraying the mixtures, the soybeans were air-dried and after 2 to 5 days, an aqueous suspension of the conidia of Phakopsora pachyrhizi was spraying-inoculated. After the inoculation, the soybeans were placed in a greenhouse of 23°C during daytime and 20°C during nighttime under a high humidity for 1 to 2 days, and were then cultivated in the greenhouse for 12 days, and a lesion area was observed. As a result, every of the lesion areas in soybean treated with each of the above present compounds showed 30% or less compared to the lesion area in an untreated soybean.

### Test Example 12: Control test against soybean rust (Phakopsora pachyrhizi)

Each of plastic pots is filled with soil and thereto soybean (cv: Kurosengoku) seeds are sown and the soybeans were grown in a greenhouse for 10 days, and an aqueous suspension containing the conidia of Phakopsora pachyrhizi is spraying-inoculated. After the inoculation, the soybeans are placed in a greenhouse of 23°C during daytime and 20°C during nighttime under a high humidity for 1 day, and are then cultivated in the greenhouse for 2 days, and thereafter, the present compound each of which was made to a formulation according to the similar method to that of Formulation Example 1, is mixed with water so as to be 200 ppm, and the resulting mixtures are sprayed to foliar parts so as to adhere adequately on the leaves of the above-mentioned soybean. After spraying the mixtures, the soybeans are air-dried and cultivated in a greenhouse for 8 days, and a lesion area is then observed. As a result, each of the present compounds shows an effect on controlling plant diseases.

### Test Example 13: Control test against soybean leaf spot (Cercospora sojina)

Each of plastic pots is filled with soil and thereto soybean (cv: Tachinagaha) seeds are sown and the soybeans were grown in a greenhouse for 13 days. Thereafter, the present compound each of which is made to a formulation according to the similar method to that of Formulation Example 1, is mixed with water so as to be 200 ppm. The resulting mixtures are sprayed to foliar parts so as to adhere adequately on the leaves of the above-mentioned soybean. After spraying the mixtures, the soybeans are air-dried and after 1 day, an aqueous suspension of the conidia of Cercospora sojina is spraying-inoculated. After the inoculation, the soybeans are placed in a greenhouse of 23°C during daytime and 20°C during nighttime under a high humidity for 3 days, and are then cultivated in the greenhouse for 16 days, and a lesion area is observed. As a result, each of the present compounds shows an effect on controlling plant diseases.

### Test Example 14: Control test against tomato early blight (Alternaria solani)

Each of plastic pots is filled with soils and thereto tomato (cv; PATIO) seeds are sown and the tomatoes were grown in a greenhouse for 20 days. Thereafter, the present compound each of which is made to a formulation according to the similar method to that of Formulation Example 1, is mixed with water so as to be 200 ppm. The resulting mixtures are sprayed to foliar parts so as to adhere adequately on the leaves of the above-mentioned tomato. After spraying the mixtures, the tomatoes are air-dried and after 1 day, an aqueous suspension of the conidia of Alternaria solani are spraying-inoculated. After the inoculation, the tomatoes are placed at 18°C under a high humidity for 6 days, and a lesion area is observed. As a result, each of the present compounds shows an effect on controlling plant diseases.

### Test example 22: Control test against soybean rust (Phakopsora pachyrhizi)

The test compound was diluted with DMSO so as to contain 150 ppm, 1 µL thereof was dispensed to a titer plate (96 well), and thereafter, an aqueous suspension of soybean rust fungus spores that was suspended soybean rust spores (1.0 × 10⁴/mL) in advance 149 µL was further dispensed to the plate. This plate was cultivated at 23°C for a few hours, and thereafter, the number of germinated spores of soybean rust fungus was counted. As a result, in the case of 1 ppm as the treated concentration, the number of germinated spores in a well containing any one of the present compound 1, 2, 3, 4, 5, 6, 7, 8, 9, 10, 11, 12, 13, 14, 15, 16, 17, 18, 19, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 65, 66, 67, 68, 69, 70, 71, 73, 74, 75, 76, 77, 78, 79, or 80 as a test compound showed 50% or less compared to the number of germinated spores in an untreated group.

### INDUSTRIAL APPLICABILITY

The present compound Y has a control efficacy against plant diseases, and can be thus used to control plant diseases.

## Claims

1. A method for controlling a plant disease which comprises applying a compound represented by formula (I): 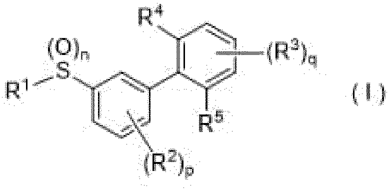 [wherein
R¹ represents a C2-C6 chain hydrocarbon group which may be optionally substituted with one or more substituents selected from Group A, a C3-C8 alicyclic hydrocarbon group which may be optionally substituted with one or more substituents selected from Group B, a three (3)- to seven (7)- membered non-aromatic heterocyclic group which may be optionally substituted with one or more substituents selected from Group B, a methyl group which is substituted with one or more substituents selected from Group C, a C6-C10 aryl group, a five (5)- to ten (10)- membered aromatic heterocyclic group {each of the C6-C10 aryl group, and the five (5)- to ten (10)- membered aromatic heterocyclic group may optionally have one or more substituents selected from Group E}, or NR²⁹R³⁰,
n is 0, 1, or 2,
R² represents a C1-C6 chain hydrocarbon group which may be optionally substituted with one or more halogen atoms, a C3-C8 alicyclic hydrocarbon group which may be optionally substituted with one or more substituents selected from Group B, OR⁶, SR⁷, S(O)R^{B}, S(O)₂R⁹, NR¹⁰R¹¹, C(O)R¹², a nitro group, a cyano group, or a halogen atom,
p is 0, 1, or 2,
when p is 2, two of the R² may be identical to or different from each other,
R³ represents a C1-C6 chain hydrocarbon group which may be optionally substituted with one or more substituents selected from Group A, a C3-C8 alicyclic hydrocarbon group, a three (3) - to eight (8) - membered non-aromatic heterocyclic group {each of the C3-C8 alicyclic hydrocarbon group, and the three (3)- to eight (8)- membered non-aromatic heterocyclic group may be optionally substituted with one or more substituents selected from Group B}, a phenyl group, a five (5)- to six (6)- membered aromatic heterocyclic group {each of the phenyl group, and the five (5)- to six (6)-membered aromatic heterocyclic group may optionally have one or more substituents selected from Group D}, OR¹³, SR¹⁴, S(O)R¹⁵, S(O)₂R¹⁶, NR¹⁷R¹⁸, C(O)R¹⁹, C(R²⁰) =NOR²¹, a cyano group, a nitro group, an amino group, a hydroxy group, or a halogen atom,
q is 1, 2, or 3,
when q is 2 or 3, two or three of the R³ may be identical to or different from each other,
R⁴ and R⁵ are identical to or different from each other, and each represents a C1-C6 chain hydrocarbon group which may be optionally substituted with one or more halogen atoms, a C3-C8 alicyclic hydrocarbon group which may be optionally substituted with one or more substituents selected from Group B, OR²², SR²³, S(O)R²⁴, S(O)₂R²⁵, NR²⁶R²⁷, C(O)R²⁸, a nitro group, a cyano group, a halogen atom, or a hydrogen atom,
R⁶, R⁷, R¹⁰, R²², R²³, R²⁶, and R²⁹ are identical to or different from each other, and each represents a C1-C6 chain hydrocarbon group which may be optionally substituted with one or more halogen atoms, or a hydrogen atom,
R⁸, R⁹, R²⁴, R²⁵, and R³⁰ are identical to or different from each other, and each represents a C1-C6 chain hydrocarbon group which may be optionally substituted with one or more halogen atoms,
R¹¹, and R²⁷ are identical to or different from each other, and each represents a C1-C6 chain hydrocarbon group which may be optionally substituted with one or more halogen atoms, a (C1-C6 alkyl)carbonyl group, a (C1-C6 alkoxy)carbonyl group {each of the (C1-C6 alkyl)carbonyl group, and the (C1-C6 alkoxy) carbonyl group may be optionally substituted with one or more halogen atoms}, or a hydrogen atom,
R¹², R¹⁹, and R²⁸ are identical to or different from each other, and each represents a C1-C6 chain hydrocarbon group, a C1-C6 alkoxy group, a C1-C6 alkylamino group, or a C2-C8 dialkylamino group {each of the C1-C6 chain hydrocarbon group, the C1-C6 alkoxy group, the C1-C6 alkylamino group, and the C2-C8 dialkylamino group may be optionally substituted with one or more halogen atoms},
R¹³, R¹⁴, R¹⁵, R¹⁶, and R²¹ are identical to or different from each other, and each represents a C1-C6 chain hydrocarbon group which may be optionally substituted with one or more substituents selected from Group A, a C3-C8 alicyclic hydrocarbon group, a three (3)- to eight (8)-membered non-aromatic heterocyclic group {each of the C3-C8 alicyclic hydrocarbon group, and the three (3)- to eight (8)- membered non-aromatic heterocyclic group may optionally have one or more substituents selected from Group B}, a phenyl group, or a five (5)- to six (6)- membered aromatic heterocyclic group {each of the phenyl group, and the five (5)- to six (6)- membered aromatic heterocyclic group may optionally have one or more substituents selected from Group D},
R¹⁷ represents a C1-C6 chain hydrocarbon group which may optionally have one or more substituents selected from Group A, a C3-C8 alicyclic hydrocarbon group, a three (3)- to eight (8)- membered non-aromatic heterocyclic group {each of the C3-C8 alicyclic hydrocarbon group, and the three (3)- to eight (8)- membered non-aromatic heterocyclic group may optionally have one or more substituents selected from Group B}, a phenyl group, a five (5)- to six (6)- membered aromatic heterocyclic group {each of the phenyl group, and the five (5)- to six (6)- membered aromatic heterocyclic group may optionally have one or more substituents selected from Group D}, a (C1-C6 alkyl)carbonyl group, or a (C1-C6 alkoxy)carbonyl group {each of the (C1-C6 alkyl)carbonyl group, and the (C1-C6 alkoxy) carbonyl group may be optionally substituted with one or more halogen atoms},
R¹⁸ and R²⁰ are identical to or different from each other, and each represents a C1-C6 chain hydrocarbon group which may be optionally substituted with one or more substituents selected from Group A, or a hydrogen atom,
the neighboring R³ and R⁴ may combine together with a carbon atom to which they are attached to form a C5-C6 alicyclic hydrocarbon, a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {each of the benzene ring, and the five (5)- to six (6)-membered aromatic heterocyclic ring may optionally have one or more substituents selected from Group E},
two of the neighboring R³ and R³ may combine together with a carbon atom to which they are attached to form a C5-C6 alicyclic hydrocarbon, a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {each of the benzene ring, and the five (5)- to six (6)-membered aromatic heterocyclic ring may optionally have one or more substituents selected from Group E},
Group A is a group consisting of a C1-C6 alkoxy group, a C1-C6 alkylsulfanyl group, a C1-C6 alkylsulfinyl group, a C1-C6 alkylsulfonyl group, {each of the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)-membered non-aromatic heterocyclic group {each of the C3-C8 alicyclic hydrocarbon group, and the three (3)- to eight (8)- membered non-aromatic heterocyclic group may be optionally substituted with one or more substituents selected from Group B}, a phenyl group, a five (5)- to six (6) - membered aromatic heterocyclic group {each of the phenyl group and the five (5)- to six (6)- membered aromatic heterocyclic group may be optionally substituted with one or more substituents selected from Group D}, a halogen atom, a cyano group, a nitro group, and a hydroxy group.
Group B is a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {each of the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a halogen atom, and a cyano group.
Group C is a group consisting of a C1-C6 alkoxy group, a C1-C6 alkylsulfanyl group, a C1-C6 alkylsulfinyl group, a C1-C6 alkylsulfonyl group {each of the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)-membered non-aromatic heterocyclic group {each of the C3-C8 alicyclic hydrocarbon group, and the three (3)- to eight (8)- membered non-aromatic heterocyclic group may be optionally substituted with one or more substituents selected from Group B}, a phenyl group, and the five (5)- to six (6)- membered aromatic heterocyclic group {each of the phenyl group and the five (5)- to six (6)- membered aromatic heterocyclic group may be optionally substituted with one or more substituents selected from Group D}.
Group D is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonyl amino group, a (C1-C6 alkoxy)carbonyl amino group {each of the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino))carbonyl group, the (C2-C8 dialkylamino) carbonyl group, the (C1-C6 alkyl)carbonyl amino group, and the (C1-C6 alkoxy)carbonyl amino group may be optionally substituted with one or more halogen atoms}, a C3-C8 alicyclic hydrocarbon group which may optionally have one or more substituents selected from Group B, an amino group, a nitro group, a cyano group, a hydroxy group, and a halogen atom.
Group E is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group {each of the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a halogen atom, and a cyano group] or an N-oxide thereof, or a salt thereof to a plant or soil for cultivating the plant.

2. The method according to claim 1 wherein the compound represented by formula (I), the N-oxide thereof, or the salt thereof represents the compound represented by formula (I) wherein R¹ represents a C2-C6 chain hydrocarbon group which may be optionally substituted with one or more substituents selected from Group A, a C3-C8 alicyclic hydrocarbon group which may be optionally substituted with one or more substituents selected from Group B, or a methyl group which is substituted with one or more substituents selected from Group C
or an N-oxide thereof, or a salt thereof.

3. The method according to claim 1 or 2 wherein the compound represented by formula (I), the N-oxide thereof, or the salt thereof represents the compound represented by formula (I) wherein R¹ represents a C2-C6 chain hydrocarbon group, R² represents a C1-C6 chain hydrocarbon group which may be optionally substituted with one or more halogen atoms, or a halogen atom, R³ represents a C1-C6 chain hydrocarbon group which may be optionally substituted with one or more substituents selected from Group A, OR¹³, SR¹⁴, S(O)R¹⁵, S(O)₂R¹⁶, NR¹⁷R¹⁸, C(O)R¹⁹, C(R²⁰)=NOR²¹, a cyano group, a nitro group, an amino group, a hydroxy group, or a halogen atom, two of the neighboring R³ and R³ may combine together with a carbon atom to which they are attached to form a C5-C6 alicyclic hydrocarbon, or a five (5)- to six (6)- membered non-aromatic heterocyclic ring {each of the C5-C6 alicyclic hydrocarbon, and the five (5)- to six (6)- membered non-aromatic heterocyclic ring may optionally have one or more substituents selected from Group B}, and R⁴ and R⁵ are identical to or different from each other, and each represents a halogen atom, or a hydrogen atom or an N-oxide thereof, or a salt thereof.

4. Use of the compound according to any one of claims 1 to 3, the N-oxide thereof, or the salt thereof for controlling a plant disease.

5. A method for controlling a soybean rust fungus which comprises applying the compound according to any one of claims 1 to 3, the N-oxide thereof, or the salt thereof to a soybean or soil for cultivating the soybean.

6. A compound represented by formula (II): 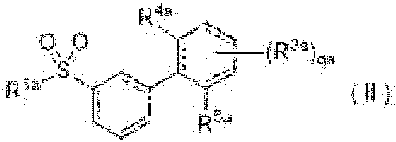 [wherein
R^{1a} represents a C2-C3 chain hydrocarbon group which may be optionally substituted with one or more halogen atoms, a cyclopropyl group which may be optionally substituted with one or more halogen atoms, a phenyl group, or a five (5)- to six (6)- membered aromatic heterocyclic group {each of the phenyl group and the five (5)- to six (6)- membered aromatic heterocyclic group may be optionally substituted with one or more halogen atoms},
R^{3a} represents a C1-C3 alkyl group, a C1-C3 alkoxy group, a cyclopropyl group {the cyclopropyl group may be optionally substituted with one or more halogen atoms}, or a halogen atom,
qa is 1, 2, or 3,
when qa is 2 or 3, two or three of the R^{3a} may be identical to or different from each other, and
R^{4a} and R^{5a} represent a hydrogen atom]
or an N-oxide thereof, or a salt thereof.

7. An agrochemical composition comprising the compound according to claim 6, the N-oxide thereof, or the salt thereof, and an inert carrier.

8. A composition comprising one or more ingredients selected from the group consisting of the following Group (a), Group (b), Group (c), and Group (d), as well as the compound according to claim 6 or an N-oxide thereof, or a salt thereof:
Group (a): a group consisting of insecticidal active ingredients, miticidal active ingredients and nematicidal active ingredients;
Group (b): fungicidal active ingredients;
Group (c): plant growth regulatory ingredients;
Group (d): repellent ingredients;

9. A method for controlling a plant disease which comprises applying an effective amount of the compound according to claim 6 or the N-oxide thereof, or the salt thereof, or an effective amount of the composition according to claim 8 to a plant or soil for cultivating the plant.

10. An agrochemical composition comprising the compound according to claim 6 or the N-oxide thereof, or the salt thereof, and an inert carrier.

11. A seed or vegetative reproductive organ carrying an effective amount of the compound according to claim 6 or an N-oxide, or a salt thereof, or an effective amount of the composition according to claim 8.
